# EUROPEAN PATENT SPECIFICATION

(11) **EP 3 451 836 B1**
(45) Date of publication and mention of the grant of the patent: **25.08.2021**
(21) Application number: 17726381.1
(22) Date of filing: 01.05.2017
(51) Int. Cl.: A01N 37/10, A01N 65/08, A01N 43/90, A01N 65/20, A01P 1/00, A61P 31/04

(54) **COMPOSITIONS OF SAPONINS AND PLANT EXTRACTS**
ZUSAMMENSETZUNGEN ENTHALTEND SAPONINE UND PFLANZENEXTRAKTE
COMPOSÉS COMPRENANT DES SAPONINS ET DES EXTRAITS DE PLANT

(30) Priority: 02.05.2016 IL 24537816; 06.10.2016 IL 24823016
(43) Date of publication of application: 13.03.2019
(73) Proprietor: Y&B Mother's Choice Ltd., 9139102 Jerusalem (IL)
(72) Inventor: SILBERSTEIN, Tova, 9314408 Jerusalem (IL); LUTZ, Rachel, 9091200 Gush Etzion (IL); COHEN, Eytan, 7630533 Rehovot (IL); SANDORI-KAZAZ, Haya, 7070000 Gedera (IL)
(74) Representative: Cabinet Becker et Associés
(86) International application number: PCT/IL2017/050482
(87) International publication number: WO 2017/191629

(56) References cited:
- WO-A1-96/41528
- WO-A2-2012/077119
- WO-A2-2012/077120
- US-A1- 2006 003 022
- DATABASE WPI Week 199719 Thomson Scientific, London, GB; AN 1997-203769 XP002109913, -& CN 1 096 918 A (HUBEI AGRIC COLLEGE) 4 January 1995 (1995-01-04)
- DATABASE WPI Week 201437 Thomson Scientific, London, GB; AN 2014-G99956 XP002771738, -& CN 103 598 225 B (UNIV ZHEJIANG NORMAL) 15 July 2015 (2015-07-15)
- YAN-QUN LI ET AL: "Analysis and evaluation of essential oil components of cinnamon barks using GC-MS and FTIR spectroscopy", INDUSTRIAL CROPS AND PRODUCTS., vol. 41, 1 January 2013 (2013-01-01), pages 269-278, XP055283423, NL ISSN: 0926-6690, DOI: 10.1016/j.indcrop.2012.04.056
- I. PASTOROVA ET AL: "Analytical Study of Free and Ester Bound Benzoic and Cinnamic Acids of Gum Benzoin Resins by GC-MS and HPLC-frit FAB-MS", PHYTOCHEMICAL ANALYSIS., vol. 8, no. 2, 1 March 1997 (1997-03-01), pages 63-73, XP055387763, GB ISSN: 0958-0344, DOI: 10.1002/(SICI)1099-1565(199703)8:2<63::AID -PCA337>3.0.CO;2-Y
- JUAN GUZMAN: "Natural Cinnamic Acids, Synthetic Derivatives and Hybrids with Antimicrobial Activity", MOLECULES, vol. 19, no. 12, 25 November 2014 (2014-11-25), pages 19292-19349, XP055388174, DOI: 10.3390/molecules191219292
- Ram Kumar Pundir ET AL: "Evaluation of five chemical food preservatives for their antibacterial activity against bacterial isolates from bakery products and mango pickles", J. Chem. Pharm. Res, 1 January 2011 (2011-01-01), pages 24-31, XP055388185, Retrieved from the Internet: URL:http://www.jocpr.com/articles/evaluati on-of-five-chemical-food-preservatives-for -their-antibacterial-activityagainst-bacte rial-isolates-from-bakery-pr.pdf [retrieved on 2017-07-05]
- D.T.U. ABEYTUNGA ET AL: "Structure-antibacterial activity relationship of some aromatic acids", JOURNAL OF THE NATIONAL SCIENCE FOUNDATION, vol. 26, no. 2, 1 January 1998 (1998-01-01), pages 133-139, XP055388187, LK ISSN: 1391-4588, DOI: 10.4038/jnsfsr.v26i2.3561
- P A Paranagama ET AL: "A COMPARISON OF ESSENTIAL OIL CONSTITUENTS OF BARK, LEAF, ROOT AND FRUIT OF CINNAMON (CMAMOMUMZEYLANICUM BLUIM) GROWN IN SRI LANKA", J. Natn. Sci Foundation Sri Lanka, 1 January 2002 (2002-01-01), pages 147-153, XP055387994, Retrieved from the Internet: URL:http://jnsfsl.sljol.info/article/10.40 38/jnsfsr.v29i3-4.2613/galley/2096/downloa d/ [retrieved on 2017-07-05]

## Description

### TECHNOLOGICAL FIELD

The invention generally concerns compositions comprising saponins and plant extracts.

### BACKGROUND

As known in the art, saponins are compounds constructed of a triterpene or steroid moiety (aglycon or sapogenin) and one or two glycoside moieties (monodesmosides or bidesmosides, respectively). The aglycon carbon skeleton may be saturated or unsaturated and/or comprise a heteroatom such as nitrogen. The glycoside moiety contains sugars such as galactose, glucose, glucuronic acid, methylpentose, rhamnose and xylose.

The saponin family is known to have a wide range of biological activities such as antimicrobial, antiherbivore and/or cytotoxic activity and their role in nature is likely to be in defense against pathogens, pests and predators. In plants, saponins appear to act as pre-formed antimicrobial barriers to pathogen attack but can also function as suppressors of induced defense responses following hydrolysis.

Public concern about the safety of synthetic preservatives used in cosmetic and foods, especially regarding their accumulation and subsequent health effect, have driven health authorities to reduce the applied concentrations or even ban synthetic preservatives. Alternatives such as plant antimicrobial substances are in the focus of many researches, however due to low potency, narrow range and high prices, they are rarely used to replace synthetic preservatives.

### REFERENCES

References considered to be relevant as background to the presently disclosed subject matter are listed below:
**[1]** "The saponins - polar isoprenoids with important and diverse biological activities" A. Osbourn, et al., Nat. Prod. Rep., 2011, 28, 1261
**[2]** WO 2000/072861
**[3]** WO 1998/048768
**[4]** US 2006/0018867
**[5]** WO 2009/153800
**[6]** WO 2012/077119
**[7]** WO 2012/077120
**[8]** CN101085222
**[9]** WO 2001/47481

### SUMMARY OF THE INVENTION

The present invention is defined by the appended claims.

Although saponin exhibits preservation properties, such may often prove to be insufficient against various microbiological contaminants. The inventors of the invention have surprisingly found that compositions of at least one saponin material and extracts of various plant species exhibit biological activity, e.g., antimicrobial activity, which is superior to the activity demonstrated for each component individually and which is at least comparable, and at times even superior, to chemical (i.e. non-natural) alternatives known for the same use.

Thus, in one of its aspects, the invention provides a composition having an antimicrobial activity, comprising saponin extract obtained from a saponin-containing plant source extracted by water, alcohol or a water/alcohol solution; and a *Styrax* extract from the resin and/or bark of at least one plant species of the genus *Styrax* selected from *Styrax paralleloneurus* (Sumatra benzoin), *Styrax tonkinensis* (Siam Benzoin) and mixtures thereof, the Styrax extract being obtained by mixing the resin and/or bark with a hydrophobic solvent, the saponin extract being from a saponin source different from said plant species of the genus *Styrax,* the *Styrax* extract containing at least one of cinnamic acid; cinnamic acid derivatives selected from P-coumaryl cinnamate, coniferyl cinnamate, cinnamyl cinnamate, benzyl cinnamate, and cinnamic acid esters; and/or benzoic acid derivatives selected from coniferyl benzoate, cinnamyl benzoate, P-coumaryl benzoate, and benzoic acid esters.

As used herein, the term ***"extract"*** refers to an active ingredient or fraction isolated from a plant, typically by solvent extraction, although other extraction techniques known *per-se* are also contemplated. The extraction procedure for obtaining any of the plant extracts employed in accordance with the invention, unless otherwise indicated, may be carried out in any commonly used technique and variation known in the art as described for example in M. Casey, J. Leonard, B. Lygo, and G. Procter "Advanced Practical organic Chemistry", 1990, Chapman & Hall, London.

Extracts of the plant species used for preparing the compositions of the invention may be prepared prior to formulation, in advance of formulation or may be commercially available. The extracts may be used without further purification.

*Saponin material,* as used herein is at least one naturally obtained saponin compound, as known in the art. When isolated from a natural source, the saponin material may be used in its substantially pure form (namely at least 85%, 87%, 92%, 95%, or 98% purity), or may be used as a ***"saponin-containing extract"*** (also referred to herein for the purpose of brevity as *"saponin extract*") isolated by a method known in the art.

In accordance with the present invention, the saponin-containing extract contains at least between 0.2% and 95 wt% saponins, out of the total weight of the dry content of the extract. In some embodiments, the extract used in accordance with the present invention comprises between 0.2% and 99 wt% saponins out of the total weight of the dry content of the extract.

In some embodiments, the saponin extract used in compositions of the present invention may comprise between about 10% and about 80 wt% saponins out of the total weight of the dry content of the extract. In other embodiments, the saponin extract may comprise between about 10% and about 60 wt% saponins, between about 10% and about 50 wt% saponins, between about 10% and about 40 wt% saponins, between about 10% and about 30 wt% saponins, or even between about 10% and about 20 wt% saponins out of the total weight of the dry content of the extract. In some embodiments, the saponin extract comprises between about 0.2% and about 10 wt% saponins out of the total weight of the dry content of the extract.

When isolated from a natural source, the saponin extract may be used in its substantially pure form (namely at least 85%, 87%, 92%, 95%, or 98% purity).

In some embodiments of the invention, the saponins are extracted from a plant source, naturally grown or genetically modified to have high saponin content. The saponin material is obtained by extraction from a plant source by employing water, alcohol or a water/alcohol solution. In some embodiments, the alcohol is ethanol or methanol.

The plant source may be selected from shikakai, soyabeans, beans, peas *(Pisum sativum*), lucerne, tea, spinach, sugar beet, quinoa, liquorice, sunflower, horse chestnut, ginseng, oats, capsicum peppers, aubergine, tomato seed, alliums, asparagus, yam, fenugreek, yucca and ginseng, lucerne, mung beans, *Bupleurum falcatum, Camellia oleifera, Camellia sinensis, Desmodium adscendens, Gypsophila, Panax quinqufolius, Panax japonicas, Quillaja saponaria, Sapindus delavayi, Sapindus mukorossi, Sapindus marginatus, Sapindus saponaria, Sapindus trifoliatus, Saponaria officinalis,* and *Yucca schidigera* or any mixture thereof

In some embodiments, the saponin extract is obtained from a plant source selected from *Camellia oleifera, Camellia sinensis, Quillaja saponaria, Sapindus mukorossi, Sapindus saponaria,* and *Saponaria officinalis* or any mixture thereof. In other embodiments, the saponin extract is obtained from *Camellia oleifera, Quillaja saponaria* and/or *Sapindus mukorossi.* Saponin containing material may be purified by any means known in the art, including: filtration, centrifugation, re-crystallization, distillation, adsorption, chromatographic methods, fractionation, etc.

The saponin extract may be obtained from any part of the plant, including leaves, stems, roots, bulbs, blossom and fruit (including the skin, flesh and seed of the fruit). In some embodiments, the extracts are obtained from the pericarp of *Sapindus mukorossi,* or the seed meal of *Camellia oleifera.*

Compositions of the invention may comprise, by some embodiments, at least 0.001 wt% saponin. In some embodiments, the compositions of the invention comprise between about 0.01 and 2 wt%, between about 0.01 and 1.5 wt%, between about 0.01 and 1 wt%, or even between about 0.01 and 0.7 wt% saponin.

As noted above, in addition to the saponin extract, the composition comprises an extract from at least one plant species of the genus *Styrax* selected from *Styrax paralleloneurus* (Sumatra benzoin), *Styrax tonkinensis* (Siam Benzoin) and mixtures thereof. It is of note that the saponin extract is from a saponin source different from said plant species of the genus *Styrax.*

The genus ***Styrax*** contains a group of small trees or shrubs in the family *Styracaceae,* which often secrete a gum-like resin containing at least benzoic acid, coniferyl benzoate and other compounds. The gum-like resin or tree bark is often called *"benzoin resin".* It should be appreciated that the extract may be an extract of more than one plant selected within the genus.

The *Styrax* extract is obtained by mixing the resin and/or bark with a hydrophobic solvent, such as at least one oil (e.g. paraffinic oil, triglycerides, non-triglyceride oils, etc.), pentane, hexane, cyclohexane, heptane, octane, dichloromethane, dichloroethane, chloroform, etc.

In other embodiments, each of the plant extracts (i.e. either or both of the saponin and *Styrax* extracts) is obtained commercially.

The inventors of the present invention have found that natural extracts of *Styrax* contain various compounds which provide, once formulated with the saponin-containing extract, comparable or even superior preservation properties compared to chemical (i.e. non-natural) alternatives are obtained.

In some embodiments, the total amount of cinnamic acid, cinnamic acid derivatives and benzoic acid derivatives in the *Styrax* extract is at least 0.001, 0.01, or even at least 0.1 wt%. In other embodiments, the total amount of cinnamic acid, cinnamic acid derivatives and benzoic acid derivatives in the *Styrax* extract may be at most 10 wt%. In some other embodiments, the total amount of cinnamic acid, cinnamic acid derivatives and benzoic acid derivatives in the *Styrax* extract may be between about 0.001 and 10wt%, between about 0.01 and 10 wt%, between about 0.1 and 10 wt%, or even between about 1 and 10 wt%.

The term ***derivative*** refers to a chemically modified compound derived from a parent compound (e.g., cinnamic acid or benzoic acid) that differs from the parent compound by one or more elements, substituents and/or functional groups such that the derivative has the same or similar properties/activities as the parent compound, as defined herein.

The Styrax extract may further comprise various terpenes and terpenoids, as well as other phenolic derivatives, such as pinoresinol.

In some embodiments, cinnamic acid, cinnamic acid derivatives and/or benzoic acid derivatives may be components of a *Styrax* extract from the resin of at least one plant species of the genus *Styrax* as defined above (such that the saponin extract of the composition is from a saponin source different from said plant species of the genus *Styrax).*

Generally, in the compositions of the invention, the weight-to-weight ratio (wt/wt) between the saponin extract and the Styrax extract may range between 1:50 and 50:1 (saponin material:*Styrax* extract). In some embodiments, the weight-to-weight ratio between the saponin extract and the *Styrax* extract is about 1:50, 1:45, 1:30, 1:25, 1:20, 1:15, 1:10, 1:8, 1:7, 1:6, 1:5, 1:4, 1:3, 1:2, 1:1, 2:1, 3:1, 4:1, 5:1, 6:1, 7:1; 8:1, 10:1, 15:1, 20:1, 25:1, 30:1, 35:1, 40:1, 45:1, or about 50:1.

In other embodiments, the weight ratio between the saponin extract and the Styrax extract may be between 1:10 and 10:1. In some other embodiments, the weight ratio is between 1:5 and 5:1.

In some embodiments, the composition may further comprise at least one additional plant extract, i.e. a plant extract other than the saponin extract or the Styrax extract.

According to some embodiments, the additional plant extract may be selected from extracts of at least one plant species of the genera *Eutrema (Wasabia), Acacia, Terminalia, Olea,* and mixtures thereof. It should be understood that the additional extract may be an extract of more than one plant selected from the genera *Eutrema, Acacia, Terminalia, Olea* genus or from a different genus. It should be further that the present invention further contemplates compositions comprising mixtures of such extracts, whether prepared and formulated individually or prepared in one-pot from a mixture of plant sources (plant parts).

The genus ***Acacia*** is a monophyletic genus of flowering plants in the legume family *Fabaceae,* and includes *inter* alia, the species *Acacia abyssinica, Acacia acuifera, Acacia albicortata, Acacia allenii, Acacia amythethophylla, Acacia ancistroclada, Acacia anegadensis, Acacia antunesii, Acacia arenaria (A. nilotica), Acacia arenaria, Acacia aroma, Acacia astringens, Acacia baessleri, Acacia barahonensis, Acacia bavazzanoi, Acacia belairoides, Acacia biaciculata, Acacia bidwillii, Acacia bilimekii, Acacia borleae, Acacia brandegeana, Acacia bravoensis, Acacia bricchettiana, Acacia bucheri, Acacia bullockii, Acacia burttii, Acacia bussei, Acacia californica, Acacia campechiana, Acacia caurina, Acacia caven, Acacia cernua, Acacia chiapensis, Acacia choriophylla, Acacia clarksoniana, Acacia collinsii, Acacia constricta, Acacia cookii, Acacia constricta, Acacia cornigera, Acacia cucuyo, Acacia curvifructa, Acacia daemon, Acacia davyi, Acacia ditricha, Acacia dolichocephala, Acacia douglasica, Acacia drepanolobium, Acacia dyeri, Acacia eburnea, Acacia ebutsiniorum, Acacia edgeworthii, Acacia elatior, Acacia erioloba, Acacia erythrophloea, Acacia etbaica, Acacia exuvialis, Acacia farnesiana, Acacia fischeri, Acacia flava, Acacia gentlei, Acacia gerrardii, Acacia glandulifera, Acacia globulifera, Acacia grandicornuta, Acacia guanacastensis, Acacia gummifera, Acacia haematoxylon, Acacia harmandiana, Acacia hebeclada, Acacia hindsii, Acacia hockii, Acacia horrida, Acacia inopinata, Acacia insulae-iacobi, Acacia janzenii, Acacia karroo, Acacia kingii, Acacia kirkii, Acacia koltermanii, Acacia kosiensis, Acacia lahai, Acacia lasiopetala, Acacia latispina, Acacia leucophloea, Acacia leucospira, Acacia luederitzii, Acacia macracantha, Acacia macrothyrsa, Acacia malacocephala, Acacia mayana, Acacia mbuluensis, Acacia melanoceras, Acacia myaingii, Acacia natalitia, Acacia nebrownii, Acacia negrii, Acacia neovernicosa, Acacia nilotica (A. arabica,* Gum arabic tree, Babul, Amrad gum, Thorny mimosa), *Acacia nubica, Acacia oerfota, Acacia origena, Acacia ormocarpoides, Acacia oviedoensis, Acacia pacensis, Acacia pachyphloia, Acacia pallidifolia, Acacia paolii, Acacia pennatula, Acacia permixta, Acacia pilispina, Acacia polypyrigenes, Acacia prasinata, Acacia pringlei, Acacia pseudofistula, Acacia qandalensis, Acacia quintanilhae, Acacia reficiens, Acacia rehmanniana, Acacia retinodes, Acacia rigidula, Acacia robbertsei, Acacia robusta, Acacia roigii, Acacia rorudiana, Acacia rovumae, Acacia ruddiae, Acacia schaffneri, Acacia schottii, Acacia schweinfurthii, Acacia sekhukhuniensis, Acacia senegal, Acacia seyal, Acacia sieberiana, Acacia sphaerocephala, Acacia stuhlmannii, Acacia suberosa, Acacia sutherlandii, Acacia swazica, Acacia tenuispina, Acacia tephrophylla, Acacia theronii, Acacia tirion, Acacia tomentosa, Acacia torrei, Acacia tortilis, Acacia tortuosa, Acacia turnbulliana, Acacia valida, Acacia villaregalis, Acacia walwalensis, Acacia xanthophloea, Acacia zanzibarica, Acacia zapatensis, Acacia bellula, Acacia bolei, Acacia callicoma, Acacia harala, Acacia hunteri, Acacia hydaspica, Acacia jacquemontii, Acacia johnwoodii, Acacia myrmecophila, Acacia planifrons, Acacia pseudo-eburnea, Acacia tanjorensis, Acacia viguieri* and *Acacia yemenensis.*

In some embodiments, the Acacia extract is an extract of *Acacia Arabica* (also known as *Acacia nilotica.* In other embodiments, the Acacia extract is an extract of *Acacia Arabica* leaves, fruit, pericarp, or a mixture thereof.

The genus ***Terminalia*** is a genus of large trees of the flowering plant family *Combretaceae,* which includes, *inter* alia, the species *Terminalia acuminata, Terminalia albida, Terminalia altissima, Terminalia amazonia, Terminalia arbuscula, Terminalia archipelagi, Terminalia arenicola, Terminalia argentea, Terminalia arjuna, Terminalia australis, Terminalia avicennioides, Terminalia bellerica (Myrobalanus bellerica), Terminalia bentzoe, Terminalia bialata, Terminalia brachystemma, Terminalia brassii, Terminalia brownii, Terminalia bucidoides, Terminalia buceras, Terminalia bursarina, Terminalia calamansanai, Terminalia carpentariae, Terminalia catappa, Terminalia chebula, Terminalia cherrieri, Terminalia ciliata, Terminalia citrina, Terminalia corticosa, Terminalia eddowesii, Terminalia elliptica, Terminalia eriostachya, Terminalia ferdinandiana, Terminalia foetidissima, Terminalia franchetii, Terminalia glabrescens, Terminalia glaucifolia, Terminalia grandiflora, Terminalia hararensis, Terminalia hecistocarpa, Terminalia intermedia, Terminalia ivorensis, Terminalia januariensis, Terminalia kaernbachii, Terminalia kangeanensis, Terminalia kuhlmannii, Terminalia latifolia, Terminalia latipes, Terminalia littoralis, Terminalia macroptera, Terminalia mantaly, Terminalia microcarpa, Terminalia muelleri, Terminalia myriocarpa, Terminalia nitens, Terminalia novocaledonica, Terminalia oblonga, Terminalia oblongata, Terminalia obovata, Terminalia oliveri, Terminalia paniculata, Terminalia parviflora, Terminalia pellucida, Terminalia petiolaris, Terminalia phanerophlebia, Terminalia phellocarpa, Terminalia porphyrocarpa, Terminalia procera, Terminalia prunioides, Terminalia reitzii, Terminalia rerei, Terminalia richii, Terminalia saffordii, Terminalia schimperiana, Terminalia sericea, Terminalia sericocarpa, Terminalia subspathulata, Terminalia superba, Terminalia triflora, Terminalia trifoliata,* and *Terminalia tripteroides.*

In some embodiments, the Terminalia extract is an extract of *Terminalia bellerica.* In other embodiments, the Terminalia extract is an extract of *Terminalia bellerica* leaves, fruit, pericarp, or a mixture thereof. In some other embodiments, the Terminalia extract is an extract of *Terminalia bellerica* fruit, pericarp, or a mixture thereof.

The genus ***Olea*** is a genus of the family *Oleaceae,* and includes *inter* alia, the species *Olea ambrensis, Olea borneensis, Olea brachiata, Olea capensis, Olea capitellata, Olea caudatilimba, Olea chimanimani, Olea cordatula, Olea dioica, Olea europaea, Olea exasperata, Olea gagnepainii, Olea gamblei, Olea hainanensis, Olea javanica, Olea lancea, Olea laxiflora, Olea moluccensis, Olea neriifolia, Olea palawanensis, Olea paniculata, Olea parvilimba, Olea polygama, Olea puberula, Olea rosea, Olea rubrovenia, Olea salicifolia, Olea schliebenii, Olea tetragonoclada, Olea tsoongii, Olea welwitschii, Olea wightiana, Olea woodiana, Oleayuennanensis,* and *Olea hoschstetteri.*

In some embodiments, the *Olea* extract is an extract of *Olea europaea.* In other embodiments, the Olea extract is an extract *of Olea europaea* leaves.

According to other embodiments, the additional plant extract may be an extract of at least one plant species of the genera *Eutrema.* The genus *Eutrema* (or *Wasabia)* belonging to the *Brassicaceae* family includes *inter alia* the species *Wasabia japonica, Wasabia koreana, Wasabia tetsuigi, Wasabia tenuis, Wasabia bracteata, Wasabia okinosimensis, Wasabia pungens, Wasabia thibeticum* and *Wasabia yunnanensis.* In some embodiments, the Wasabia extract is an extract of *Wasabia japonica, Wasabia koreana, Wasabia tetsuigi, Wasabia tenuis, Wasabia bracteata, Wasabia okinosimensis, Wasabia pungens, Wasabia thibeticum* and/or *Wasabia yunnanensis.* In other embodiments, the Wasabia extract is an extract of *Wasabia japonica.* In some other embodiments, the Wasabia extract is an extract of *Wasabia japonica* roots, bulbs, or a mixture thereof.

Generally, in the compositions of the invention, the weight-to-weight ratio (wt/wt) between the additional plant extract and the Styrax extract may range between about 1:400 and 400:1, between about 200:1 and 1:200, or even between about 1:100 and 100:1. In some embodiments, the weight-to-weight ratio between the additional plant extract and the *Styrax* extract is about 1:100, 1:90, 1:80, 1:70, 1:60, 1:50, 1:40, 1:30, 1:20, 1:19, 1:18, 1:17, 1:16, 1:15, 1:14, 1:13, 1:12, 1:11, 1:10, 1:9, 1:8, 1:7, 1:6, 1:5, 1:4, 1:3, 1:2, 1:1,2:1, 3:1, 4:1, 5:1, 6:1, 7:1; 8:1, 9:1, 10:1, 11:1, 12:1, 13:1, 14:1, 15:1, 16:1, 17:1, 18:1, 19:1, 20:1, 30:1, 40:1, 50:1, 60:1, 70:1, 80:1, 90:1 or about 100:1.

In other embodiments, the weight ratio between the additional plant extract and the *Styrax* extract may be between 1:25 and 25:1. In some other embodiments, the weight ratio is between 1:20 and 20:1.

According to some embodiments, the weight ratio between the extract of the genera *Eutrema* and the *Styrax* extract may be between 1:100 and 100:1, between 1:25 and 25:1, or even between 1:20 and 20:1.

According to other embodiments, the weight ratio between the extract of the genera *Acacia* and the *Styrax* extract may be between 1:100 and 100:1, between 1:25 and 25:1, or even between 1:20 and 20:1.

According to some other embodiments, the weight ratio between the extract of the genera ***Terminalia*** and the *Styrax* extract may be between 1:100 and 100:1, between 1:25 and 25:1, or even between 1:20 and 20:1.

According to further embodiments, the weight ratio between the extract of the genera *Olea* and the Styrax extract may be between 1:100 and 100:1, between 1:25 and 25:1, or even between 1:20 and 20:1.

The content of the additional plant extract in the composition may be, by some embodiments, at least 0.001 wt%. In some embodiments, the content of the additional plant extract in compositions of the invention may be between about 0.005 and 1 wt%, between about 0.005 and 0.5 wt% or even between about 0.001 and 0.2 wt%.

In the compositions of the invention, the weight-to-weight ratio (wt/wt) between the saponin extract and the additional plant extract may range between 1:200 and 200:1 (saponin material : additional plant extract). In some embodiments, the weight-to-weight ratio between the saponin extract and the additional plant extract is about 1:200, 1:175, 1:150, 1:125, 1:100, 1:75, 1:50, 1:45, 1:30, 1:25, 1:20, 1:15, 1:10, 1:8, 1:7, 1:6, 1:5, 1:4, 1:3, 1:2, 1:1, 2:1, 3:1, 4:1, 5:1, 6:1, 7:1; 8:1, 10:1, 15:1, 20:1, 25:1, 30:1, 35:1, 40:1, 45:1, 50:1, 75:1, 100:1, 125:1, 150:1, 175:1, or even between 200:1.

In other embodiments, the weight ratio between the saponin extract and the additional plant extract may be between 100:1 and 1:100. In some other embodiments, the weight ratio is between 100:1 and 1:1.

According to other embodiments, the additional plant extract may be selected from extracts of the genera *Wasabia, Acacia, Terminalia, Olea,* and mixtures thereof. In such embodiments, the additional extract may be selected from *Wasabia japonica* extract, *Acacia arabica* (gum arabic tree, Babul) extract, *Terminalia* extract, *Olea europaea* extract and mixtures thereof.

In some embodiments, the additional plant extract may an extract of the genera *Acacia,* such as an extract of *Acacia arabica* (gum arabic tree, Babul) extract.

In other embodiments, the additional plant extract may an extract of the genera *Terminalia* extract.

In some other embodiments, the additional plant extract may an extract of the genera, *Olea europaea.*

According to further embodiments, the additional plant extract may be *E. japonicum (Wasabia japonica,* wasabi) extract.

In some embodiments, the composition may comprise at least one co-emulsifier. The ***co-emulsifier,*** together with the saponin extract and the *Styrax* extract may function provide at least one desired effect (such as foaming, viscosity, skin-feel, etc.). According to some embodiments, the co-emulsifier may be selected from fatty acids glycerides (mono- or diglycerols), polyglycerols of fatty acids, and other. The term means to encompass short, medium or long chain fatty acids glycerols. Further, the term also encompasses fatty acids glycerides derivatives, such as esters and ethoxylated glycerols. Surprisingly, as exemplified herein, it was found that the co-emulsifier may also improve antibacterial properties of the composition.

According to some embodiments, the fatty acid is a naturally obtained fatty acid and/or fatty acid derived from a plant source (i.e. from vegetation).

By some embodiments, the co-emulsifier is glyceryl caprylate and derivatives thereof.

The composition may comprise between about 0.001 and 0.5 wt% of said co-emulsifier.

Another aspect of this disclosure provides a composition comprising saponin extract, an extract from at least one plant species of the genus *Styrax,* and glyceryl caprylate.

In a further aspect, this disclosure provides a composition comprising saponin extract, an extract from at least one plant species of the genus *Styrax,* and *Wasabia* extract.

It is of note that the compositions of the invention may not always comprise an additional plant extract. Hence, another aspect of this disclosure provides a composition comprising at antimicrobial effective amount of a mixture of agents, the mixture of agents consisting of a saponin extract and a *Styrax* extract from the resin of at least one plant species of the genus *Styrax,* the saponin extract being from a saponin source different from said plant species of the genus *Styrax.*

In another aspect, there is provided a composition comprising at antimicrobial effective amount of a mixture of agents, the mixture of agents consisting of a saponin extract selected from *Camellia oleifera* extract, *Camellia sinensis* extract, *Quillaja saponaria* extract, *Sapindus mukorossi* extract, *Sapindus saponaria* extract, and *Saponaria officinalis* extract, and any mixture thereof, and a *Styrax* extract from *Styrax paralleloneurus* (Sumatra benzoin) extract, *Styrax tonkinensis* (Siam Benzoin) extract and mixtures thereof.

Yet a further aspect provides for an add-on composition consisting of a combination of at least one saponin extract and at least one *Styrax* extract from the resin of at least one plant species of the genus *Styrax,* the saponin extract being from a saponin source different from said plant species of the genus *Styrax.*

The term ***add-on composition*** (or *add-on formulation*) is meant to refer to a composition that is added to another, already-prepared composition. For example, the add-on composition may be added to a variety of other products, e.g. shampoo, soap, cream, lotion, etc., to provide these products with a desired property. Such property may be antimicrobial activity, foaming, viscosity modification, improved absorbance, and the like. It is of note that the add-on formulation may be formulated separately from the composition of the product, and then added to the product. However, it is to be understood that each of the components of the add-on formulation may be individually added to the composition of the product at any desired addition sequence.

The inventors of the present invention have also found that natural extracts may contain various compounds which provide, once formulated with the saponin-containing extract, comparable or even superior preservation properties compared to chemical (i.e. non-natural) alternatives are obtained.

Thus, in another aspect, the present disclosure provides a composition comprising saponin extract, and a mixture comprising at least one amino acid and at least one organic acid. Without wishing to be bound by theory, it is contemplated that the combination of the amino acid and the organic acid forms a complex which both preserves the antimicrobial activity of each component, as well as balances the pH of the composition, such that the composition is not overly acidified.

In some embodiments, the amino acid is arginine and the organic acid is salicylic acid.

According to some embodiments, the weight ratio between arginine and salicylic acid in the mixture is between about 1:1 and about 1:3. In some embodiments, the weight ratio between arginine and salicylic acid in the mixture is between about 1:1.2 and about 1:3, between about 1:1.4 and about 1:3, between about 1:1.6 and about 1:3, between about 1:1.8 and about 1:3, or even between about between about 1:2 and about 1:3. In other embodiments, the weight ratio between arginine and salicylic acid in the mixture is between about 1:1 and about 1:2.8, between about 1:1 and about 1:2.6, between about 1:1 and about 1:2.4, between about 1:1 and about 1:2, or even between about between about 1:1 and about 1:2.

According to other embodiments, the mixture may comprise between about 20 wt% and 30 wt% of salicylic acid.

According to some other embodiments, the mixture may comprise between about 1 wt and 20 wt% arginine.

The mixture and/or the composition, by some embodiments, may further comprise at least one sugar, i.e. a mono- or di-saccharide. Without wishing to be bound by theory, it is contemplated that the presence of the sugar increases the solubility of the acids in the composition, thereby assisting in the formulation and/or enabling a higher load of the acids into the composition.

In some embodiments, the at least one sugar is selected from glucose, fructose, galactose, glucosamine, sucrose, lactulose, lactose, maltose, trehalose, cellobiose and chitobiose.

According to other embodiments, the mixture comprises glucose and/or trehalose. In such embodiments, the content of the glucose in the mixture may be between about 40 wt% and about 55 wt% glucose. In other such embodiments, the mixture may comprise between about 3 wt% and about 6 wt% trehalose.

The mixture, according to some embodiments, may be present in composition in a content of between about 0.001 and about 2 wt% of the composition. In other embodiments, the mixture is in a content of between about 0.001 and about 1.5 wt%, between about 0.001 and about 1 wt%, or even between about 0.001 and about 0.5 wt% of the composition.

The composition of this aspect may further comprise at least one plant extract, which may, by some embodiments, be selected from an extract from at least one plant species of the genera *Styrax, Acacia, Terminalia, Olea,* and mixtures thereof. In such embodiments, the plant extract is selected from a *Styrax* extract of *Styrax paralleloneurus* (Sumatra benzoin), *Styrax tonkinensis* (Siam Benzoin) and mixtures thereof; by some embodiments, the weight ratio between the saponin extract and the *Styrax* extract is between about 1:50 to about 50:1.

In other embodiments, the plant extract is an *E. japonicum* (*Wasabia japonica,* wasabi) extract. By some embodiments, the weight ratio between the saponin extract and the *Styrax* extract is between about 1:50 to about 50:1.

In some other embodiments, the plant extract is an *Acacia arabica* extract.

In further embodiments, the plant extract is a *Terminalia bellerica* extract.

In yet further embodiments, the plant extract is an *Olea europaea* extract.

In another aspect, the present disclosure provides a composition comprising arginine, salicylic acid, glucose and trehalose.

According to some embodiments, the weight ratio between arginine and salicylic acid is between about 1:1 and about 1:3. According to other embodiments, the compositions may comprise between about 20 wt% and 30 wt% of salicylic acid. According to some other embodiments, the compositions may comprise between about 1 wt and 20 wt% arginine.

According to some embodiments, the content of the glucose in the composition may be between about 40 wt% and about 55 wt%. In other embodiments, the composition may comprise between about 3 wt% and about 6 wt% trehalose.

In yet another aspect, the present disclosure provides an antibacterial composition comprising arginine, salicylic acid, glucose and trehalose.

The composition may be formulated into various formulations which require preservation, disinfection or reduction in bacterial contaminant. In such formulations, the content of the composition may be between about 0.001 and about 2 wt% of the formualtion.

Another one of the aspects of the present disclosure provides a composition comprising saponin extract, an extract from at least one plant species of the genus *Styrax,* and a mixture comprising arginine and salicylic acid.

According to some embodiments, mixture is present in a content of between about 0.001 and about 2 wt% of the composition.

According to other embodiments, the weight ratio between arginine and salicylic acid in the mixture is between about 1:1 and about 1:3.

According to some other embodiments, the mixture may comprise between about 10 wt% and 30 wt% of salicylic acid.

According to additional embodiments, the mixture may comprise between about 15 wt and 30 wt% arginine.

In some embodiments of this aspect, the composition may further comprise glucose and/or trehalose. In such embodiments, the content of the glucose in the mixture may be between about 40 wt% and about 55 wt% glucose. In other such embodiments, the mixture may comprise between about 3 wt% and about 6 wt% trehalose.

The mixture, according to some embodiments, may be present in composition in a content of between about 0.001 and about 2 wt% of the composition.

The extract from at least one plant species of the genus *Styrax* is as defined hereinabove. In some embodiments, the *Styrax* extract is an extract of *Styrax paralleloneurus* (Sumatra benzoin), *Styrax tonkinensis* (Siam Benzoin) and mixtures thereof. In such embodiments, the weight ratio between the saponin extract and the *Styrax* extract is between about 1:50 to about 50:1.

The composition of this aspect may further comprise at least one additional plant extract (i.e. other than the Styrax extract), which may, by some embodiments, be selected from an extract from at least one plant species of the genera, *Acacia, Terminalia, Olea,* and mixtures thereof.

In other embodiments, the plant extract is of at least one plant species of the genera *Eutrema,* such as *E. japonicum* (*Wasabiajaponica,* wasabi) extract.

In some other embodiments, the plant extract is an *Acacia arabica* extract.

In further embodiments, the plant extract is a *Terminalia bellerica* extract.

In yet further embodiments, the plant extract is an *Olea europaea* extract.

The compositions of the invention can be prepared by any commonly used method for preparing a composition of materials. For example, the components of the compositions may be added as solids and mixed together, or one of the components may be added to the other in the form of a solution which may, if desired be evaporated or lyophilized after mixing for obtaining a homogeneous solution.

As will be further demonstrated below, the compositions of the invention exhibit antimicrobial properties which render the compositions suitable for a variety of applications in the fields of, e.g., cosmetics, therapeutics, foodstuffs and as material preservation.

The composition of the invention may thus be formulated into a variety of formulations, such as a cosmetic formulation, a therapeutic formulation, an antimicrobial formulation, a food additive formulation and a preservative formulation. Each of the aforementioned formulations may further comprise an excipient, diluents, or carrier suitable for the particular application, together with at least one additional additive as disclosed herein.

In another of its aspects, the invention provides a cosmetic or cleansing formulation comprising compositions of the present disclosure as defined in the various embodiments hereinabove.

The cosmetic/cleansing formulations according to the invention are typically formulated in a form adapted for topical application comprising a cosmetically or dermatologically acceptable medium, namely a medium which is suitable for application onto the skin of a subject (human or non-human). The medium may be in the form of aqueous or hydroalcoholic solution, an oil-in-water or water-in-oil emulsion, a microemulsion, aqueous or anhydrous gels, serum, or else a dispersion of vesicles, a patch, cream, spray, salve, ointment, lotion, gel, solution, suspension, or any other known cosmetically acceptable form. The formulation may alternatively be formulated for application to the human skin, hair, eyelashes, eyebrows, or nails.

In addition, the formulation may contain other standard additives such as an emollient, moisturizer, thickener, emulsifier, neutralizer, coloring agent, a fragrance, absorber or filter, preservative and/or gelling agent such as those described below, filler such as nylon, a sun screen agent, electrolytes, proteins, antioxidants and chelating agents.

The formulation may also further comprise at least one active ingredient such as peptide active ingredients, vegetable extracts, anti-age agents, anti-wrinkle agents, soothing agents, radical scavengers, UV absorbing agents, agents stimulating the synthesis of dermal macromolecules or the energy metabolism, hydrating agents, antibacterial agents, anti-fungal agents, anti-inflammatory agents, anesthetic agents, agents modulating cutaneous differentiation, pigmentation or de-pigmentation, agents stimulating nail or hair growth.

In some embodiments, each of the aforementioned additives/active ingredients is generally present in an amount of between about 0.1 and 30 wt% of the total weight of the formulation.

Suitable emollients for use in a cosmetic/cleansing formulation according to the invention include, for example, optionally hydroxy-substituted C₈-C₅₀ unsaturated fatty acids and esters thereof, C₁-C₂₄ esters of C₈-C₃₀ saturated fatty acids such as isopropyl myristate, cetyl palmitate and octyldodecylmyristate (Wickenol 142), beeswax, saturated and unsaturated fatty alcohols such as behenyl alcohol and cetyl alcohol, hydrocarbons such as mineral oils, petrolatum, squalane, fatty sorbitan esters, lanolin and lanolin derivatives, such as lanolin alcohol ethoxylated, hydroxylated and acetylated lanolins, cholesterol and derivatives thereof, animal and vegetable triglycerides such as almond oil, peanut oil, wheat germ oil, linseed oil, jojoba oil, oil of apricot pits, walnuts, palm nuts, pistachio nuts, sesame seeds, rapeseed, cade oil, corn oil, peach pit oil, poppy seed oil, pine oil, castor oil, soybean oil, avocado oil, safflower oil, coconut oil, hazelnut oil, olive oil, grape seed oil, and sunflower seed oil and C₁-C₂₄ esters of dimer and trimer acids such as diisopropyl dimerate, diisostearylmalate, diisostearyldimerate and triisostearyltrimerate.

In some embodiments, the emollients used in a formulation according to the invention include isocetyl alcohol, octyl palmitate, isostearyl neopentanoate and isocetyl stearyl stearate, natural or synthetic oils selected from mineral, vegetable, and animal oils, fats and waxes, fatty acid esters, fatty alcohols, alkylene glycol and polyalkylene glycol ethers and esters, fatty acids and mixtures thereof.

The emollients may be used independently or in mixtures and may be present in the composition of the present invention in an amount from about 1 to about 98% by weight, and in some embodiments are present in an amount from about 5% to about 15% by weight of the total formulation.

Suitable emulsifiers for use in a cosmetic/cleansing formulation according to the present invention include glyceryl stearate and laureth 23, PEG 20 stearate, and mink-amidopropyl dimethyl 2-hydroxyethylammonium chloride.

Typical moisturizers are glycerin, petrolatum and maleated vegetable oil.

The formulation of the invention may also contain a hydrophilic gelling agent. In some embodiments, the gelling agent is selected amongst such having a viscosity (1% aqueous solution, 20°C., Brookfield RVT) of at least about 4000 mPa. According to other embodiments, the gelling agent has a viscosity of about 10,000 mPa or at least 50,000 mPa.

In other embodiments, the hydrophilic gelling agents are selected from water-soluble or colloidal water-soluble polymers, such as cellulose ethers (e.g., hydroxyethyl cellulose, methyl cellulose, hydroxypropylmethyl cellulose), polyvinylalcohol, polyquaternium-10, guar gum, hydroxypropyl guar gum, xanthan gum, Aloe vera gel, amla, carrageenan, oat flour, starch (from corn rice or other plants), gelatin (porcine skin), ghatty gum, gum Arabic, inulin (from chicory), Konjac gum, locust bean gum, marshmallow root, pectin, quinoa extract, red alga, solagum and tragacanth gum (TG).

In further embodiments, the hydrophilic gelling agents are selected amongst acrylic acid/ethyl acrylate copolymers and the carboxyvinyl polymers sold by the B.F. Goodrich Company under the trademark of Carbopol resins. These resins consist essentially of a colloidal water-soluble polyalkenyl polyether crosslinked polymer of acrylic acid crosslinked with from 0.75% to 2.00% of a crosslinking agent such as polyallyl sucrose or polyallyl pentaerythritol. Examples include Carbopol 934, Carbopol 940, Carbopol 950, Carbopol 980, Carbopol 951 and Carbopol 981. Carbopol 934 is a water-soluble polymer of acrylic acid crosslinked with about 1 of polyallyl ether of sucrose having an average of about 5.8 allyl groups for each sucrose molecule. Also suitable for use herein are hydrophobically-modified crosslinked polymers of acrylic acid having amphipathic properties available under the Trade Name Carbopol 1382, Carbopol 1342 and Pemulen TR-1. A combination of the polyalkenyl polyether cross-linked acrylic acid polymer and the hydrophobically modified crosslinked acrylic acid polymer is also suitable for use herein.

Other suitable gelling agents suitable for use herein are oleogels such as trihydroxystearin and aluminum magnesium hydroxy stearate.

In some embodiments, the gelling agent is present in the cosmetic/cleansing formulation in an amount from about 0.01% to about 10% of the total weight of the formulation. In some embodiments, the formulation comprises a hydrophilic gelling agent in an amount between about 0.02% to about 2%. In other embodiments, the amount of the gelling agent is from about 0.02% to about 0.5%.

The cosmetic/cleansing formulation may also comprise a thickener, such as crosslinked maleic anhydride-alkyl methylvinylethers, and copolymers, commercially available as Stabilizes QM (International Specialty Products (ISP)), Carbomer, natural gums, highly crosslinked polymethacrylate copolymer, such as Microspongess 5647, which take the form of generally spherical particles of crosslinked hydrophobic polymer having a pore size of from about 0.01 to about 0.05 µm and a surface area of 200-300 m²/g.

Neutralizing agents suitable for use in a cosmetic/cleansing formulation of the invention include neutralizing acidic group containing hydrophilic gelling agents, as listed herein, sodium hydroxide, potassium hydroxide, ammonium hydroxide, monoethanolamine, diethanolamine and triethanolamine and aminomethyl propanol.

In some embodiments, the cosmetic/cleansing formulation comprises one or more ultraviolet absorbing agents. Ultraviolet absorbing agents, often described as sun screening agents, may be present in a concentration between about 1% and about 25% by weight, based on the total weight of composition. According to some embodiments of the invention, the UV absorbing agent constitutes between about 2% and 15% by weight. According to other embodiments, the UV absorbing agent constitutes between about 4% and about 10% by weight. Non-limiting examples of ultraviolet absorbing agents include benzophenone-3, benzophenone-4, octyl dimethyl PABA (Padimate 0), octyl methoxy cinnamate, octyl salicylate, octocrylene, p-methylbenzylidene camphor, butyl methoxy dibenzoyl methane (Parsol 1789), titanium dioxide, zinc oxide and mixtures thereof.

In a further aspect, the invention provides an antimicrobial formulation comprising the compositions disclosed herein.

The antimicrobial formulation of the invention is effective in reducing or eliminating a microorganism population or a biofilm of such microorganisms. As demonstrated herein, the formulations of the invention provide instant and persistent antimicrobial activity against a wide spectrum of microorganisms and specifically against a broad spectrum of bacteria. The term ***microorganism*** relates herein to a single cell (unicellular), cell clusters, or no cell (acellular) organism such as bacteria, fungi, yeast, mold, archaea, protists, viruses and algae.

In some embodiments, the microorganism is a bacteria, being selected, in some embodiments from *Bordetella pertussis, Borrelia burgdorferi, Brucella abortus, Brucella canis, Brucella melitensis, Brucella suis, Campylobacter jejuni, Chlamydia pneumonia, Chlamydia psittaci, Chlamydia trachomatis, Clostridium botulinum, Clostridium difficile, Clostridium perfringens, Clostridium tetani, Corynebacterium diphtheria, Enterococcus faecalis, Enterococcus faecium, Escherichia coli* (E. coli), *Enterotoxigenic Escherichia coli* (ETEC), *Enteropathogenic E. coli, Francisella tularensis, Haemophilus influenza, Helicobacter pylori, Legionella pneumophila, Leptospira interrogans, Listeria monocytogenes, Mycobacterium leprae, Mycobacterium tuberculosis, Mycoplasma pneumonia, Neisseria gonorrhoeae, Neisseria meningitidis, Pseudomonas aeruginosa, Rickettsia rickettsii, Salmonella typhi, Salmonella typhimurium, Shigella sonnei, Staphylococcus epidermidis, Staphylococcus saprophyticus, Streptococcus agalactiae, Streptococcus mutans Streptococcus pneumonia, Streptococcus pyogenes, Treponema pallidum, Vibrio cholera, Vibrio harveyi* and *Yersinia pestis.*

In some embodiments, the microorganism is a fungus, selected in some embodiments from *Absidia corymbifera, Ajellomyces capsulatus, Ajellomyces dermatitidis, Arthroderma benhamiae, Arthroderma fulvum, Arthroderma gypseum, Arthroderma incurvatum, Arthroderma otae, Arthroderma vanbreuseghemii, Aspergillus flavus, Aspergillus fumigates, Aspergillus niger (Aspergillus brasiliensis), Blastomyces dermatitidis, Candida albicans, Candida albicans var. stellatoidea, Candida dublinensis, Candida glabrata, Candida guilliermondii, Candida krusei, Candida parapsilosis, Candida pelliculosa, Candida tropicalis, Cladophialophora carrionii, Coccidioides immitis, Cryptococcus neoformans, Cunninghamella sp., Epidermophyton floccosum, Exophiala dermatitidis, Filobasidiella neoformans, Fonsecaea pedrosoi, Geotrichum candidum, Histoplasma capsulatum, Hortaea werneckii, Issatschenkia orientalis, Madurella grisae, Malassezia furfur, Malassezia furfur complex, Malassezia globosa, Malassezia obtuse, Malassezia pachydermatis, Malassezia restricta, Malassezia slooffiae, Malassezia sympodialis, Microsporum canis, Microsporum fulvum, Microsporum gypseum, Microsporum gypseum complex, Microsporum gypseum, Mucor circinelloides, Nectria haematococca, Paecilomyces variotii, Paracoccidioides brasiliensis, Penicillium marneffei, Phialophora verrucosa, Pichia anomala, Pichia guilliermondii, Pneumocystis jirovecii, Pseudallescheria boydii, Rhizopus oryzae, Rodotorula rubra, Saccharomyces cerevisiae, Scedosporium apiospermum, Schizophyllum commune, Sporothrix schenckii, Stachybotrys chartarum, Trichophyton mentagrophytes, Trichophyton mentagrophytes complex, Trichophyton mentagrophytes, Trichophyton mentagrophytes, Trichophyton rubrum, Trichophyton tonsurans, Trichophyton verrucosum, Trichophyton violaceum, Trichosporon asahii, Trichosporon cutaneum, Trichosporon cutaneum complex, Trichosporon inkin* and *Trichosporon mucoides.*

In some embodiments, the microorganism is yeast, being selected, in some embodiments, from *Candida albicans, Candida albicans var. stellatoidea, Candida dublinensis, Candida glabrata, Candida guilliermondii, Candida krusei, Candida parapsilosis, Candida pelliculosa, Candida tropicalis, Cryptococcus neoformans, Filobasidiella neoformans, Geotrichum candidum, Issatschenkia orientalis, Malassezia furfur, Malassezia pachydermatis, Pichia anomala, Pichia guilliermondii, Pneumocystis jirovecii, Rodotorula rubra, Trichosporon asahii, Trichosporon cutaneum, Trichosporon inkin* and *Trichosporon mucoides.*

In some embodiments, the microorganism is mold, being selected, in some embodiments, from *Absidia corymbifera, Arthroderma benhamiae, Arthrodermafulvum, Arthroderma gypseum, Arthroderma incurvatum, Arthroderma otae, Arthroderma vanbreuseghemii, Aspergillus flavus, Aspergillus fumigates, Aspergillus niger, Cladophialophora carrionii, Coccidioides immitis, Epidermophyton floccosum, Exophiala dermatitidis, Fonsecaea pedrosoi, Hortaea werneckii, Madurella grisae, Microsporum canis, Microsporum fulvum, Microsporum gypseum, Microsporum gypseum, Microsporum gypseum, Mucor circinelloides, Nectria haematococca, Paecilomyces variotii, Paracoccidioides brasiliensis, Penicillium marneffei, Pseudallescheria boydii, Rhizopus oryzae, Scedosporium apiospermum, Schizophyllum commune, Sporothrix schenckii, Stachybotrys chartarum, Trichophyton mentagrophytes complex, Trichophyton mentagrophytes, Trichophyton mentagrophytes, Trichophyton rubrum, Trichophyton tonsurans, Trichophyton verrucosum* and *Trichophyton violaceum.*

According to some embodiments of the invention, the antimicrobial formulations of the invention are effective against bacteria such as *Escherichia coli (E. Coli), Salmonella, Staphylococcus, Saccharomyces, Staphylococcus aureus, Pseudomonas aeruginosa, Candida albicans,* or *A. niger.*

In a further aspect, the invention provides a therapeutic formulation (pharmaceutical composition) comprising the compositions of the present disclosure as described herein.

The pharmaceutical formulation of the invention may be effective in the treatment and/or prevention of a variety of diseases and disorders. As demonstrated hereinbelow, the formulations of the invention provide instant and persistent antimicrobial activity against a wide spectrum of microorganisms, as defined herein. In some embodiments, the disease or disorder to be treated is associated with bacterial infection, fungal infection or viral infection.

Non-limiting examples of disease or disorder associated with a bacterial infection include lyme disease, brucellosis, acute enteritis, psittacosis, nongonococcal urethritis (NGU), trachoma, inclusion conjunctivitis of the newborn (ICN), lymphogranuloma venereum (LGV), botulism, pseudomembranous colitis, gas gangrene, acute food poisoning, anaerobic cellulitis, tetanus, diphtheria, nosocomial infections, urinary tract infections (UTI), diarrhea, meningitis, meningitis in infants, hemorrhagic colitis, hemolytic-uremic syndrome, tularemia, upper respiratory tract infections, pneumonia, mycoplasma pneumonia, secondary pneumonia, bronchitis, peptic ulcer, legionnaire's disease, gastric B-cell lymphoma, pontiac fever, leptospirosis, listeriosis, leprosy (Hansens disease), tuberculosis, gonorrhea, ophthalmia neonatorum, meningococcal disease, Waterhouse-Friderichsen, localized infection (of eye, ear, skin, urinary, respiratory), gastrointestinal tract infection, central nervous system infection, systemic infection with bacteremia, bone and joint infections, endocarditis, typhoid fever type salmonellosis, dysentery, colitis, salmonellosis with gastroenteritis and enterocolitis, bacillary dysentery/shigellosis, Streptococcal pharyngitis, Scarlet fever, rheumatic fever, impetigo and erysipelas, puerperal fever, necrotizing fasciitis, syphilis, congenital syphilis and cholera.

In some embodiments, the bacterial disease or disorder is associated with *Staphylococcus* or *Escherichia coli* (E. coli) or *salmonella* infections; the disease or disorder being selected from:
*Staphylococcus:* coagulase-positive staphylococcal infections such as Localized skin infections, diffuse skin infection (impetigo), deep and localized infections, acute infective endocarditis, septicemia, necrotizing pneumonia, toxinoses, toxic shock syndrome, staphylococcal food poisoning, infections of implanted prostheses e.g., heart valves and catheters and cystitis in women;
*E. coli:* urinary tract infections (UTI), diarrhea, meningitis in infants, traveler's diarrhea, hemorrhagic colitis and hemolytic-uremic syndrome;
*Salmonella:* typhoid fever type salmonellosis, dysentery, colitis, salmonellosis, e.g., with gastroenteritis and enterocolitis.

In some embodiments, the pharmaceutical composition of the invention is used in the treatment or prevention of a disease or disorder associated with a fungal infection. In some embodiments, the pathogen is yeast. In some other embodiments, the pathogen is mold.

The pharmaceutical composition may be adapted for administration by a variety of routes including topical, oral, rectal, vaginal, transdermal, subcutaneous, intravenous, intramuscular, eye drops and intranasal. Such pharmaceutical composition is prepared in a manner well known in the pharmaceutical art. In making the pharmaceutical composition of the invention, the aforementioned components are usually mixed with an excipient, diluted by an excipient or enclosed within such a carrier which can be manipulated to the desired form. Based on the particular mode of administration, the pharmaceutical composition may be formulated into tablets, pills, capsules, sachets, granules, powders, chewing gum, suspensions, emulsions and solutions.

The pharmaceutically acceptable carriers, for example, vehicles, adjuvants, excipients, or diluents, are well-known to those who are skilled in the art and are readily available to the public. It is preferred that the pharmaceutically acceptable carrier be one which is chemically inert to the active formulation and each of its components and one which has no detrimental side effects or toxicity under the conditions of use.

The choice of carrier will be determined in part by the particular formulation of the invention, as well as by the particular method used to administer the composition. Accordingly, there is a wide variety of suitable formulations of the pharmaceutical composition of the present invention.

Formulations suitable for oral administration can consist of (a) liquid solutions, such as an effective amount of the compound, or composition comprising same, dissolved in diluents, such as water, saline, or juice (e.g. orange juice); (b) capsules, sachets, tablets, lozenges, and troches, each containing a predetermined amount of the active ingredient, as solids or granules; (c) powders; (d) suspensions in an appropriate liquid; and (e) suitable emulsions. Liquid formulations may include diluents, such as water and alcohols, for example, ethanol, benzyl alcohol, and the polyethylene alcohols, either with or without the addition of a pharmaceutically acceptable surfactant, suspending agent, or emulsifying agent. Capsule forms can be of the ordinary hard- or soft-shelled gelatin type containing, for example, surfactants, lubricants, and inert fillers, such as lactose, sucrose, calcium phosphate, and corn starch. Tablet forms can include one or more of lactose, sucrose, mannitol, corn starch, potato starch, alginic acid, microcrystalline cellulose, acacia, gelatin, guar gum, colloidal silicon dioxide, talc, magnesium stearate, calcium stearate, zinc stearate, stearic acid, and other excipients, colorants, diluents, buffering agents, disintegrating agents, moistening agents, preservatives, flavoring agents, and pharmacologically compatible carriers. Lozenge forms can comprise the active ingredient in a flavor, usually sucrose and acacia or tragacanth, as well as pastilles comprising the active formulation in an inert base, such as gelatin and glycerin, or sucrose and acacia, emulsions, gels, and the like containing, in addition to the active formulation, such carriers as are known in the art.

The formulations of the present invention, alone or in combination with other suitable components, e.g., active or non-active additives/ingredients can be made into aerosol formulations to be administered via inhalation. These aerosol formulations can be placed into pressurized acceptable propellants, such as dichlorodifluoromethane, propane, nitrogen, and the like. They also may be formulated as pharmaceuticals for non-pressured preparations, such as in a nebulizer or an atomizer

Formulations suitable for parenteral administration include aqueous and non-aqueous, isotonic sterile injection solutions, which can contain anti-oxidants, buffers, bacteriostats, and solutes that render the formulation isotonic with the blood of the intended recipient, and aqueous and non-aqueous sterile suspensions that include suspending agents, solubilizers, thickening agents, stabilizers, and preservatives. The formulation can be administered in a physiologically acceptable diluent in a pharmaceutical carrier, such as a sterile liquid or mixture of liquids, including water, saline, aqueous dextrose and related sugar solutions, an alcohol, such as ethanol, isopropanol, or hexadecyl alcohol, glycols, such as propylene glycol or polyethylene glycol, glycerol ketals, such as 2,2-dimethyl-1,3-dioxolane-4-methanol, ethers, such as poly(ethyleneglycol) 400, an oil, a fatty acid, a fatty acid ester or glyceride, or an acetylated fatty acid glyceride with or without the addition of a pharmaceutically acceptable surfactant, such as a soap or a detergent, suspending agent, such as pectin, carbomers, methylcellulose, hydroxypropylmethylcellulose, or carboxymethylcellulose, or emulsifying agents and other pharmaceutical additives.

Oils, which can be used in parenteral formulations, include petroleum, animal, vegetable, or synthetic oils. Specific examples of oils include peanut, soybean, sesame, cottonseed, corn, olive, petrolatum, and mineral. Suitable fatty acids for use in parenteral formulations include oleic acid, stearic acid, and isostearic acid. Ethyl oleate and isopropyl myristate are examples of suitable fatty acid esters. Suitable soaps for use in parenteral formulations include fatty alkali metal, ammonium, and triethanolamine salts, and suitable detergents include (a) cationic detergents such as, for example, dimethyl dialkyl ammonium halides, and alkyl pyridinium halides, (b) anionic detergents such as, for example, alkyl, aryl, and olefin sulfonates, alkyl, olefin, ether, and monoglyceride sulfates, and sulfosuccinates, (c) nonionic detergents such as, for example, fatty amine oxides, fatty acid alkanolamides, and polyoxy- ethylenepolypropylene copolymers, (d) amphoteric detergents such as, for example, alkyl-β-aminopriopionates, and 2-alkyl-imidazoline quaternary ammonium salts, and (3) mixtures thereof.

In order to minimize or eliminate irritation at the site of injection, such compositions may contain one or more nonionic surfactants having a hydrophile-lipophile balance (HLB) of from about 12 to about 17. The quantity of surfactant in such formulations ranges from about 5 to about 15% by weight. Suitable surfactants include polyethylene sorbitan fatty acid esters, such as sorbitan monooleate and the high molecular weight adducts of ethylene oxide with a hydrophobic base, formed by the condensation of propylene oxide with propylene glycol. The parenteral formulations can be presented in unit-dose or multi-dose sealed containers, such as ampules and vials, and can be stored in a freeze-dried (lyophilized) condition requiring only the addition of the sterile liquid carrier, for example, water, for injections, immediately prior to use. Extemporaneous injection solutions and suspensions can be prepared from sterile powders, granules, and tablets of the kind previously described.

The active formulation is effective over a wide dosage range and may generally be administered in a pharmaceutically effective amount. It should be understood, however, that the amount of the formulation or each component thereof to be administered, will be determined by a physician, in the light of the relevant circumstances, including the condition to be treated, the chosen route of administration, the actual formulation, the age, weight, and response of the individual patient, the severity of the patient's symptoms, and the like.

In another aspect of the invention, there is provided the use of a formulation of the invention as herein defined, for the preparation of a pharmaceutical composition for treating or preventing a disease or disorder in a mammal (human or non-human).

In another aspect of the invention, there is provided the use of a topical formulation of the invention as herein defined, for the preparation of a pharmaceutical composition for treating or preventing a disease or disorder in a mammal (human or non-human).

In some embodiments, the disease or disorder is associated with a bacteria, virus, fungus, yeast or mold.

As used herein, the term ***treatment*** or any lingual variation thereof, refers to the administering of a therapeutic amount of the composition of the present invention which is effective to ameliorate undesired symptoms associated with a disease, to prevent the manifestation of such symptoms before they occur, to slow down the progression of the disease, slow down the deterioration of symptoms, to enhance the onset of remission period, slow down the irreversible damage caused in the progressive chronic stage of the disease, to delay the onset of said progressive stage, to lessen the severity or cure the disease, to improve survival rate or more rapid recovery, or to prevent the disease form occurring or a combination of two or more of the above. The ***effective amount*** for purposes disclosed herein is determined by such considerations as may be known in the art. The amount must be effective to achieve the desired therapeutic effect as described above, depending, *inter alia,* on the type and severity of the disease to be treated and the treatment regime. The effective amount is typically determined in appropriately designed clinical trials (dose range studies) and the person versed in the art will know how to properly conduct such trials in order to determine the effective amount. As generally known, an effective amount depends on a variety of factors including the affinity of the ligand to the receptor, its distribution profile within the body, a variety of pharmacological parameters such as half life in the body, on undesired side effects, if any, on factors such as age and gender, etc.

In yet another aspect, the invention provides a preservative formulation comprising the compositions of the disclosure as described herein.

The preservative formulation of the invention may be used to reduce, inhibit or completely eliminate pathogen population in a variety of consumer products, such as personal care products, industrial products, food products, therapeutics, and others. As demonstrated herein, the formulation of the invention may be used to replace currently available chemicals which are used as preservatives, some of which known as toxic to humans and animals, or at reduce their concentration in such products for human or animal use. The preservative formulation may be added to any such product, such as cosmetics and toiletries in aqueous or hydroalcoholic solution, oil-in-water or water-in-oil emulsion, aqueous or anhydrous gels, cream, ointment, lotion, gel, solution and suspension; therapeutics and over-the-counter pharmaceutical products, water-based paints, cutting oils, latex solutions, food products such as beverages, frozen foods, candy and canned products.

In some embodiments, the formulation of the invention is an antimicrobial preservative, attesting to the ability of the formulations of the invention to suppress microbial growth, reduce microbial infestation, treat products or surfaces to improve product resistance to microbial infestation, reduce biofilm, prevent conversion of bacteria to biofilm, prevent or inhibit microbial infection, prevent spoilage, retard or minimize or prevent quorum sensing, and retard microbial reproduction. Typically, the preservative formulation according to the invention comprises the saponin and plant extract(s) at a concentration which suffices to prevent spoilage or growth of microorganisms, thereby extending the shelf- or useful-life of the product.

The formulation of the invention may also be employed as a disinfectant or bacteriocide agent. The formulations of the invention may be applied onto a surface to be disinfected, including human or animal skin, by various means including by washing, spraying, wiping, etc.

As used herein, the term ***about*** is meant to encompass deviation of ±10% from the specifically mentioned value of a parameter, such as temperature, pressure, concentration, etc.

Whenever a numerical range is indicated herein, it is meant to include any cited numeral (fractional or integral) within the indicated range. The phrases *"****ranging*/*ranges between"*** a first indicate number and a second indicate number and "ranging/ranges from" a first indicate number "to" a second indicate number are used herein interchangeably and are meant to include the first and second indicated numbers and all the fractional and integral numerals therebetween.

### DETAILED DESCRIPTION OF EMBODIMENTS

### Example 1: Composition of Styrax tonkinensis (Siam benzoin) and saponin

### Extraction of Styrax tonkinensis by oil:

1g of ground *Styrax tonkinensis* resin was dissolved in 10 ml of MCT (middle chain triglyceride) coconut-origin oil, in a water bath at 90°C. The resulting solution was filtered via a PTFE filter, 0.45 microns.

Although some material loss during filtration was observed (un-dissolved compounds and oil), the sample's concentration was estimated to be 10 wt%.

### Preparation of saponin-Benzoin tonkinensis formulation:

1g of *Sapindus mukorossi* extract powder was dissolved in 10 ml of distilled water to provide the saponin extract. The *Sapindus mukorossi* extract (SN) and/or *Styrax tonkinensis* extract (BZ-2) was added to 25g a non-preserved lotion in sterilized conditions and mixed thoroughly. Various concentrations of *Sapindus mukorossi* extract and/or *Styrax tonkinensis* extract were tested as follows.

### Demonstrated effect in a challenge test

The various lotion or cleansing formulae were contaminated by suitable microorganisms (bacteria and yeast), as described below. The so-prepared samples were incubated as described below. Using serial dilutions and plate counts, aliquots were taken during the incubation period for determining microorganism count.

Tested Microorganisms: *E. coli* (ATCC 8739); S. *aureus* (ATCC 6538); *P. aeruginosa (ATCC 9027); C. albicans (ATCC 10231) and A. Brasiliensis (ATCC 16404)).* Bacteria, yeast, and mold suspensions were prepared according to ISO 11930; TSA culture medium for the bacteria was obtained from Hylab (Cat. No PD-087/A); PDA culture medium for A. brasiliensis was obtained from Hylab (Cat. No PD-043); SDA culture medium for C. albicans was obtained from Hylab (Cat. No PD-044); 0.1% peptone was prepared by mixing 1 g/l Peptone in sterile water. The test was performed according ISO 11930. Each sample was contaminated with microorganisms to obtain the range of 10⁵-10⁶ cfu/ml. The inoculated formulation was mixed thoroughly to ensure a homogeneous distribution of the inoculum.

Samples were stored at 25°C. Each sample was sampled in intervals of 2, 7, 14, 21 and 28 days, according to the following procedure: 100µl of the inoculated formulation was streaked on suitable agar medium. The plates were incubated at 37°C for bacteria and 25°C for yeast and mold. After incubation, the colonies were enumerated. *Sapindus mukorossi* extract (SN) and *Styrax tonkinensis* extract (BZ-2) were used

Sharomix, a liquid bland of methylparaben, ethylparaben, propylparaben, propylene glycol in phenoxyethanol, was used as a negative control.

Test results are provided in **Table 1.**

**Table 1: Total count for different microorganisms after contact with the examined ingredients (used a non-preserved lotion)**

| **Formula** | **Tested organism** | **1^{st} inoculation** | | **2^{nd} inoculation, 14 days** | **3^{rd} inoculation** | |
|---|---|---|---|---|---|---|
| | | **2 days** | **7 days** | | **21 days** | **28 days** |
| BZ-**2** 0.2 wt% | *A. brasiliensis* | 2.4×10⁴ | Full | Full | Full | Full |
| | *C. albicans* | Full | Full | Full | Full | Full |
| | *P. aeruginosa* | 0 | 0 | 0 | 50 | 80 |
| | *E. coli* | 0 | 10 | 0 | 0 | 0 |
| | *S. aureus* | 0 | 0 | 0 | 0 | 0 |
| SN-**2** 0.2 wt% | *A. brasiliensis* | 1.4×10⁴ | Full | Full | Full | Full |
| | *C. albicans* | 0 | 0 | 0 | 0 | 0 |
| | *P. aeruginosa* | Full | Full | Full | Full | Full |
| | *E. coli* | 500 | 0 | 0 | 0 | 0 |
| | *S. aureus* | 12×10⁴ | 0 | 0 | Full | Full |
| BZ-**2** 0.2 wt% + SN 0.2 wt% | *A. brasiliensis* | 1.6×10⁴ | Full | 1.6×10⁴ | 5.2×10⁴ | 5.2×10⁴ |
| | *C. albicans* | 600 | 10 | 0 | 0 | 0 |
| | *P. aeruginosa* | 0 | 0 | 0 | 0 | 0 |
| | *E. coli* | 0 | 0 | 0 | 10 | 0 |
| | *S. aureus* | 0 | 0 | 0 | 0 | 0 |
| Lotion + bacteria (positive control) | *A. brasiliensis* | Full | Full | Full | Full | Full |
| | *C. albicans* | Full | Full | Full | Full | Full |
| | *P. aeruginosa* | 2×10⁴ | Full | Full | Full | Full |
| | *E. coli* | 1.3×10³ | 0 | 0 | 0 | 0 |
| | *S. aureus* | 5.5×10³ | 0 | 20 | 8000 | 8700 |
| Sharomix (negative control) | *A. brasiliensis* | 0 | 0 | 0 | 0 | 0 |
| | *C. albicans* | 0 | 0 | 0 | 0 | 0 |
| | *P. aeruginosa* | 0 | 0 | 0 | 0 | 0 |
| | *E. coli* | 0 | 0 | 0 | 0 | 0 |
| | *S. aureus* | 0 | 0 | 0 | 0 | 0 |
| Lotion uncontaminated | - | 0 | 0 | 0 | 0 | 0 |

As can be seen from **Table 1,** the SN extract showed activity against *Candida, E. coli* and *Staphylococcus,* while the BZ-2 extract showed activity against all types of tested bacteria except for mold.

The SN-BZ-2 formulations showed significant activity for all tested microorganisms, killing all colonies except for the mold. It is of note, however that amount of mold dropped by two orders of magnitude when incubated for at least 14 days with the SN-BZ-2 formulation. Besides killing or reducing growth, the mixture prevented re-growth for additional two to three weeks.

### Example 2: Antimicrobial Effectiveness Testing - concentration changing

The test was conducted on 25 g samples of a lotion formulation comprising different concentrations of SN and or BZ-2. All formulas included a mixture of BZ-2+SN at various concentrations. *Sapindus mukorossi* extract (SN) and *Styrax tonkinensis* extract (BZ-2) were used.

Each sample was separately inoculated by one of the five test organisms. The inoculated containers were incubated in 25°C together with an un-inoculated sample. The number of surviving microorganisms was monitored periodically during an incubation of 3 weeks and the colony forming units (CFU) were counted. The added ingredient, its concentration and the microorganisms' counts appear in **Table 2.**

**Table 2: Total count for different microorganisms after contact with different conc. of the examined ingredients**

| **Tested Formula** | **Tested Organism** | **After 2 days** | **After 7 days** | **After 14 days** | **After 21 days** | **After 28 days** |
|---|---|---|---|---|---|---|
| SN 0.7 wt% + BZ-2 0.2 wt% | *A. brasiliensis* | 2.0×10⁴ | 2.0×10⁴ | 2.0×10⁴ | 2.0×10⁴ | 2.0×10⁴ |
| | *C. albicans* | 0 | 0 | 0 | 0 | 0 |
| | *P. aeruginosa* | 0 | 0 | 0 | 0 | 0 |
| | *E. coli* | 0 | 0 | 0 | 0 | 0 |
| | *S. aureus* | 0 | 0 | 0 | 0 | 0 |
| SN 0.5 wt% + BZ-2 0.3 wt% | *A. brasiliensis* | 2.0×10⁴ | 6.0×10³ | 3.4×10³ | 3.4×10³ | 3.4×10³ |
| | *C. albicans* | 300 | 10 | 0 | 0 | 0 |
| | *P. aeruginosa* | 0 | 0 | 0 | 0 | 0 |
| | *E. coli* | 0 | 0 | 0 | 0 | 0 |
| | *S. aureus* | 0 | 0 | 0 | 0 | 0 |
| SN 0.2 wt% + BZ-2 0.2 wt% | *A. brasiliensis* | 5.2×10⁴ | 1.6×10⁴ | 6.0×10³ | Full | Full |
| | *C. albicans* | 100 | 20 | 0 | 0 | 0 |
| | *P. aeruginosa* | 0 | 0 | 0 | 0 | 0 |
| | *E. coli* | 0 | 0 | 0 | 0 | 0 |
| | *S. aureus* | 0 | 0 | 0 | 0 | 0 |
| SN 0.2 wt% + BZ-2 0.1 wt% | *A. brasiliensis* | 1.4×10⁴ | 1.1×10⁴ | 2.0×10⁴ | 2.0×10⁴ | 2.3×10⁴ |
| | *C. albicans* | 280 | 10 | 0 | 0 | 0 |
| | *P. aeruginosa* | 0 | 0 | 0 | 0 | 0 |
| | *E. coli* | 0 | 0 | 0 | 0 | 0 |
| | *S. aureus* | 0 | 0 | 0 | 0 | 0 |
| SN 0.1 wt% + BZ-2 0.2 wt% | *A. brasiliensis* | 2.6×10⁴ | 1.6×10⁴ | 1.6×10⁴ | 1.8×10⁴ | 1.8×10⁴ |
| | *C. albicans* | 100 | 0 | 0 | 0 | 0 |
| | *P. aeruginosa* | 0 | 0 | 0 | 0 | 0 |
| | *E. coli* | 0 | 0 | 0 | 0 | 0 |
| | *S. aureus* | 0 | 0 | 0 | 0 | 0 |
| SN 0.2 wt% + BZ-2 0.3 wt% | *A. brasiliensis* | 3.4×10⁴ | 1.7×10⁴ | 1.0×10⁴ | Full | Full |
| | *C. albicans* | 0 | 0 | 0 | 0 | 0 |
| | *P. aeruginosa* | 0 | 0 | 0 | 0 | 0 |
| | *E. coli* | 0 | 0 | 0 | 0 | 0 |
| | *S. aureus* | 0 | 0 | 0 | 0 | 0 |
| SN 0.2 wt% + BZ-2 0.5 wt% | *A. brasiliensis* | 1.3×10⁴ | 6.4×10⁴ | 9.0×10³ | 9.0×10³ | 92×10³ |
| | *C. albicans* | 400 | 10 | 0 | 0 | 0 |
| | *P. aeruginosa* | 0 | 0 | 0 | 0 | 0 |
| | *E. coli* | 0 | 0 | 0 | 0 | 0 |
| | *S. aureus* | 0 | 0 | 0 | 0 | 0 |
| lotion + bacteria (positive control) | *A. brasiliensis* | Full | Full | Full | Full | Full |
| | *C. albicans* | Full | Full | Full | Full | Full |
| | *P. aeruginosa* | 2×10⁴ | Full | Full | Full | Full |
| | *E. coli* | 1.3×10³ | 0 | 0 | 0 | 0 |
| | *S. aureus* | 5.5×10³ | 0 | 20 | 8000 | 9700 |
| Sharomix (negative control) | *A. brasiliensis* | 0 | 0 | 0 | 0 | 0 |
| | *C. albicans* | 0 | 0 | 0 | 0 | 0 |
| | *P. aeruginosa* | 0 | 0 | 0 | 0 | 0 |
| | *E. coli* | 0 | 0 | 0 | 0 | 0 |
| | *S. aureus* | 0 | 0 | 0 | 0 | 0 |
| Lotion (uncontaminated) | | 0 | 0 | 0 | 0 | 0 |

As can be seen from **Table 2,** all the microorganisms were killed except for the mold, for which a count drop of 1-2 orders of magnitudes was observed. Besides killing or reducing growth, the mixture prevented re-growth for additional two to three weeks.

### Example 3: Composition with Benzoin from various species and saponin

10 wt% in MCT extracts of *Styrax paralleloneurus* (Sumatra benzoin) and *Styrax tonkinensis* (Siam benzoin) were prepared according to the method described in Example 1.

1g of *Sapindus mukorossi* extract powder was dissolved in 10 ml of distilled water to provide the saponin extract. The *Sapindus mukorossi* extract (SN), Sumatra benzoin (BZ-1) and Siam benzoin (BZ-2) were added to a 25g non-preserved cream in sterilized conditions and mixed thoroughly. Various concentrations of *Sapindus mukorossi* extract and/or benzoin extract were tested in accordance with the procedure of the challenge test of Example 1. The results are provided in **Table 3.**

Tested Microorganisms: *E. coli* (ATCC 8739); S. *aureus* (ATCC 6538); *P. aeruginosa (ATCC 9027); C. albicans (ATCC 10231) and A. Brasiliensis (ATCC 16404).*

**Table 3: Total count for different microorganisms after contact with the examined ingredients**

| **Tested Formula** | **Tested Organism** | **Initial inoculums level (cfu/ml)** | **2 days** | **7 days** | **14 days** | **28 days** |
|---|---|---|---|---|---|---|
| BZ-10.1wt% | *A. brasiliensis* | 4×10⁵ | 4×10⁴ | Full | 2.6×10⁴ | 3.5×10³ |
| | *C. albicans* | 1.4×10⁵ | Full | Full | Full | 7.5×10³ |
| | *P. aeruginosa* | 3×10⁶ | Full | Full | Full | Full |
| | *E. coli* | 1×10⁶ | 1×10³ | 0 | 0 | 0 |
| | *S. aureus* | 1×10⁶ | Full | 1.2×10⁴ | 3×10³ | 1×10¹ |
| BZ-1 0.2 wt% | *A. brasiliensis* | 4×10⁵ | 32×10⁴ | 12×10⁴ | 1.3×10⁴ | 2.4×10³ |
| | *C. albicans* | 1.4×10⁵ | Full | Full | Full | 1.3×10³ |
| | *P. aeruginosa* | 3×10⁶ | Full | Full | Full | Full |
| | *E. coli* | 1×10⁶ | 6×10² | 1×10¹ | 0 | 0 |
| | *S. aureus* | 1×10⁶ | Full | Full | 2.5×10³ | 0 |
| BZ-1 0.3 wt% | *A. brasiliensis* | 4×10⁵ | 2×10⁴ | 4×10⁴ | 1.5×10⁴ | 7.2×10³ |
| | *C. albicans* | 1.4×10⁵ | Full | Full | Full | 2.3×10³ |
| | *P. aeruginosa* | 3×10⁶ | Full | Full | Full | Full |
| | *E. coli* | 1×10⁶ | 8×10² | 0 | 0 | 0 |
| | *S. aureus* | 1×10⁶ | Full | Full | 3×10³ | 0 |
| BZ-1 0.5 wt% | *A. brasiliensis* | 4×10⁵ | 3.2×10⁴ | 1×10⁴ | 9×10³ | 3.8×10³ |
| | *C. albicans* | 1.4×10⁵ | Full | Full | 1.8×10⁴ | 2.3×10⁴ |
| | *P. aeruginosa* | 3×10⁶ | Full | Full | Full | Full |
| | *E. coli* | 1×10⁶ | 5×10² | Full | 0 | 0 |
| | *S. aureus* | 1×10⁶ | Full | 5×10³ | 3×10⁶ | 0 |
| SN 0.2 wt% + BZ-1 0.1 wt% | *A. brasiliensis* | 4×10⁵ | 1.3×10⁴ | 4×10³ | 3.5×10³ | 3.1×10³ |
| | *C. albicans* | 1.4×10⁵ | 0 | 0 | 0 | 0 |
| | *P. aeruginosa* | 3×10⁶ | Full | Full | Full | Full |
| | *E. coli* | 1×10⁶ | 3×10⁶ | 0 | 0 | 0 |
| | *S. aureus* | 1×10⁶ | 4×10⁴ | 1.7×10⁴ | 1.4×10³ | 0 |
| SN 0.2 wt% + BZ-1 0.2 wt% | *A. brasiliensis* | 4×10⁵ | 1.1×10⁴ | 3×10³ | 3×10³ | 4.8×10² |
| | *C. albicans* | 1.4×10⁵ | 0 | 1×10¹ | 0 | 0 |
| | *P. aeruginosa* | 3×10⁶ | Full | Full | Full | Full |
| | *E. coli* | 1×10⁶ | 4×10² | 0 | 0 | 0 |
| | *S. aureus* | 1×10⁶ | 1.4×10⁵ | 1.6×10⁴ | 1×10³ | 0 |
| SN 0.2 wt% + BZ-1 0.3 wt% | *A. brasiliensis* | 4×10⁵ | 9×10³ | 5×10³ | 1.7×10⁴ | 1.7×10⁴ |
| | *C. albicans* | 1.4×10⁵ | 0 | 0 | Full | 0 |
| | *P. aeruginosa* | 3×10⁶ | Full | Full | 0 | Full |
| | *E. coli* | 1×10⁶ | 1.2×10³ | 0 | 0 | 0 |
| | *S. aureus* | 1×10⁶ | 1.6×10⁴ | 7.8×10³ | 3.8×10² | 0 |
| SN 0.2 wt% + BZ-1 0.5 wt% | *A. brasiliensis* | 4×10⁵ | 8.5×10³ | 3×10³ | 1.3×10³ | 1×10¹ |
| | *C. albicans* | 1.4×10⁵ | 0 | 0 | 0 | 0 |
| | *P. aeruginosa* | 3×10⁶ | Full | Full | Full | Full |
| | *E. coli* | 1×10⁶ | 1.5×10³ | 0 | 0 | 0 |
| | *S. aureus* | 1×10⁶ | 1.6×10⁴ | 6.8×10³ | 2×10² | 0 |
| SN 0.2 wt% + BZ-2 0.1 wt% | *A. brasiliensis* | 4×10⁵ | 1.4×10⁴ | 1.1×10⁴ | 2×10⁴ | 12×10⁴ |
| | *C. albicans* | 1.4×10⁵ | 1×10² | 2×10¹ | 0 | 0 |
| | *P. aeruginosa* | 3×10⁶ | 0 | 0 | 0 | 0 |
| | *E. coli* | 1×10⁶ | 0 | 0 | 0 | 0 |
| | *S. aureus* | 1×10⁶ | 0 | 0 | 0 | 0 |
| SN 0.2 wt% + BZ-2 0.2 wt% | *A. brasiliensis* | 4×10⁵ | 2×10⁴ | 6×10³ | 1×10⁴ | 1.1×10⁴ |
| | *C. albicans* | 1.4×10⁵ | 1×10² | 0 | 0 | 0 |
| | *P. aeruginosa* | 3×10⁶ | 0 | 0 | 10 | 0 |
| | *E. coli* | 1×10⁶ | 1×10² | 0 | 0 | 0 |
| | *S. aureus* | 1×10⁶ | 0 | 0 | 10 | 0 |
| SN 0.2 wt% + BZ-2 0.3 wt% | *A. brasiliensis* | 4×10⁵ | 1.1×10⁴ | 9×10³ | 1×10⁴ | 1.8×10⁴ |
| | *C. albicans* | 1.4×10⁵ | 4×10² | 0 | 0 | 0 |
| | *P. aeruginosa* | 3×10⁶ | 0 | 0 | 0 | 0 |
| | *E. coli* | 1×10⁶ | 0 | 0 | 0 | 0 |
| | *S. aureus* | 1×10⁶ | 0 | 0 | 0 | 0 |
| SN 0.2 wt% + BZ-2 0.5 wt% | *A. brasiliensis* | 4×10⁵ | 1.3×10⁴ | 6.4 x10³ | 9×10³ | 1.8×10⁴ |
| | *C. albicans* | 1.4×10⁵ | 4×10² | 1×10¹ | 0 | 0 |
| | *P. aeruginosa* | 3×10⁶ | 0 | 0 | 0 | 0 |
| | *E. coli* | 1×10⁶ | 0 | 0 | 0 | 0 |
| | *S. aureus* | 1×10⁶ | 0 | 0 | 0 | 0 |
| SN 0.2 wt% + BZ-1 0.1 wt% + BZ-2 0.1wt% | *A. brasiliensis* | 4×10⁵ | 4×10⁴ | 9.2×10³ | 9×10³ | 1.9×10⁴ |
| | *C. albicans* | 1.4×10⁵ | 0 | 0 | 0 | 0 |
| | *P. aeruginosa* | 3×10⁶ | 0 | 0 | 0 | 0 |
| | *E. coli* | 1×10⁶ | 0 | 0 | 0 | 0 |
| | *S. aureus* | 1×10⁶ | 0 | 0 | 0 | 0 |
| SN 0.2 wt% + BZ-1 0.2 wt% + BZ-2 0.2wt% | *A. brasiliensis* | 4×10⁵ | 12×10⁴ | 6.5×10³ | 3×10³ | 3×10³ |
| | *C. albicans* | 1.4×10⁵ | 0 | 0 | 0 | 0 |
| | *P. aeruginosa* | 3×10⁶ | 0 | 0 | 0 | 0 |
| | *E. coli* | 1×10⁶ | 0 | 0 | 0 | 0 |
| | *S. aureus* | 1×10⁶ | 0 | 0 | 0 | 0 |
| Cream + bacteria | *A. brasiliensis* | 4×10⁵ | 6×10⁴ | Full | Full | Full |
| | *C. albicans* | 1.4×10⁵ | Full | Full | 2.6×10³ | 6×10² |
| | *P. aeruginosa* | 3×10⁶ | Full | Full | Full | Full |
| | *E. coli* | 1×10⁶ | 6×10² | 0 | 0 | 0 |
| | *S. aureus* | 1×10⁶ | 2.3×10⁵ | 3.1×10² | 1×10¹ | 1×10¹ |
| Sharomix | *A. brasiliensis* | 4×10⁵ | 0 | 0 | 0 | 0 |
| | *C. albicans* | 1.4×10⁵ | 0 | 0 | 0 | 0 |
| | *P. aeruginosa* | 3×10⁶ | 0 | 0 | 0 | 0 |
| | *E. coli* | 1×10⁶ | 0 | 0 | 0 | 0 |
| | *S. aureus* | 1×10⁶ | 0 | 0 | 0 | 0 |

As can be seen from **Table 3,** both Siam benzoin and Sumatra benzoin show antimicrobial activity. However, a more predominant effect was observed for the combination of Siam benzoin (BZ-2) and saponin specifically against *A. brasiliensis, P. aeruginosa* and *S. aureus.*

### Example 4: Benzoin resin from different sources

Benzoin Sumatra extracts were obtained from various sources; the resin was milled and extracted at an MCT/resin ratio of 9:1 (w/w). The antimicrobial activity of the various extracts was tested with and without saponin extract (*Sapindus mukorossi* extract (SN)), and with or without wasabi (WS) in a non-preserved cream. The samples were inoculated (1×10⁶) with *S. aureus* (ATCC 6538). The results are provided in **Tables 4-1 and 4-2.**

**Table 4-1: Total count for S. aureus after contact with the examined ingredients (2-days after inoculation)**

| **Tested Formula** | **BZ source 1** | **BZ source 2** | **BZ source 3** | **BZ source 4** | **BZ source 5** | **BZ source 6** | **BZ source 7** |
|---|---|---|---|---|---|---|---|
| BZ 0.1wt% | 1.48×10³ | 0 | Full | Full | 1.3×10² | Full | Full |
| BZ 0.2wt% | 0 | 0 | Full | Full | 0 | 6.2×10² | 4×10³ |
| SN 0.5 wt% +BZ 0.1wt% | 7.7×10² | 0 | Full | Full | 0 | Full | Full |
| SN 0.5 wt% +BZ 0.2wt% | 0 | 0 | Full | Full | 0 | Full | 1.2×10³ |
| SN 0.5 wt% +BZ 0.2wt% +WS 0.5wt% | 0 | 0 | Full | Full | 0 | full | full |

**Table 4-2: Total count for S. aureus after contact with the examined ingredients (7-days after inoculation)**

| **Tested Formula** | **BZ source 1** | **BZ source 2** | **BZ source 3** | **BZ source 4** | **BZ source 5** | **BZ source 6** | **BZ source 7** |
|---|---|---|---|---|---|---|---|
| BZ 0.1wt% | 0 | 0 | 2.1×10² | 0 | 0 | 0 | 0 |
| BZ 0.2wt% | 0 | 0 | 0 | 0 | 0 | 0 | 0 |
| SN 0.5 wt% +BZ 0.1wt% | 0 | 0 | Full | 0 | 0 | 0 | 0 |
| SN 0.5 wt% +BZ 0.2wt% | 0 | 0 | 7.0×10¹ | 0 | 0 | 0 | 0 |
| SN 0.5 wt% +BZ 0.2wt% +WS 0.5wt% | 0 | 0 | 1×10¹ | 0 | 0 | 0 | 0 |

As can be seen, the tested combinations resulted in significant antimicrobial activity after 7-days from inoculation. In addition, it was observed that benzoin extracts that were rich in certain phenolic derivatives, such as benzoic acid derivatives (e.g. coniferyl benzoate) and cinnamic acid derivatives (e.g. cinnamic acid, cinnamyl cinnamate) showed also significant activity as early as 2-fays from inoculation.

### Example 5: Composition with ASGT and saponin (not in accordance with the invention)

A mixture of 0.72g arginine (A), 0.6g salicylic acid (S), 1.2g glucose (G) and 0.108g trehalose (T) was prepared by dissolving the component in water. The final concentration of the mixture in the water was 10 wt%.

The mixture ASGT was added to a non-preserved cleansing formula in different concentration, with and without *Sapindus mukorossi* extract. The results are provided in **Table 5.**

Tested Microorganisms: *E. coli* (ATCC 8739); S. *aureus* (ATCC 6538); *P. aeruginosa (ATCC 9027); C. albicans (ATCC 10231) and A. Brasiliensis (ATCC 16404).*

**Table 5: Total count for different microorganisms after contact with the examined ingredients (non-preserved cleansing formula)**

| **Tested Formula** | **Tested organism** | **Initial inoculums level (cfu/ml)** | **2 days** | **7 days** | **14 days** | **28 days** |
|---|---|---|---|---|---|---|
| ASGT 0.05 wt% | *A. brasiliensis* | 4.5×10⁵ | 7.6×10⁴ | 4.7×10³ | 1.25×10³ | 2×10² |
| | *C. albicans* | 2.5×10⁵ | 0 | 0 | 0 | 0 |
| | *P. aeruginosa* | 6.8×10⁶ | 0 | 0 | 0 | 0 |
| | *E. coli* | 3.2×10⁶ | 3×10¹ | 0 | 0 | 0 |
| | *S. aureus* | 1×10⁶ | 0 | 0 | 0 | 0 |
| ASGT 0.1 wt% | *A. brasiliensis* | 4.5×10⁵ | 8.2×10⁴ | 1.3×10³ | 1×10² | 0 |
| | *C. albicans* | 2.5×10⁵ | 0 | 0 | 0 | 0 |
| | *P. aeruginosa* | 6.8×10⁶ | 0 | 0 | 0 | 0 |
| | *E. coli* | 3.2×10⁶ | 5×10¹ | 0 | 0 | 0 |
| | *S. aureus* | 1×10⁶ | 0 | 0 | 0 | 0 |
| ASGT 0.2 wt% | *A. brasiliensis* | 4.5×10⁵ | 8×10⁴ | 3×10² | 1×10² | 0 |
| | *C. albicans* | 2.5×10⁵ | 0 | 0 | 0 | 0 |
| | *P. aeruginosa* | 6.8×10⁶ | 0 | 0 | 0 | 0 |
| | *E. coli* | 3.2×10⁶ | 2.5×10² | 0 | 0 | 0 |
| | *S. aureus* | 1×10⁶ | 0 | 0 | 0 | 0 |
| ASGT 0.5 wt% | *A. brasiliensis* | 4.5×10⁵ | 6.8×10⁴ | 1×10³ | 3×10¹ | 0 |
| | *C. albicans* | 2.5×10⁵ | 0 | 0 | 0 | 0 |
| | *P. aeruginosa* | 6.8×10⁶ | 0 | 0 | 0 | 0 |
| | *E. coli* | 3.2×10⁶ | 6×10¹ | 0 | 0 | 0 |
| | *S. aureus* | 1×10⁶ | 0 | 0 | 0 | 0 |
| SN 0.05 wt% + ASGT 0.05 wt% | *A. brasiliensis* | 4.5×10⁵ | 7.2×10⁴ | 1.2×10³ | 5×10² | 6×10¹ |
| | *C. albicans* | 2.5×10⁵ | 0 | 0 | 0 | 0 |
| | *P. aeruginosa* | 6.8×10⁶ | 0 | 0 | 0 | 0 |
| | *E. coli* | 3.2×10⁶ | 5×10¹ | 0 | 0 | 0 |
| | *S. aureus* | 1×10⁶ | 0 | 0 | 0 | 0 |
| SN 0.05 wt% + ASGT 0.1 wt% | *A. brasiliensis* | 4.5×10⁵ | 7.2×10⁴ | 5×10² | 1×10² | 1×10¹ |
| | *C. albicans* | 2.5×10⁵ | 0 | 0 | 0 | 0 |
| | *P. aeruginosa* | 6.8×10⁶ | 0 | 0 | 0 | 0 |
| | *E. coli* | 3.2×10⁶ | 0 | 0 | 0 | 0 |
| | *S. aureus* | 1×10⁶ | 0 | 0 | 0 | 0 |
| SN 0.05 wt% + ASGT 0.2 wt% | *A. brasiliensis* | 4.5×10⁵ | 6.8×10⁴ | 5×10² | 1×10¹ | 0 |
| | *C. albicans* | 2.5×10⁵ | 0 | 0 | 0 | 0 |
| | *P. aeruginosa* | 6.8×10⁶ | 0 | 0 | 0 | 0 |
| | *E. coli* | 3.2×10⁶ | 5×10¹ | 0 | 0 | 0 |
| | *S. aureus* | 1×10⁶ | 0 | 0 | 0 | 0 |
| SN 0.05 wt% + ASGT 0.5 wt% | *A. brasiliensis* | 4.5×10⁵ | 7.28×10⁴ | 4×10² | 0 | 0 |
| | *C. albicans* | 2.5×10⁵ | 0 | 0 | 0 | 0 |
| | *P. aeruginosa* | 6.8×10⁶ | 0 | 0 | 0 | 0 |
| | *E. coli* | 3.2×10⁶ | 1.7×10² | 0 | 0 | 0 |
| | *S. aureus* | 1×10⁶ | 0 | 0 | 0 | 0 |
| SN 0.2 wt% + ASGT 0.05 wt% | *A. brasiliensis* | 4.5×10⁵ | 4.8×10⁴ | 5×10² | 1×10² | 0 |
| | *C. albicans* | 2.5×10⁵ | 0 | 0 | 0 | 0 |
| | *P. aeruginosa* | 6.8×10⁶ | 0 | 0 | 0 | 0 |
| | *E. coli* | 3.2×10⁶ | 2.6×10² | 0 | 0 | 0 |
| | *S. aureus* | 1×10⁶ | 0 | 0 | 0 | 0 |
| SN 0.2 wt% + ASGT 0.1 wt% | *A. brasiliensis* | 4.5×10⁵ | 6×10⁴ | 8×10² | 3×10¹ | 0 |
| | *C. albicans* | 2.5×10⁵ | 0 | 0 | 0 | 0 |
| | *P. aeruginosa* | 6.8×10⁶ | 0 | 0 | 0 | 0 |
| | *E. coli* | 3.2×10⁶ | 1.27×10³ | 0 | 0 | 0 |
| | *S. aureus* | 1×10⁶ | 0 | 0 | 0 | 0 |
| SN 0.2 wt% + ASGT 0.2 wt% | *A. brasiliensis* | 4.5×10⁵ | 6×10⁴ | 7×10² | 3×10¹ | 0 |
| | *C. albicans* | 2.5×10⁵ | 0 | 0 | 0 | 0 |
| | *P. aeruginosa* | 6.8×10⁶ | 0 | 0 | 0 | 0 |
| | *E. coli* | 3.2×10⁶ | 2.22×10³ | 0 | 0 | 0 |
| | *S. aureus* | 1×10⁶ | 0 | 0 | 0 | 0 |
| SN 0.2 wt% + ASGT 0.5 wt% | *A. brasiliensis* | 4.5×10⁵ | 6.4×10⁴ | 1.3×10³ | 0 | 0 |
| | *C. albicans* | 2.5×10⁵ | 0 | 0 | 0 | 0 |
| | *P. aeruginosa* | 6.8×10⁶ | 0 | 0 | 0 | 0 |
| | *E. coli* | 3.2×10⁶ | 2.04×10³ | 0 | 0 | 0 |
| | *S. aureus* | 1×10⁶ | 0 | 0 | 0 | 0 |
| SN 0.5 wt% + ASGT 0.05 wt% | *A. brasiliensis* | 4.5×10⁵ | 3.15×10⁴ | 7×10² | 0 | 0 |
| | *C. albicans* | 2.5×10⁵ | 0 | 0 | 0 | 0 |
| | *P. aeruginosa* | 6.8×10⁶ | 0 | 0 | 0 | 0 |
| | *E. coli* | 3.2×10⁶ | 4×10³ | 0 | 0 | 0 |
| | *S. aureus* | 1×10⁶ | 0 | 0 | 0 | 0 |
| SN 0.5 wt% + ASGT 0.1 wt% | *A. brasiliensis* | 4.5×10⁵ | 3.61×10⁴ | 7×10² | 1×10¹ | 0 |
| | *C. albicans* | 2.5×10⁵ | 0 | 0 | 0 | 0 |
| | *P. aeruginosa* | 6.8×10⁶ | 0 | 0 | 0 | 0 |
| | *E. coli* | 3.2×10⁶ | 5.34×10³ | 0 | 0 | 0 |
| | *S. aureus* | 1×10⁶ | 0 | 0 | 0 | 0 |
| SN 0.5 wt% + ASGT 0.2 wt% | *A. brasiliensis* | 4.5×10⁵ | 2.5×10⁴ | 2×10² | 0 | 0 |
| | *C. albicans* | 2.5×10⁵ | 0 | 0 | 0 | 0 |
| | *P. aeruginosa* | 6.8×10⁶ | 0 | 0 | 0 | 0 |
| | *E. coli* | 3.2×10⁶ | 9.9×10³ | 0 | 0 | 0 |
| | *S. aureus* | 1×10⁶ | 0 | 0 | 0 | 0 |
| SN 0.5 wt% + ASGT 0.5 wt% | *A. brasiliensis* | 4.5×10⁵ | 4.3×10⁴ | 8×10² | 1×10¹ | 0 |
| | *C. albicans* | 2.5×10⁵ | 0 | 0 | 0 | 0 |
| | *P. aeruginosa* | 6.8×10⁶ | 0 | 0 | 0 | 0 |
| | *E. coli* | 3.2×10⁶ | 6.1×10³ | 0 | 0 | 0 |
| | *S. aureus* | 1×10⁶ | 0 | 0 | 0 | 0 |
| Soap + bacteria | *A. brasiliensis* | 4.5×10⁵ | 1.1×10⁵ | Full | 5×10⁴ | Full |
| | *C. albicans* | 2.5×10⁵ | 0 | 0 | 0 | 0 |
| | *P. aeruginosa* | 6.8×10⁶ | 0 | 0 | 0 | 0 |
| | *E. coli* | 3.2×10⁶ | 0 | 0 | 0 | 0 |
| | *S. aureus* | 1×10⁶ | 0 | 0 | 0 | 0 |
| Sharomix 1% | *A. brasiliensis* | 4.5×10⁵ | 1.7×10³ | 0 | 0 | 0 |
| | *C. albicans* | 2.5×10⁵ | 0 | 0 | 0 | 0 |
| | *P. aeruginosa* | 6.8×10⁶ | 0 | 0 | 0 | 0 |
| | *E. coli* | 3.2×10⁶ | 0 | 0 | 0 | 0 |
| | *S. aureus* | 1×10⁶ | 0 | 0 | 0 | 0 |

As can be seen from Table 5, the ASGT mixture shows activity against all types of tested microorganisms. When combined with saponin, significant improvement in antimicrobial activity was observed, especially for mold *(A. brasiliensis).*

### Example 6: Composition with ASGT, benzoin and saponin

A 10% Siam benzoin in MCT extract was prepared as in Example 1. A 10% in water mixture of ASGT was prepared according to Example 5.

The benzoin extract and ASGT mixture were added to a non-preserved cream formulation in different concentration, with and without *Sapindus mukorossi* extract. The results are provided in **Table 6.**

Tested Microorganisms: *E. coli* (ATCC 8739); S. *aureus* (ATCC 6538); P. *aeruginosa (ATCC 9027); C. albicans (ATCC 10231) and A. Brasiliensis (ATCC 16404).*

**Table 6: Total count for different microorganisms after contact with the examined ingredients (non-preserved cream)**

| **Tested Formula** | **Tested organism** | **Initial inoculums level (cfu/ml)** | **2 days** | **7 days** | **14 days** | **28 days** |
|---|---|---|---|---|---|---|
| ASGT 0.05 wt% | *A. brasiliensis* | 4.5×10⁵ | Full | 6×10⁴ | 8.8×10⁴ | Full |
| | *C. albicans* | 1.4×10⁵ | Full | Full | 5.64×10³ | 4.4×10² |
| | *P. aeruginosa* | 5.5×10⁶ | Full | Full | Full | Full |
| | *E. coli* | 1.5×10⁶ | 2.7×10³ | 4×10¹ | 0 | 1×10¹ |
| | *S. aureus* | 1.4×10⁶ | 1.2×10⁵ | 4.64×10³ | 3.15×10³ | 6×10¹ |
| ASGT 0.1 wt% | *A. brasiliensis* | 4.5×10⁵ | Full | 8×10⁴ | 7.6×10⁴ | Full |
| | *C. albicans* | 1.4×10⁵ | Full | Full | 1.56×10³ | 5×10¹ |
| | *P. aeruginosa* | 5.5×10⁶ | Full | Full | Full | Full |
| | *E. coli* | 1.5×10⁶ | 3.8×10³ | 8×10¹ | 0 | 0 |
| | *S. aureus* | 1.4×10⁶ | 6×10⁴ | 3×10³ | 1.3×10² | 3×10¹ |
| ASGT 0.2 wt% | *A. brasiliensis* | 4.5×10⁵ | Full | 8.8×10⁴ | 7.6×10⁴ | Full |
| | *C. albicans* | 1.4x10⁵ | Full | Full | 3.5×10² | 4.4×10¹ |
| | *P. aeruginosa* | 5.5×10⁶ | Full | Full | Full | Full |
| | *E. coli* | 1.5×10⁶ | 3.4×10³ | 2×10¹ | 0 | 0 |
| | *S. aureus* | 1.4×10⁶ | 8.62×10⁴ | 2×10³ | 2.5×10² | 0 |
| ASGT 0.5 wt% | *A. brasiliensis* | 4.5×10⁵ | Full | 6×10⁴ | 6.8×10⁴ | Full |
| | *C. albicans* | 1.4×10⁵ | Full | Full | 5.9×10² | 0 |
| | *P. aeruginosa* | 5.5×10⁶ | Full | Full | Full | Full |
| | *E. coli* | 1.5×10⁶ | 6.7×10³ | 1.5×10² | 0 | 0 |
| | *S. aureus* | 1.4×10⁶ | 7.72×10⁴ | 3.52×10³ | 8.9×10² | 5×10¹ |
| SN 0.5 wt% + ASGT 0.05 wt% | *A. brasiliensis* | 4.5×10⁵ | 9×10³ | 3.8×10³ | 3.2×10³ | 2.4×10³ |
| | *C. albicans* | 1.4×10⁵ | 0 | 0 | 0 | 0 |
| | *P. aeruginosa* | 5.5×10⁶ | Full | Full | Full | Full |
| | *E. coli* | 1.5×10⁶ | 1.7×10³ | 0 | 0 | 0 |
| | *S. aureus* | 1.4×10⁶ | | 6.8×10³ | 4.4×10² | 3×10¹ |
| SN 0.5 wt% + ASGT 0.1 wt% | *A. brasiliensis* | 4.5×10⁵ | 4.9×10³ | 1.6×10³ | 8×10² | 5.3×10² |
| | *C. albicans* | 1.4×10⁵ | 0 | 0 | 0 | 0 |
| | *P. aeruginosa* | 5.5×10⁶ | Full | Full | Full | Full |
| | *E. coli* | 1.5×10⁶ | 2.4×10³ | 0 | 0 | 0 |
| | *S. aureus* | 1.4×10⁶ | 3×10⁴ | 2.11×10³ | 1.3×10² | 0 |
| SN 0.5 wt% + ASGT 0.2 wt% | *A. brasiliensis* | 4.5×10⁵ | 4.2×10³ | 1×10² | 2.3×10² | 2.2×10² |
| | *C. albicans* | 1.4×10⁵ | 0 | 0 | 0 | 0 |
| | *P. aeruginosa* | 5.5×10⁶ | Full | Full | Full | Full |
| | *E. coli* | 1.5×10⁶ | 3.4×10³ | 2×10¹ | 0 | 0 |
| | *S. aureus* | 1.4×10⁶ | 2.42×10⁴ | 1.78×10³ | 1.3×10² | 1×10¹ |
| SN 0.5 wt% + ASGT 0.5 wt% | *A. brasiliensis* | 4.5×10⁵ | 4.6×10³ | 5×10² | 1.8×10² | 3×10¹ |
| | *C. albicans* | 1.4×10⁵ | 0 | 0 | 0 | 0 |
| | *P. aeruginosa* | 5.5×10⁶ | Full | Full | Full | Full |
| | *E. coli* | 1.5×10⁶ | 3.9×10³ | 3×10¹ | 0 | 0 |
| | *S. aureus* | 1.4×10⁶ | 1.45×10⁴ | 3.2×10³ | 2.6×10² | 0 |
| SN 0.5 wt% + BZ 0.2 wt% + ASGT 0.05 wt% | *A. brasiliensis* | 4.5×10⁵ | 3.6×10³ | 9×10² | 7×10² | 8×10² |
| | *C. albicans* | 1.4×10⁵ | 0 | 0 | 0 | 0 |
| | *P. aeruginosa* | 5.5×10⁶ | 0 | 0 | 0 | 0 |
| | *E. coli* | 1.5×10⁶ | 0 | 0 | 0 | 0 |
| | *S. aureus* | 1.4×10⁶ | 0 | 0 | 0 | 0 |
| SN 0.5 wt% + BZ 0.2 wt% + ASGT 0.1 wt% | *A. brasiliensis* | 4.5×10⁵ | 3.9×10³ | 2×10² | 1.7×10² | 2.8×10² |
| | *C. albicans* | 1.4×10⁵ | 0 | 0 | 0 | 0 |
| | *P. aeruginosa* | 5.5×10⁶ | 0 | 0 | 0 | 0 |
| | *E. coli* | 1.5×10⁶ | 0 | 0 | 0 | 0 |
| | *S. aureus* | 1.4×10⁶ | 0 | 0 | 0 | 0 |
| SN 0.5 wt% + BZ 0.2 wt% + ASGT 0.2 wt% | *A. brasiliensis* | 4.5×10⁵ | | 5×10² | 1×10² | 6×10¹ |
| | *C. albicans* | 1.4×10⁵ | 0 | 0 | 0 | 0 |
| | *P. aeruginosa* | 5.5×10⁶ | 4×10² | 0 | 0 | 0 |
| | *E. coli* | 1.5×10⁶ | 0 | 0 | 0 | 0 |
| | *S. aureus* | 1.4×10⁶ | 0 | 0 | 0 | 0 |
| SN 0.5 wt% + BZ 0.2 wt% + ASGT 0.5 wt% | *A. brasiliensis* | 4.5×10⁵ | 4.5×10³ | 1×10² | 8×10¹ | 2×10¹ |
| | *C. albicans* | 1.4×10⁵ | 0 | 0 | 0 | 0 |
| | *P. aeruginosa* | 5.5×10⁶ | 2×10² | 0 | 0 | 0 |
| | *E. coli* | 1.5×10⁶ | 0 | 0 | 0 | 0 |
| | *S. aureus* | 1.4×10⁶ | 0 | 0 | 0 | 0 |
| SN 0.5 wt% + BZ 0.2% | *A. brasiliensis* | 4.5×10⁵ | 5×10³ | 3.1×10³ | 2.5×10³ | 2.8×10³ |
| | *C. albicans* | 1.4×10⁵ | 0 | 0 | 0 | 0 |
| | *P. aeruginosa* | 5.5×10⁶ | 0 | 0 | 0 | 0 |
| | *E. coli* | 1.5×10⁶ | 3×10² | 0 | 0 | 0 |
| | *S. aureus* | 1.4×10⁶ | 0 | 0 | 0 | 0 |
| Cream + bacteria | *A. brasiliensis* | 4.5×10⁵ | Full | 1.28×10⁵ | 8×10⁴ | Full |
| | *C. albicans* | 1.4×10⁵ | Full | Full | 4×10³ | 2×10¹ |
| | *P. aeruginosa* | 5.5×10⁶ | Full | Full | Full | Full |
| | *E. coli* | 1.5×10⁶ | 3.5×10³ | 2×10¹ | 0 | 0 |
| | *S. aureus* | 1.4×10⁶ | 2.16×10⁵ | 1.4×10⁴ | 6×10² | 1×10¹ |
| Sharomix 1% | *A. brasiliensis* | 4.5×10⁵ | 0 | 0 | 0 | 0 |
| | *C. albicans* | 1.4×10⁵ | 0 | 0 | 0 | 0 |
| | *P. aeruginosa* | 5.5×10⁶ | 0 | 0 | 0 | 0 |
| | *E. coli* | 1.5×10⁶ | 0 | 1×10¹ | 0 | 0 |
| | *S. aureus* | 1.4×10⁵ | 0 | 0 | 0 | 0 |

As evident from **Table 6,** the combination of ASGT with saponin and benzoin SN extract showed significant activity against *A. brasiliensis,* while eliminating almost entirely the other types of microorganisms.

The reduction of *A. brasiliensis* colonies can attest to an effect between the benzoin, saponin and ASGT.

### Example 7: Composition with Acacia arabica, benzoin and saponin

A 10% Siam benzoin in MCT extract was prepared as in Example 1. An extract of *Acacia arabica* (AC, babool) fruit was prepared according to the following procedure.

50 g of *Acacia arabica* fruit powder were placed in a filtering paper thimble and inserted into a glass vessel. 250 ml of distilled water at 40°C were added and the vessel was placed in a water bath at 100°C and shaked at 150 RPM for 2 hours. The solution was then left to cool, and filtered to obtain the extract.

The babool and benzoin extracts were added to a non-preserved cream formulation in different concentration, with and without *Sapindus mukorossi* and/or Benzoin Siam extracts. The results are provided in **Table 7.**

Tested Microorganisms: *E. coli* (ATCC 8739); S. *aureus* (ATCC 6538); *P. aeruginosa (ATCC 9027); C. albicans (ATCC 10231) and A. Brasiliensis (ATCC 16404).*

**Table 7: Total count for different microorganisms after contact with the examined ingredients**

| **Tested Formula** | **Tested organism** | **Initial inoculums level (cfu/ml)** | **2 days** | **7 days** | **14 days** | **28 days** |
|---|---|---|---|---|---|---|
| AC 0.5 wt% | *A. brasiliensis* | 2.2×10⁵ | Full | 7.04×10⁴ | 2.96×10⁴ | 1.88×10⁴ |
| | *C. albicans* | 3.5×10⁵ | Full | Full | 1.02×10⁴ | 9.4×10² |
| | *P. aeruginosa* | 6.4×10⁶ | 6.0×10² | 4×10² | 2.3×10² | 6^{×}10¹ |
| | *E. coli* | 4×10⁶ | 5.6×10⁴ | 1×10¹ | 0 | 1×10¹ |
| | *S. aureus* | 8×10⁶ | 0 | 1×10¹ | 2^{×}10¹ | 1×10¹ |
| AC 0.1 wt% | *A. brasiliensis* | 2.2×10⁵ | Full | 5.76×10⁴ | 6.48×10⁴ | 3.24×10⁴ |
| | *C. albicans* | 3.5×10⁵ | Full | Full | 2.08×10³ | 2^{×}10¹ |
| | *P. aeruginosa* | 6.4×10⁶ | 4×10² | 6×10² | 1.4×10⁵ | 12^{×}10³ |
| | *E. coli* | 4×10⁶ | 8.24×10⁴ | 3.1×10² | 0 | 0 |
| | *S. aureus* | 8×10⁶ | 0 | 0 | 0 | 0 |
| AC 0.05 wt% | *A. brasiliensis* | 2.2×10⁵ | Full | 7.76×10⁴ | 6.4×10⁴ | 4.32×10⁴ |
| | *C. albicans* | 3.5×10⁵ | Full | Full | 9.9×10² | 0 |
| | *P. aeruginosa* | 6.4×10⁶ | 8×10² | 4.7×10² | 1.4×10⁵ | 8×10¹ |
| | *E. coli* | 4×10⁶ | 2.82×10⁵ | 4.8×10³ | 0 | 1×10¹ |
| | *S. aureus* | 8×10⁶ | 0 | 0 | 2^{×}10¹ | 0 |
| AC 0.01 wt% | *A. brasiliensis* | 2.2×10⁵ | Full | 9.12×10⁴ | 6.8×10⁴ | 4.64×10⁴ |
| | *C. albicans* | 3.5×10⁵ | Full | 4.56×10³ | 1.34×10³ | 0 |
| | *P. aeruginosa* | 6.4×10⁶ | 12^{×}10³ | 4.3×10² | 1.8×10² | 1.2×10² |
| | *E. coli* | 4×10⁶ | 2.09×10⁴ | 2.44×10³ | 0 | 10 |
| | *S. aureus* | 8×10⁶ | 12^{×}10³ | 0 | 0 | 0 |
| BZ-2 0.2 wt% + AC 0.5 wt% | *A. brasiliensis* | 2.2×10⁵ | Full | 4.88×10⁴ | 3.52×10⁴ | 7.6×10³ |
| | *C. albicans* | 3.5×10⁵ | Full | Full | 1.18×10⁴ | 1.7×10³ |
| | *P. aeruginosa* | 6.4×10⁶ | 1×10² | 4×10¹ | 1×10¹ | 1×10¹ |
| | *E. coli* | 4×10⁶ | Full | 7.6×10³ | 1×10¹ | 0 |
| | *S. aureus* | 8×10⁶ | 1×10² | 1×10¹ | 2^{×}10¹ | 0 |
| BZ-2 0.2 wt% + AC 0.1 wt% | *A. brasiliensis* | 2.2×10⁵ | 6.4×10⁴ | 3.2×10⁴ | 2.56×10⁴ | 1.2×10⁴ |
| | *C. albicans* | 3.5×10⁵ | Full | 9.04×10³ | 7.3×10² | 0 |
| | *P. aeruginosa* | 6.4×10⁶ | 0 | 0 | 0 | 0 |
| | *E. coli* | 4×10⁶ | 0 | 0 | 0 | 0 |
| | *S. aureus* | 8×10⁶ | 1×10² | 0 | 0 | 0 |
| BZ-2 0.2 wt% + AC 0.05 wt% | *A. brasiliensis* | 2.2×10⁵ | 2.25×10⁴ | 1.96×10⁴ | 8.4×10³ | 6.6×10³ |
| | *C. albicans* | 3.5×10⁵ | 4.4×10⁴ | 6.5×10² | 6^{×}10¹ | 0 |
| | *P. aeruginosa* | 6.4×10⁶ | 0 | 1×10¹ | 0 | 0 |
| | *E. coli* | 4×10⁶ | 1×10² | 0 | 1×10¹ | 0 |
| | *S. aureus* | 8×10⁶ | 0 | 0 | 0 | 0 |
| BZ-2 0.2 wt% + AC 0.01 wt% | *A. brasiliensis* | 2.2×10⁵ | 1.5×10⁴ | 1.32×10⁴ | 1.08×10⁴ | 1.36×10⁴ |
| | *C. albicans* | 3.5×10⁵ | 2.3×10³ | 9×10¹ | 1.2×10² | 0 |
| | *P. aeruginosa* | 6.4×10⁶ | 0 | 0 | 0 | 0 |
| | *E. coli* | 4×10⁶ | 0 | 0 | 0 | 1×10¹ |
| | *S. aureus* | 8×10⁶ | 0 | 2×10¹ | 2^{×}10¹ | 0 |
| SN 0.2 wt% + AC 0.5 wt% | *A. brasiliensis* | 2.2×10⁵ | 4.8×10³ | 4.1×10³ | 1.3×10³ | 5×10² |
| | *C. albicans* | 3.5×10⁵ | 1×10² | 0 | 0 | 0 |
| | *P. aeruginosa* | 6.4×10⁶ | 8.2×10¹ | 0 | 0 | 0 |
| | *E. coli* | 4×10⁶ | 1.41×10³ | 6^{×}10¹ | 0 | 1×10¹ |
| | *S. aureus* | 8×10⁶ | 0 | 2×10¹ | 2^{×}10¹ | 0 |
| SN 0.2 wt% + AC 0.1 wt% | *A. brasiliensis* | 2.2×10⁵ | 3.08×10⁴ | 4.2×10³ | 5.1×10³ | 3.4×10³ |
| | *C. albicans* | 3.5×10⁵ | 0 | 0 | 0 | 0 |
| | *P. aeruginosa* | 6.4×10⁶ | 0 | 0 | 0 | 0 |
| | *E. coli* | 4×10⁶ | 9.36×10⁴ | 1.03×10³ | 1×10¹ | 0 |
| | *S. aureus* | 8×10⁶ | 0 | 0 | 0 | 0 |
| SN 0.2 wt% + AC 0.05 wt% | *A. brasiliensis* | 2.2×10⁵ | 32×10⁴ | 2.5×10³ | 2.1×10³ | 1×10³ |
| | *C. albicans* | 3.5×10⁵ | 0 | 0 | 0 | 0 |
| | *P. aeruginosa* | 6.4×10⁶ | 0 | 0 | 0 | 0 |
| | *E. coli* | 4×10⁶ | 1.31×10⁵ | 4×10³ | 0 | 1×10¹ |
| | *S. aureus* | 8×10⁶ | 3×10² | 0 | 0 | 0 |
| SN 0.2 wt% + AC 0.01 wt% | *A. brasiliensis* | 2.2×10⁵ | 7×10⁴ | 9×10³ | 7.2×10³ | 5.7×10³ |
| | *C. albicans* | 3.5×10⁵ | 0 | 0 | 0 | 0 |
| | *P. aeruginosa* | 6.4×10⁶ | 1×10² | 0 | 1×10¹ | 0 |
| | *E. coli* | 4×10⁶ | 6.8×10³ | 4.2×10² | 0 | 0 |
| | *S. aureus* | 8×10⁶ | 1.9×10³ | 3×10² | 0 | 0 |
| SN 0.2 wt% + BZ-2 0.2 wt% + AC 0.5 wt% | *A. brasiliensis* | 2.2×10⁵ | 2×10⁴ | 5.2×10³ | 1.5×10³ | 7×10² |
| | *C. albicans* | 3.5×10⁵ | 0 | 0 | 0 | 0 |
| | *P. aeruginosa* | 6.4×10⁶ | Full | Full | Full | Full |
| | *E. coli* | 4×10⁶ | Full | Full | 4×10¹ | 0 |
| | *S. aureus* | 8×10⁶ | 0 | 1×10¹ | 0 | 0 |
| SN 0.2 wt% + BZ-2 0.2 wt% + AC 0.1 wt% | *A. brasiliensis* | 2.2×10⁵ | 4×10⁴ | 1×10³ | 7.1×10² | 3×10² |
| | *C. albicans* | 3.5×10⁵ | 1×10² | 0 | 1×10¹ | 0 |
| | *P. aeruginosa* | 6.4×10⁶ | 0 | 1×10¹ | 1×10¹ | 0 |
| | *E. coli* | 4×10⁶ | 0 | 1×10¹ | 1×10¹ | 0 |
| | *S. aureus* | 8×10⁶ | 0 | 2×10¹ | 1×10¹ | 1×10¹ |
| SN 0.2 wt% + BZ-2 0.2 wt% + AC 0.05 wt% | *A. brasiliensis* | 2.2×10⁵ | 2.04×10⁴ | 7×10² | 2×10³ | 3×10² |
| | *C. albicans* | 3.5×10⁵ | 0 | 0 | 0 | 0 |
| | *P. aeruginosa* | 6.4×10⁶ | 0 | 0 | 0 | 0 |
| | *E. coli* | 4×10⁶ | 0 | 0 | 1×10¹ | 0 |
| | *S. aureus* | 8×10⁶ | 0 | 0 | 1×10¹ | 2^{×}10¹ |
| SN 0.2 wt% + BZ-2 0.2 wt% +AC 0.01wt% | *A. brasiliensis* | 2.2×10⁵ | 1.56×10⁴ | 8.9×10³ | 7×10³ | 8.4×10³ |
| | *C. albicans* | 3.5×10⁵ | 0 | 0 | 0 | 0 |
| | *P. aeruginosa* | 6.4×10⁶ | 0 | 0 | 0 | 0 |
| | *E. coli* | 4×10⁶ | 0 | 1×10¹ | 0 | 0 |
| | *S. aureus* | 8×10⁶ | 0 | 1×10¹ | 0 | 0 |
| SN 0.5% wt% + AC 0.5 wt% | *A. brasiliensis* | 2.2×10⁵ | 1.63×10⁴ | 1.9×10³ | 1.3×10³ | 4×10² |
| | *C. albicans* | 3.5×10⁵ | 0 | 0 | 0 | 0 |
| | *P. aeruginosa* | 6.4×10⁶ | 7.52×10⁴ | Full | Full | Full |
| | *E. coli* | 4×10⁶ | 1.66×10⁵ | 5.4×10² | 0 | 0 |
| | *S. aureus* | 8×10⁶ | 0 | 0 | 1×10¹ | 0 |
| SN 0.5 wt% + AC 0.1 wt% | *A. brasiliensis* | 2.2×10⁵ | 1.6×10⁴ | 1.8×10³ | 7×10² | 6×10² |
| | *C. albicans* | 3.5×10⁵ | 0 | 0 | 0 | 0 |
| | *P. aeruginosa* | 6.4×10⁶ | 0 | 0 | 1×10¹ | 1×10¹ |
| | *E. coli* | 4×10⁶ | 1.12×10⁵ | 1×10¹ | 0 | 0 |
| | *S. aureus* | 8×10⁶ | 2×10² | 1×10¹ | 0 | 0 |
| SN 0.5 wt% + AC 0.05 wt% | *A. brasiliensis* | 2.2×10⁵ | 1.67×10⁴ | 1.5×10³ | 1×10³ | 6×10² |
| | *C. albicans* | 3.5×10⁵ | 0 | 0 | 0 | 0 |
| | *P. aeruginosa* | 6.4×10⁶ | 0 | 0 | 1×10¹ | 0 |
| | *E. coli* | 4×10⁶ | 1.82×10⁵ | 1.04×10⁴ | 0 | 0 |
| | *S. aureus* | 8×10⁶ | 3×10² | 1×10¹ | 0 | 0 |
| SN 0.5 wt% + AC 0.01 wt% | *A. brasiliensis* | 2.2×10⁵ | 4.32×10⁴ | 4×10³ | 3.5×10³ | 2.3 ×10³ |
| | *C. albicans* | 3.5×10⁵ | 0 | 0 | 0 | 0 |
| | *P. aeruginosa* | 6.4×10⁶ | 0 | 0 | 3.2×10² | 0 |
| | *E. coli* | 4×10⁶ | 2×10² | 0 | 0 | 0 |
| | *S. aureus* | 8×10⁶ | 3.16×10⁴ | 1.2×10² | 0 | 1×10¹ |
| SN 0.5 wt% + BZ-2 0.2 wt% + AC 0.5 wt% | *A. brasiliensis* | 2.2×10⁵ | 1.49×10⁴ | 4.5×10³ | 3.4×10³ | 3×10² |
| | *C. albicans* | 3.5×10⁵ | 0 | 0 | 0 | 0 |
| | *P. aeruginosa* | 6.4×10⁶ | Full | Full | Full | Full |
| | *E. coli* | 4×10⁶ | Full | Full | 3×10¹ | 0 |
| | *S. aureus* | 8×10⁶ | 0 | 1×10¹ | 0 | 0 |
| SN 0.5 wt% + BZ-2 0.2 wt% + AC 0.1 wt% | *A. brasiliensis* | 2.2×10⁵ | 1.84×10⁴ | 3×10² | 3×10¹ | 0 |
| | *C. albicans* | 3.5×10⁵ | 0 | 0 | 0 | 0 |
| | *P. aeruginosa* | 6.4×10⁶ | 0 | 0 | 0 | 0 |
| | *E. coli* | 4×10⁶ | 0 | 0 | 0 | 0 |
| | *S. aureus* | 8×10⁶ | 0 | 1×10¹ | 0 | 0 |
| SN 0.5 wt% + BZ-2 0.2 wt% + AC 0.05 wt% | *A. brasiliensis* | 2.2×10⁵ | 1.21×10⁴ | 6×10² | 3.7×10² | 4×10² |
| | *C. albicans* | 3.5×10⁵ | 0 | 0 | 1×10¹ | 0 |
| | *P. aeruginosa* | 6.4×10⁶ | 0 | 1×10¹ | 2^{×}10¹ | 0 |
| | *E. coli* | 4×10⁶ | 0 | 0 | 0 | 0 |
| | *S. aureus* | 8×10⁶ | 0 | 1×10¹ | 0 | 0 |
| SN 0.5 wt% + BZ-2 0.2 wt% + AC 0.01 wt% | *A. brasiliensis* | 2.2×10⁵ | 1.23×10⁴ | 4.9×10³ | 3.6×10³ | 4.4×10⁴ |
| | *C. albicans* | 3.5×10⁵ | 0 | 0 | 0 | 0 |
| | *P. aeruginosa* | 6.4×10⁶ | 0 | 0 | 0 | 0 |
| | *E. coli* | 4×10⁶ | 0 | 0 | 0 | 0 |
| | *S. aureus* | 8×10⁶ | 0 | 0 | 0 | 0 |
| SN 0.2 wt% + BZ-2 0.2 wt% | *A. brasiliensis* | 2.2×10⁵ | 1.24×10⁴ | 1.2×10⁴ | 9.6×10³ | 9.5×10³ |
| | *C. albicans* | 3.5×10⁵ | 0 | 0 | 0 | 0 |
| | *P. aeruginosa* | 6.4×10⁶ | 0 | 0 | 0 | 0 |
| | *E. coli* | 4×10⁶ | 0 | 0 | 0 | 0 |
| | *S. aureus* | 8×10⁶ | 0 | 0 | 0 | 0 |
| SN 0.5 wt% + BZ-2 0.2 wt% | *A. brasiliensis* | 2.2×10⁵ | 1.56×10⁴ | 6.8×10³ | 6.5x10³ | 6.5×10³ |
| | *C. albicans* | 3.5×10⁵ | 0 | 0 | 0 | 0 |
| | *P. aeruginosa* | 6.4×10⁶ | 0 | 0 | 0 | 0 |
| | *E. coli* | 4×10⁶ | 0 | 0 | 0 | 1×10¹ |
| | *S. aureus* | 8×10⁶ | 0 | 0 | 0 | 0 |
| Cream + bacteria | *A. brasiliensis* | 2.2×10⁵ | 12×10⁵ | 9.6×10⁴ | 6.72×10⁴ | Full |
| | *C. albicans* | 3.5×10⁵ | 1.92×10⁵ | 2.56×10³ | 2.71×10³ | 0 |
| | *P. aeruginosa* | 6.4×10⁶ | 1.1×10³ | 1.3×10⁴ | Full | Full |
| | *E. coli* | 4×10⁶ | 4×10² | 1×10¹ | 1×10¹ | 0 |
| | *S. aureus* | 8×10⁶ | 2.22×10⁵ | 1.36×10⁴ | 6.1×10² | 0 |
| Sharomix 1% | *A. brasiliensis* | 2.2×10⁵ | 0 | 0 | 0 | 0 |
| | *C. albicans* | 3.5×10⁵ | 0 | 0 | 0 | 0 |
| | *P. aeruginosa* | 6.4×10⁶ | 0 | 0 | 0 | 0 |
| | *E. coli* | 4×10⁶ | 0 | 0 | 0 | 0 |
| | *S. aureus* | 8×10⁶ | 0 | 0 | 0 | 0 |

As observed from **Table** 7, the Acacia extract demonstrates some activity against *S. aureus* and *E. coli;* when combined with saponin an effect is observed also against candida.

However, when combining the Acacia and saponin extracts with benzoin, a significant effect is observed for all types of microorganism, especially for *A*. *brasiliensis.*

### Example 8: Composition with Olea Europaea, benzoin and saponin

A 10% Siam benzoin in MCT extract was prepared as in Example 1. A commercial extract of *Olea Europaea* (OE) leaves was used.

The *Olea* and benzoin extracts were added to a non-preserved cream formulation in different concentration, with and without *Sapindus mukorossi* extract. The results are provided in **Table 8.**

Tested Microorganisms: *E. coli* (ATCC 8739); S. *aureus* (ATCC 6538); *P. aeruginosa (ATCC 9027); C. albicans (ATCC 10231) and A. Brasiliensis (ATCC 16404).*

**Table 8: Total count for different microorganisms after contact with the examined ingredients**

| **Tested Formula** | **Tested Organism** | **Initial inoculums level (cfu/ml)** | **2 days** | **7 days** | **14 days** | **28 days** |
|---|---|---|---|---|---|---|
| OE 0.5 wt% | *A. brasiliensis* | 3.5×10⁵ | 5.6×10⁴ | 4×10⁴ | 4.4×10⁴ | 3.84×10⁴ |
| | *C. albicans* | 2×10⁵ | 1.92×10⁵ | 1.61×10³ | 9×10¹ | 0 |
| | *P. aeruginosa* | 2×10⁷ | 1.1×10⁴ | Full | Full | Full |
| | *E. coli* | 2×10⁶ | 8×10² | 0 | 0 | 0 |
| | *S. aureus* | 1.5×10⁶ | 8.8×10⁴ | 7×10² | 0 | 0 |
| OE 0.1 wt% | *A. brasiliensis* | 3.5×10⁵ | 5.6×10⁴ | 3.2×10⁴ | 3.6×10⁴ | 3.92×10⁴ |
| | *C. albicans* | 2×10⁵ | Full | 5.16×10³ | 1.1×10² | 0 |
| | *P. aeruginosa* | 2×10⁷ | 5.2×10³ | 1.66×10⁴ | Full | Full |
| | *E. coli* | 2×10⁶ | 2×10² | 0 | 0 | 0 |
| | *S. aureus* | 1.5×10⁶ | 1.06×10³ | 6.2×10² | 3×10¹ | 0 |
| OE 0.05 wt% | *A. brasiliensis* | 3.5×10⁵ | 4.8×10³ | 4.8×10³ | 2.4×10⁴ | 2.72×10⁴ |
| | *C. albicans* | 2×10⁵ | 1.44×10⁵ | 2.13×10³ | 3×10¹ | 0 |
| | *P. aeruginosa* | 2×10⁷ | 1.62×10⁴ | Full | Full | Full |
| | *E. coli* | 2×10⁶ | 2×10² | 0 | 0 | 0 |
| | *S. aureus* | 1.5×10⁶ | 8×10⁴ | 4.4×10⁴ | 0 | 0 |
| OE 0.01 wt% | *A. brasiliensis* | 3.5×10⁵ | 4×10⁴ | 4×10⁴ | 3.6×10⁴ | 4.08×10⁴ |
| | *C. albicans* | 2×10⁵ | 2.52×10⁵ | 1.05×10⁴ | 1.4×10⁵ | 0 |
| | *P. aeruginosa* | 2×10⁷ | 1.87×10⁴ | Full | Full | Full |
| | *E. coli* | 2×10⁶ | 8×10² | 1×10¹ | 0 | 0 |
| | *S. aureus* | 1.5×10⁶ | 1.44×10⁵ | 8.7×10² | 3×10¹ | 0 |
| SN 0.2 wt% + OE 0.5 wt% | *A. brasiliensis* | 3.5×10⁵ | 32×10⁴ | 1.33×10⁴ | 1×10⁴ | 9.7×10³ |
| | *C. albicans* | 2×10⁵ | 0 | 0 | 0 | 0 |
| | *P. aeruginosa* | 2×10⁷ | 4×10² | 1.1×10² | 8×10¹ | Full |
| | *E. coli* | 2×10⁶ | 5×10² | 0 | 0 | 0 |
| | *S. aureus* | 1.5×10⁶ | 8.8×10⁴ | 6×10² | 0 | 0 |
| SN 0.2 wt% + OE 0.1 wt% | *A. brasiliensis* | 3.5×10⁵ | 2.8×10⁴ | 1.66×10⁴ | 1.13×10⁴ | 9.8×10³ |
| | *C. albicans* | 2×10⁵ | 0 | 0 | 0 | 0 |
| | *P. aeruginosa* | 2×10⁷ | 2×10² | 6.3 ×10³ | Full | Full |
| | *E. coli* | 2×10⁶ | 1×10² | 0 | 0 | 0 |
| | *S. aureus* | 1.5×10⁶ | 1.16×10⁵ | 1×10³ | 0 | 0 |
| SN 0.2 wt% + OE 0.05 wt% | *A. brasiliensis* | 3.5×10⁵ | 3.6×10⁴ | 1.73×10⁴ | 1.6×10⁴ | 1.09×10⁴ |
| | *C. albicans* | 2×10⁵ | 1×10² | 0 | 0 | 0 |
| | *P. aeruginosa* | 2×10⁷ | 1×10² | 3×10¹ | 1×10² | Full |
| | *E. coli* | 2×10⁶ | 5×10² | 0 | 0 | 0 |
| | *S. aureus* | 1.5×10⁶ | 1.2×10⁵ | 1.94×10³ | 0 | 0 |
| SN 0.2 wt% + OE 0.01 wt% | *A. brasiliensis* | 3.5×10⁵ | 32×10⁴ | 1.93×10⁴ | 1.4×10⁵ | 1.24×10⁴ |
| | *C. albicans* | 2×10⁵ | 1×10² | 0 | 0 | 0 |
| | *P. aeruginosa* | 2×10⁷ | 1×10² | 1.3×10² | Full | Full |
| | *E. coli* | 2×10⁶ | 1×10² | 0 | 0 | 0 |
| | *S. aureus* | 1.5×10⁶ | 1.33×10⁵ | 1.8×10³ | 3×10¹ | 0 |
| SN 0.2 wt% + BZ 0.2 wt% + OE 0.5 wt% | *A. brasiliensis* | 3.5×10⁵ | 8.3 ×10³ | 2.5×10³ | 2.4×10³ | 1.3×10³ |
| | *C. albicans* | 2×10⁵ | 1×10² | 0 | 0 | 0 |
| | *P. aeruginosa* | 2×10⁷ | 0 | 0 | 0 | 0 |
| | *E. coli* | 2×10⁶ | 0 | 0 | 0 | 0 |
| | *S. aureus* | 1.5×10⁶ | 0 | 0 | 0 | 0 |
| SN 0.2 wt% + BZ 0.2 wt% + OE 0.1 wt% | *A. brasiliensis* | 3.5×10⁵ | 9×10³ | 3.1×10³ | 2.5×10³ | 1.9×10³ |
| | *C. albicans* | 2×10⁵ | 0 | 0 | 0 | 0 |
| | *P. aeruginosa* | 2×10⁷ | 0 | 0 | 0 | 0 |
| | *E. coli* | 2×10⁶ | 0 | 0 | 0 | 0 |
| | *S. aureus* | 1.5×10⁶ | 0 | 0 | 0 | 0 |
| SN 0.2 wt% + BZ 0.2 wt% + OE 0.05 wt% | *A. brasiliensis* | 3.5×10⁵ | 7.1×10³ | 2.3 ×10³ | 2.3 ×10³ | 1.9×10³ |
| | *C. albicans* | 2×10⁵ | 0 | 0 | 0 | 0 |
| | *P. aeruginosa* | 2×10⁷ | 0 | 0 | 0 | 0 |
| | *E. coli* | 2×10⁶ | 0 | 0 | 0 | 0 |
| | *S. aureus* | 1.5×10⁶ | 0 | 0 | 0 | 0 |
| SN 0.2 wt% + BZ 0.2 wt% + OE 0.01 wt% | *A. brasiliensis* | 3.5×10⁵ | 7.3×10³ | 3.6×10³ | 1.7×10³ | 1.4×10⁵ |
| | *C. albicans* | 2×10⁵ | 7×10² | 0 | 0 | 0 |
| | *P. aeruginosa* | 2×10⁷ | 0 | 0 | 0 | 0 |
| | *E. coli* | 2×10⁶ | 0 | 0 | 0 | 0 |
| | *S. aureus* | 1.5×10⁶ | 0 | 0 | 0 | 0 |
| SN 0.5%wt% + OE 0.5 wt% | *A. brasiliensis* | 3.5×10⁵ | 2.8×10⁴ | 4.8×10³ | 4×10³ | 3.1×10³ |
| | *C. albicans* | 2×10⁵ | 0 | 0 | 0 | 0 |
| | *P. aeruginosa* | 2×10⁷ | 8×10² | 4.03 ×10³ | 2.5×10⁴ | Full |
| | *E. coli* | 2×10⁶ | 2×10² | 0 | 0 | 0 |
| | *S. aureus* | 1.5×10⁶ | 1.08×10⁵ | 1.2×10³ | 4×10¹ | 0 |
| SN 0.5 wt% +OE0.1 wt% | *A. brasiliensis* | 3.5×10⁵ | 2.8×10⁴ | 5×10³ | 3.2×10³ | 2.3×10³ |
| | *C. albicans* | 2×10⁵ | 0 | 0 | 0 | 0 |
| | *P. aeruginosa* | 2×10⁷ | 1×10² | Full | Full | Full |
| | *E. coli* | 2×10⁶ | 5×10² | 0 | 0 | 0 |
| | *S. aureus* | 1.5×10⁶ | 1.96×10⁵ | 1.1×10³ | 2×10¹ | 1×10¹ |
| SN 0.5 wt% + OE 0.05 wt% | *A. brasiliensis* | 3.5×10⁵ | 4.8×10⁴ | 1.1×10⁴ | 7.7×10³ | 62×10³ |
| | *C. albicans* | 2×10⁵ | 0 | 0 | 0 | 0 |
| | *P. aeruginosa* | 2×10⁷ | 2×10² | Full | Full | Full |
| | *E. coli* | 2×10⁶ | 2×10² | 0 | 0 | 0 |
| | *S. aureus* | 1.5×10⁶ | 1.12×10⁵ | 1.4×10³ | 0 | 0 |
| SN 0.5 wt% + OE 0.01 wt% | *A. brasiliensis* | 3.5×10⁵ | 3.2×10⁴ | 1×10⁴ | 7×10³ | 62×10³ |
| | *C. albicans* | 2×10⁵ | 0 | 0 | 0 | 0 |
| | *P. aeruginosa* | 2×10⁷ | 2×10² | 2×10⁴ | Full | Full |
| | *E. coli* | 2×10⁶ | 5×10² | 0 | 0 | 0 |
| | *S. aureus* | 1.5×10⁶ | 1.6×10³ | 5×10³ | 6×10¹ | 0 |
| SN 0.5 wt% + BZ 0.2 wt% + OE 0.5 wt% | *A. brasiliensis* | 3.5×10⁵ | 62×10³ | 2.3 ×10³ | 2.1×10³ | 1.8×10³ |
| | *C. albicans* | 2×10⁵ | 0 | 0 | 10 | 0 |
| | *P. aeruginosa* | 2×10⁷ | 0 | 0 | 10 | 0 |
| | *E. coli* | 2×10⁶ | 0 | 20 | 0 | 0 |
| | *S. aureus* | 1.5×10⁶ | 0 | 0 | 0 | 0 |
| SN 0.5 wt% + BZ 0.2 wt% + OE 0. 1 wt% | *A. brasiliensis* | 3.5×10⁵ | 5.4×10³ | 300 | 1.1×10³ | 9×10² |
| | *C. albicans* | 2×10⁵ | 0 | 0 | 0 | 0 |
| | *P. aeruginosa* | 2×10⁷ | 0 | 500 | 20 | 10 |
| | *E. coli* | 2×10⁶ | 0 | 0 | 0 | 0 |
| | *S. aureus* | 1.5×10⁶ | 100 | 200 | 30 | 30 |
| SN 0.5 wt% + BZ 0.2 wt% + OE 0.05 wt% | *A. brasiliensis* | 3.5×10⁵ | 6.4×10³ | 2.3 ×10³ | 6×10² | 4×10² |
| | *C. albicans* | 2×10⁵ | 1×10² | 0 | 0 | 0 |
| | *P. aeruginosa* | 2×10⁷ | 0 | 0 | 0 | 0 |
| | *E. coli* | 2×10⁶ | 0 | 0 | 0 | 0 |
| | *S. aureus* | 1.5×10⁶ | 0 | 0 | 0 | 0 |
| SN 0.5 wt% + BZ 0.2 wt% + OE 0.01 wt% | *A. brasiliensis* | 3.5×10⁵ | 62×10³ | 2.4×10³ | 2×10³ | 1.6×10³ |
| | *C. albicans* | 2×10⁵ | 0 | 0 | 0 | 0 |
| | *P. aeruginosa* | 2×10⁷ | 0 | 0 | 0 | 0 |
| | *E. coli* | 2×10⁶ | 0 | 0 | 0 | 0 |
| | *S. aureus* | 1.5×10⁶ | 0 | 0 | 0 | 0 |
| Cream + bacteria | *A. brasiliensis* | 3.5×10⁵ | 3.2×10⁴ | 6.1×10⁴ | Full | 3.12×10⁴ |
| | *C. albicans* | 2×10⁵ | 1.22×10⁵ | 3.5×10² | 0 | 0 |
| | *P. aeruginosa* | 2×10⁷ | 4.9×10³ | Full | Full | Full |
| | *E. coli* | 2×10⁶ | 1.4×10³ | 0 | 40 | 0 |
| | *S. aureus* | 1.5×10⁶ | 6.2×10⁴ | 3×10² | 3×10² | 0 |
| Sharomix 1% | *A. brasiliensis* | 3.5×10⁵ | 8×10² | 0 | 0 | 0 |
| | *C. albicans* | 2×10⁵ | 0 | 0 | 0 | 0 |
| | *P. aeruginosa* | 2×10⁷ | 0 | 0 | 10 | 0 |
| | *E. coli* | 2×10⁶ | 0 | 0 | 0 | 0 |
| | *S. aureus* | 1.5×10⁶ | 0 | 0 | 0 | 0 |

As observed from **Table 8,** the *Olea* extract demonstrates some activity against *S. aureus, C. albicans* and *E. coli;* when combined with saponin another reduction in the number of colonies is observed.

However, when combining the *Olea* and saponin extracts with benzoin, a dramatic effect is observed for all types of microorganisms, completely eliminating most microorganisms and significantly reducing the number of *A. brasiliensis* colonies.

### Example 9: Composition with Terminalia, benzoin and saponin

10% Siam benzoin (BZ-2) in MCT extract was prepared as in Example 1. A commercial extract of 25 wt% *Terminalia Bellerica* (TB) fruit was used.

The *Terminalia* and benzoin extracts were added to a non-preserved cream formulation in different concentration, with and without *Sapindus mukorossi* extract. The results are provided in **Table 9.**

Tested Microorganisms: *E. coli* (ATCC 8739); S. *aureus* (ATCC 6538); *P. aeruginosa (ATCC 9027); C. albicans (ATCC 10231) and A. Brasiliensis (ATCC 16404).*

**Table 9: Total count for different microorganisms after contact with the examined ingredients**

| **Tested Formula** | **Tested organism** | **Initial inoculums level (cfu/ml)** | **2 days** | **7 days** | **14 days** | **28 days** |
|---|---|---|---|---|---|---|
| TB 0.05 wt% | *A. brasiliensis* | 4.5×10⁵ | Full | 4.3×10⁴ | N/A | 2×10⁴ |
| | *C. albicans* | 1.7×10⁵ | Full | Full | 1.6×10³ | 70 |
| | *P. aeruginosa* | 4.5×10⁶ | 0 | Full | Full | Full |
| | *E. coli* | 1.2×10⁶ | 1×10³ | 180 | 0 | 0 |
| | *S. aureus* | 2×10⁶ | 8.8×10³ | 1.6×10³ | 0 | 0 |
| TB 0.03 wt% | *A. brasiliensis* | 4.5×10⁵ | Full | 4.8×10⁴ | 3.5×10⁴ | 2.4×10⁴ |
| | *C. albicans* | 1.7×10⁵ | Full | Full | 1×10⁴ | 170 |
| | *P. aeruginosa* | 4.5×10⁶ | 0 | 2.5×10³ | 3.8×10³ | Full |
| | *E. coli* | 1.2×10⁶ | 1.2×10³ | 60 | 0 | 10 |
| | *S. aureus* | 2×10⁶ | 2.5×10³ | 560 | 0 | 0 |
| TB 0.01 wt% | *A. brasiliensis* | 4.5×10⁵ | Full | 5.2×10⁴ | Full | Full |
| | *C. albicans* | 1.7×10⁵ | Full | Full | 2.6×10³ | 20 |
| | *P. aeruginosa* | 4.5×10⁶ | 0 | Full | 1.3×10³ | Full |
| | *E. coli* | 1.2 ×10⁶ | 200 | 60 | 0 | 0 |
| | *S. aureus* | 2×10⁶ | 1×10⁴ | 1.2×10³ | 20 | 0 |
| TB 0.005 wt% | *A. brasiliensis* | 4.5×10⁵ | Full | 5.2×10⁴ | Full | Full |
| | *C. albicans* | 1.7×10⁵ | Full | Full | Full | 40 |
| | *P. aeruginosa* | 4.5×10⁶ | 0 | Full | Full | Full |
| | *E. coli* | 1.2×10⁶ | 100 | 10 | 0 | 0 |
| | *S. aureus* | 2×10⁶ | 2.6×10⁴ | 2.8×10⁴ | 240 | 20 |
| SN 0.5 wt% + TB 0.05 wt% | *A. brasiliensis* | 4.5×10⁵ | 5×10² | 700 | 1.5×10³ | 800 |
| | *C. albicans* | 1.7×10⁵ | 0 | 0 | 0 | 0 |
| | *P. aeruginosa* | 4.5×10⁶ | 0 | 0 | 10 | 0 |
| | *E. coli* | 1.2×10⁶ | 800 | 60 | 0 | 0 |
| | *S. aureus* | 2×10⁶ | 5×10² | 0 | 0 | 0 |
| SN 0.5 wt% + TB 0.01 wt% | *A. brasiliensis* | 4.5×10⁵ | 1.4×10⁴ | 9.5×10³ | 1.7×10³ | 7.7×10³ |
| | *C. albicans* | 1.7×10⁵ | 0 | 0 | 0 | 0 |
| | *P. aeruginosa* | 4.5×10⁶ | 0 | 0 | 0 | 0 |
| | *E. coli* | 1.2×10⁶ | 0 | 10 | 0 | 0 |
| | *S. aureus* | 2×10⁶ | 9.6×10⁴ | 1×10⁴ | 120 | 0 |
| SN 0.5 wt% + BZ-2 0.2 wt% + TB 0.05 wt% | *A. brasiliensis* | 4.5×10⁵ | 7×10³ | 1.9×10³ | 800 | 300 |
| | *C. albicans* | 1.7×10⁵ | 0 | 0 | 0 | 0 |
| | *P. aeruginosa* | 4.5×10⁶ | 0 | 0 | 0 | 0 |
| | *E. coli* | 1.2×10⁶ | 300 | 110 | 0 | 0 |
| | *S. aureus* | 2×10⁶ | 0 | 0 | 0 | 0 |
| SN 0.5 wt% + BZ-2 0.2 wt% + TB 0.03 wt% | *A. brasiliensis* | 4.5×10⁵ | 5×10² | 1.6×10³ | 1.1×10³ | 1×10³ |
| | *C. albicans* | 1.7×10⁵ | 0 | 0 | 0 | 0 |
| | *P. aeruginosa* | 4.5×10⁶ | 0 | 0 | 0 | 0 |
| | *E. coli* | 1.2×10⁶ | 0 | 0 | 0 | 0 |
| | *S. aureus* | 2×10⁶ | 0 | 0 | 0 | 0 |
| SN 0.5 wt% + BZ-2 0.2 wt% + TB 0.01 wt% | *A. brasiliensis* | 4.5×10⁵ | 9.5×10³ | 2.8×10³ | 2.9×10³ | 2×10³ |
| | *C. albicans* | 1.7×10⁵ | 0 | 0 | 0 | 0 |
| | *P. aeruginosa* | 4.5×10⁶ | 0 | Full | 0 | Full |
| | *E. coli* | 1.2×10⁶ | 0 | 0 | 0 | 10 |
| | *S. aureus* | 2×10⁶ | 0 | 10 | 0 | 0 |
| SN 0.5 wt% +BZ-2 0.2 wt% + TB 0.005 wt% | *A. brasiliensis* | 4.5×10⁵ | 1×10⁴ | 2.7×10³ | 3.8×10³ | 2.6×10⁴ |
| | *C. albicans* | 1.7×10⁵ | 0 | 0 | 0 | 0 |
| | *P. aeruginosa* | 4.5×10⁶ | 0 | 0 | 0 | 0 |
| | *E. coli* | 1.2×10⁶ | 0 | 0 | 0 | 0 |
| | *S. aureus* | 2×10⁶ | 0 | 0 | 0 | 0 |
| SN 0.5 wt% +BZ-2 0.2 wt% | *A. brasiliensis* | 4.5×10⁵ | 1.2×10⁴ | 9×10⁴ | 6.6×10³ | 6.7×10³ |
| | *C. albicans* | 1.7×10⁵ | 0 | 0 | 0 | 0 |
| | *P. aeruginosa* | 4.5×10⁶ | 0 | 0 | 0 | 0 |
| | *E. coli* | 1.2×10⁶ | 0 | 0 | 0 | 0 |
| | *S. aureus* | 2×10⁶ | 0 | 0 | 0 | 0 |
| SN 0.2 wt% +BZ-2 0.2 wt% | *A. brasiliensis* | 4.5×10⁵ | 1.7×10⁵ | 1.4×10⁴ | 1.6×10³ | 1.3×10⁴ |
| | *C. albicans* | 1.7×10⁵ | 0 | 0 | 0 | 0 |
| | *P. aeruginosa* | 4.5×10⁶ | 0 | 0 | 0 | 0 |
| | *E. coli* | 1.2×10⁶ | 0 | 10 | 0 | 0 |
| | *S. aureus* | 2×10⁶ | 0 | 0 | 0 | 0 |
| Cream + bacteria | *A. brasiliensis* | 4.5×10⁵ | Full | 6×10⁴ | 5.5×10⁴ | Full |
| | *C. albicans* | 1.7×10⁵ | Full | 3.5×10⁴ | 350 | 10 |
| | *P. aeruginosa* | 4.5×10⁶ | 1×10³ | Full | Full | Full |
| | *E. coli* | 1.2×10⁶ | 200 | 10 | 0 | 0 |
| | *S. aureus* | 2×10⁶ | 4×10⁴ | 1.4×10³ | 20 | 0 |
| Sharomix 1% | *A. brasiliensis* | 4.5×10⁵ | 1.3×10³ | 0 | 0 | 0 |
| | *C. albicans* | 1.7×10⁵ | 0 | 0 | 0 | 0 |
| | *P. aeruginosa* | 4.5×10⁶ | 0 | 0 | 0 | 0 |
| | *E. coli* | 1.2×10⁶ | 0 | 0 | 0 | 0 |
| | *S. aureus* | 2×10⁶ | 0 | 0 | 0 | 0 |

As observed from **Table 9,** the *Terminalia* extract demonstrates some activity against *S. aureus, C. albicans* and *E. coli;* when combined with saponin an additional unexpected effect is observed for *P*. *aeruginosa.*

### Example 10: Composition with Wasabia, benzoin and saponin

10% Sumatra benzoin (BZ-1) in MCT extract was prepared as in Example 1. A commercial extract of 25 wt% *Wasabia japonica* (WS) bulb was used.

The *Wasabia* and benzoin extracts were added to a non-preserved cream formulation in different concentration, with and without *Sapindus mukorossi* extract. The results are provided in **Table 10.**

Tested Microorganisms: *E. coli* (ATCC 8739); S. *aureus* (ATCC 6538); *P. aeruginosa (ATCC 9027); C. albicans (ATCC 10231) and A. Brasiliensis (ATCC 16404).*

**Table 10: Total count for different microorganisms after contact with the examined ingredients**

| **Tested Formula** | **Tested Organism** | **Initial inoculums level (cfu/ml)** | **2 days** | **4 days** | **6 days** | **14 days** | **28 days** |
|---|---|---|---|---|---|---|---|
| Sn 0.5wt% | *A. brasiliensis* | 2.5×10⁵ | 2×10⁴ | 1.08×10⁵ | 1.02×10⁴ | 7.7×10³ | 1.2×10⁴ |
| | *C. albicans* | 3×10² | 0 | 0 | 0 | 0 | 0 |
| | *P. aeruginosa* | 9.4×10⁶ | Full | Full | Full | Full | Full |
| | *E. coli* | 4×10⁶ | 9.5×10² | 5×10² | 0 | 0 | 0 |
| | *S. aureus* | 9×10⁶ | Full | Full | Full | Full | 2.82×10³ |
| WS 0.2 wt% | *A. brasiliensis* | 2.5×10⁵ | Full | 5.2×10⁴ | 3.6×10⁴ | 8.7×10³ | 4.5×10³ |
| | *C. albicans* | 3×10⁵ | Full | Full | Full | Full | Full |
| | *P. aeruginosa* | 9.4×10⁶ | Full | Full | Full | Full | Full |
| | *E. coli* | 4×10⁶ | Full | 2.8×10⁴ | 1.69×10³ | 4×10¹ | 0 |
| | *S. aureus* | 9×10⁶ | Full | Full | Full | Full | Full |
| WS 0.5 wt% | *A. brasiliensis* | 2.5×10⁵ | Full | 4×10⁴ | 2.32×10⁴ | 2.3 ×10³ | 2×10⁴ |
| | *C. albicans* | 3×10⁵ | Full | Full | Full | Full | Full |
| | *P. aeruginosa* | 9.4×10⁶ | 0 | 1×10¹ | 0 | 0 | 0 |
| | *E. coli* | 4×10⁶ | Full | 8×10³ | 4.8×10³ | 2.1×10² | 0 |
| | *S. aureus* | 9×10⁶ | Full | Full | Fulll | Full | Full |
| BZ 0.05 wt% | *A. brasiliensis* | 2.5×10⁵ | Full | 4.8×10⁴ | 2.64×10⁴ | 2.52×10⁴ | 1.6×10³ |
| | *C. albicans* | 3×10⁵ | Full | Full | Full | Full | Full |
| | *P. aeruginosa* | 9.4×10⁶ | Full | Full | Full | Full | Full |
| | *E. coli* | 4×10⁶ | Full | 1.8×10³ | 5×10² | 0 | 0 |
| | *S. aureus* | 9×10⁶ | Full | Full | 3.2×10³ | 0 | 0 |
| BZ 0.1 wt% | *A. brasiliensis* | 2.5×10⁵ | Full | 3.6×10⁴ | 2.4×10⁴ | 2.92×10⁴ | 2×10⁴ |
| | *C. albicans* | 3×10⁵ | Full | Full | Full | 1.05×10⁴ | 2×10³ |
| | *P. aeruginosa* | 9.4×10⁶ | Full | Full | Full | Full | Full |
| | *E. coli* | 4×10⁶ | Full | 1.8×10³ | 3×10² | 1×10¹ | 0 |
| | *S. aureus* | 9×10⁶ | Full | 8×10¹ | 0 | 0 | 0 |
| BZ 0.2 wt% | *A. brasiliensis* | 2.5×10⁵ | Full | 4×10⁴ | 1.72×10⁴ | 2.44×10⁴ | 1.6×10³ |
| | *C. albicans* | 3×10⁵ | Full | Full | Full | 7.3×10³ | 6.5×10² |
| | *P. aeruginosa* | 9.4×10⁶ | 9.6×10³ | 4×10³ | 1.2×10⁴ | Full | Full |
| | *E. coli* | 4×10⁶ | 1.44×10⁴ | 3.7×10³ | 4×10¹ | 0 | 0 |
| | *S. aureus* | 9×10⁶ | 3.6×10³ | 0 | 0 | 0 | 0 |
| BZ 0.5 wt% | *A. brasiliensis* | 2.5×10⁵ | Full | 3.84×10⁴ | 2.96×10⁴ | 2.08×10⁴ | 1.6×10⁴ |
| | *C. albicans* | 3×10⁵ | Full | Full | Full | Full | 3.6×10³ |
| | *P. aeruginosa* | 9.4×10⁶ | 4.8×10³ | 2×10³ | 1.2×10² | 0 | 0 |
| | *E. coli* | 4×10⁶ | Full | 1.08×10⁵ | 4×10¹ | 0 | 0 |
| | *S. aureus* | 9×10⁶ | 1×10¹ | 0 | 0 | 0 | 0 |
| SN 0.5 wt% + WS 0.2 wt% | *A. brasiliensis* | 2.5×10⁵ | 6×10³ | 3.6×10³ | 1.9×10³ | 7×10² | 7×10² |
| | *C. albicans* | 3×10⁵ | 0 | 0 | 0 | 0 | 0 |
| | *P. aeruginosa* | 9.4×10⁶ | 5.6×10³ | Full | 1.4×10³ | Full | Full |
| | *E. coli* | 4×10⁶ | 5.2×10³ | 1.16×10³ | 1.3×10² | 0 | 0 |
| | *S. aureus* | 9×10⁶ | Full | Full | Full | Full | 1.1×10³ |
| SN 0.5 wt% + WS 0.5 wt% | *A. brasiliensis* | 2.5×10⁵ | 2×10⁴ | 6.3 ×10³ | 3.3×10³ | 1.76×10³ | 5×10² |
| | *C. albicans* | 3×10⁵ | 0 | 0 | 0 | 0 | 0 |
| | *P. aeruginosa* | 9.4×10⁶ | 2.6×10² | 0 | 0 | 0 | 0 |
| | *E. coli* | 4×10⁶ | 1.2×10⁴ | 4.8×10⁴ | 1.6×10³ | 0 | 0 |
| | *S. aureus* | 9×10⁶ | Full | Full | Full | 0 | 2.75×10⁴ |
| | *A. brasiliensis* | 2.5×10⁵ | 5.1×10³ | 2.7×10³ | 2.1×10³ | 1.48×10³ | 5×10² |
| SN 0.5 wt% + BZ 0.05 wt% | *C. albicans* | 3×10² | 0 | 0 | 0 | 0 | 0 |
| | *P. aeruginosa* | 9.4×10⁶ | Full | Full | Full | Full | Full |
| | *E. coli* | 4×10⁶ | 2.6×10⁴ | 6×10¹ | 2×10¹ | 0 | 0 |
| | *S. aureus* | 9×10⁶ | Full | 1×10¹ | 0 | 0 | 0 |
| SN 0.5 wt% + BZ 0.1 wt% | *A. brasiliensis* | 2.5×10⁵ | 9.5×10³ | 2.2×10³ | 5×10² | 7.5×10² | 3×10² |
| | *C. albicans* | 3×10² | 0 | 0 | 0 | 0 | 0 |
| | *P. aeruginosa* | 9.4×10⁶ | Full | Full | Full | Full | Full |
| | *E. coli* | 4×10⁶ | 5.2×10³ | 0 | 1×10¹ | 0 | 0 |
| | *S. aureus* | 9×10⁶ | 0 | 0 | 0 | 0 | 0 |
| SN 0.5 wt% + BZ 0.2 wt% | *A. brasiliensis* | 2.5×10⁵ | 7.4×10³ | 2.4×10³ | 1.7×10³ | 1.16×10⁴ | 3×10² |
| | *C. albicans* | 3×10² | 0 | 0 | 0 | 0 | 0 |
| | *P. aeruginosa* | 9.4×10⁶ | Full | 9×10³ | 2.8×10⁴ | 1.8×10³ | Full |
| | *E. coli* | 4×10⁶ | 2.4×10⁴ | 1×10² | 1×10¹ | 0 | 0 |
| | *S. aureus* | 9×10⁶ | 0 | 0 | 0 | 0 | 0 |
| SN 0.5 wt% + BZ 0.5 wt% | *A. brasiliensis* | 2.5×10⁵ | 2.4×10⁴ | 5.1×10³ | 4×10³ | 1.24×10³ | 1×10² |
| | *C. albicans* | 3×10² | 3×10² | 1×10¹ | 2×10¹ | 0 | 0 |
| | *P. aeruginosa* | 9.4×10⁶ | 7.2×10³ | 5×10² | 0 | 0 | 0 |
| | *E. coli* | 4×10⁶ | Full | 6×10¹ | 5×10² | 0 | 0 |
| | *S. aureus* | 9×10⁶ | 0 | 0 | 0 | 0 | 0 |
| BZ 0.05 wt% + WS 0.2wt% | *A. brasiliensis* | 2.5×10⁵ | Full | 4.48×10⁴ | Full | 2.24×10⁴ | 4×10³ |
| | *C. albicans* | 3×10² | Full | Full | Full | Full | Full |
| | *P. aeruginosa* | 9.4×10⁶ | 9.4×10² | 0 | 0 | 0 | 0 |
| | *E. coli* | 4×10⁶ | Full | 3.1×10² | 1.6×10³ | 1×10¹ | 0 |
| | *S. aureus* | 9×10⁶ | Full | 8.8×10³ | 6×10¹ | 0 | 0 |
| BZ 0.1 wt% + WS 0.2wt% | *A. brasiliensis* | 2.5×10⁵ | Full | 5.6×10⁴ | Full | 2.64×10⁴ | 1×10² |
| | *C. albicans* | 3×10² | Full | Full | Full | Full | 3.36×10³ |
| | *P. aeruginosa* | 9.4×10⁶ | 3.5×10² | 0 | 0 | 0 | 0 |
| | *E. coli* | 4×10⁶ | Full | 2.88×10³ | 1.22×10³ | 10 | 0 |
| | *S. aureus* | 9×10⁶ | Full | 0 | 0 | 0 | 0 |
| BZ 0.2 wt% +WS 0.2wt% | *A. brasiliensis* | 2.5×10⁵ | Full | 4.8×10⁴ | 2.24×10⁴ | 2.04×10⁴ | 3×10² |
| | *C. albicans* | 3×10² | Full | Full | Full | 7.36×10³ | 7.9×10² |
| | *P. aeruginosa* | 9.4×10⁶ | 2.3×10² | 1×10¹ | 0 | 0 | 0 |
| | *E. coli* | 4×10⁶ | Full | 8×10³ | 8.5×10² | 1×10¹ | 0 |
| | *S. aureus* | 9×10⁶ | Full | 0 | 0 | 0 | 0 |
| BZ 0.5wt% + WS 0.2wt% | *A. brasiliensis* | 2.5×10⁵ | Full | 1.68×10⁴ | 3.12×10⁴ | 2.24×10⁴ | 8×10³ |
| | *C. albicans* | 3×10² | Full | Full | Full | Full | 4.32×10³ |
| | *P. aeruginosa* | 9.4×10⁶ | 2×10¹ | 0 | 0 | 0 | 0 |
| | *E. coli* | 4×10⁶ | Full | 1×10⁴ | 2.8×10⁴ | 0 | 0 |
| | *S. aureus* | 9×10⁶ | 0 | 0 | 0 | 0 | 0 |
| BZ 0.05wt% +WS 0.5wt% | *A. brasiliensis* | 2.5×10⁵ | Full | 1.8×10⁴ | 5.6×10⁴ | 2.8×10⁴ | 4×10² |
| | *C. albicans* | 3×10² | Full | Full | Full | Full | Full |
| | *P. aeruginosa* | 9.4×10⁶ | 2×10¹ | 0 | 0 | 0 | 0 |
| | *E. coli* | 4×10⁶ | Full | 1.2×10⁴ | 4.8×10⁴ | 1×10¹ | 0 |
| | *S. aureus* | 9×10⁶ | Full | Full | 5.8×10² | 0 | 0 |
| BZ 0.1wt% + WS 0.5wt% | *A. brasiliensis* | 2.5×10⁵ | Full | 4.48×10⁴ | 52×10⁴ | 3.3×10² | 0 |
| | *C. albicans* | 3×10⁵ | Full | Full | Full | Full | 42×10³ |
| | *P. aeruginosa* | 9.4×10⁶ | 0 | 0 | 0 | 0 | 0 |
| | *E. coli* | 4×10⁶ | Full | Full | 4.8×10³ | 0 | 0 |
| | *S. aureus* | 9×10⁶ | Full | 8×10³ | 0 | 0 | 0 |
| BZ 0.2wt% + WS 0.5 wt% | *A. brasiliensis* | 2.5×10⁵ | Full | 4×10⁴ | 4×10⁴ | 2.64×10⁴ | 1×10² |
| | *C. albicans* | 3×10⁵ | Full | Full | Full | Full | 2.32×10³ |
| | *P. aeruginosa* | 9.4×10⁶ | 0 | 0 | 0 | 0 | 0 |
| | *E. coli* | 4×10⁶ | Full | Full | 6×10³ | 0 | 0 |
| | *S. aureus* | 9×10⁶ | Full | 0 | 0 | 0 | 0 |
| BZ 0.5wt% + WS 0.5wt% | *A. brasiliensis* | 2.5×10⁵ | Full | Full | 3.92×10⁴ | 2.84×10⁴ | 0 |
| | *C. albicans* | 3×10⁵ | Full | Full | Full | Full | 2.32×10³ |
| | *P. aeruginosa* | 9.4×10⁶ | 0 | 0 | 0 | 0 | 0 |
| | *E. coli* | 4×10⁶ | Full | Full | Full | 2^{X}10¹ | 0 |
| | *S. aureus* | 9×10⁶ | Full | 0 | 0 | 0 | 0 |
| SN 0.5wt% +BZ 0.05wt% +WS 0.2wt% | *A. brasiliensis* | 2.5×10⁵ | 4×10³ | 9×10² | 1.1×10³ | 3×10⁵ | 1×10² |
| | *C. albicans* | 3×10⁵ | 0 | 0 | 0 | 0 | 0 |
| | *P. aeruginosa* | 9.4×10⁶ | 3×10⁵ | 0 | 0 | 0 | 0 |
| | *E. coli* | 4×10⁶ | Full | 6×10³ | 4.8×10² | 0 | 0 |
| | *S. aureus* | 9×10⁶ | 7.5×10² | 0 | 0 | 0 | 0 |
| SN 0.5wt% +BZ 0.1 wt% +WS 0.2wt% | *A. brasiliensis* | 2.5×10⁵ | 4×10³ | 7×10² | 4×10² | 3×10⁵ | 7×10¹ |
| | *C. albicans* | 3×10⁵ | 0 | 0 | 0 | 0 | 0 |
| | *P. aeruginosa* | 9.4×10⁶ | 1.5×10² | 0 | 0 | 0 | 0 |
| | *E. coli* | 4×10⁶ | Full | 2.48×10³ | 4.6×10² | 0 | 0 |
| | *S. aureus* | 9×10⁶ | Full | 7×10¹ | 0 | 0 | 0 |
| SN 0.5wt% +BZ 0.2wt% +0.2 wt% | *A. brasiliensis* | 2.5×10⁵ | 7.3×10³ | 1.7×10³ | 1.2×10³ | 4.7×10² | 1.2×10² |
| | *C. albicans* | 3×10⁵ | 0 | 0 | 0 | 0 | 0 |
| | *P. aeruginosa* | 9.4×10⁶ | 8×10¹ | 0 | 0 | 0 | 0 |
| | *E. coli* | 4×10⁶ | Full | 3.6×10³ | 5×10² | 0 | 0 |
| | *S. aureus* | 9×10⁶ | 0 | 0 | 0 | 0 | 0 |
| SN 0.5wt% +BZ 0.5wt% +WS 0.2 wt% | *A. brasiliensis* | 2.5×10⁵ | 1.8×10⁴ | 6.5×10³ | 2.5×10³ | 9×10² | 1×10² |
| | *C. albicans* | 3×10⁵ | 9×10¹ | 0 | 0 | 0 | 0 |
| | *P. aeruginosa* | 9.4×10⁶ | 1×10¹ | 0 | 0 | 0 | 0 |
| | *E. coli* | 4×10⁶ | Full | 6×10³ | 4×10³ | 0 | 0 |
| | *S. aureus* | 9×10⁶ | 0 | 0 | 0 | 0 | 0 |
| SN 0.5wt% +BZ 0.05wt% +WS 0.5wt% | *A. brasiliensis* | 2.5×10⁵ | 6.4×10³ | 7×10² | 1×10² | 9×10¹ | 1×10² |
| | *C. albicans* | 3×10⁵ | 0 | 0 | 0 | 0 | 0 |
| | *P. aeruginosa* | 9.4×10⁶ | 2×10¹ | 0 | 0 | 0 | 0 |
| | *E. coli* | 4×10⁶ | Full | 5.6×10³ | 3.2×10³ | 0 | 0 |
| | *S. aureus* | 9×10⁶ | Full | 0 | 0 | 0 | 0 |
| SN 0.5wt% + BZ 0.1%wt +WS 0.5wt% | *A. brasiliensis* | 2.5×10⁵ | 6×10³ | 1.1×10³ | 3×10⁵ | 5^{×}10¹ | 1×10¹ |
| | *C. albicans* | 3×10⁵ | 0 | 0 | 0 | 0 | 0 |
| | *P. aeruginosa* | 9.4×10⁶ | 0 | 0 | 0 | 0 | 0 |
| | *E. coli* | 4×10⁶ | Full | 5.2×10³ | 1.2×10³ | 0 | 0 |
| | *S. aureus* | 9×10⁶ | 9×10³ | 0 | 0 | 0 | 0 |
| SN 0.5wt% + BZ 0.2 wt% +WS 0.5 wt% | *A. brasiliensis* | 2.5×10⁵ | 9.7×10³ | 1.3×10³ | 6×10² | 3.6×10² | 1×10² |
| | *C. albicans* | 3×10⁵ | 0 | 0 | 0 | 0 | 0 |
| | *P. aeruginosa* | 9.4×10⁶ | 0 | 0 | 0 | 0 | 0 |
| | *E. coli* | 4×10⁶ | Full | 8×10³ | 3.6×10³ | 0 | 0 |
| | *S. aureus* | 9×10⁶ | 7×10³ | 0 | 0 | 0 | 0 |
| SN 0.5wt% +BZ 0.5wt% +WS 0.5 wt% | *A. brasiliensis* | 2.5×10⁵ | 2×10⁴ | 6.8×10³ | 5.5×10³ | 6×10² | 7×10¹ |
| | *C. albicans* | 3×10⁵ | 4×10¹ | 0 | 0 | 0 | 0 |
| | *P. aeruginosa* | 9.4×10⁶ | 0 | 0 | 0 | 0 | 0 |
| | *E. coli* | 4×10⁶ | Full | Full | Full | 0 | 0 |
| | *S. aureus* | 9×10⁶ | 0 | 0 | 0 | 0 | 0 |
| Cream + bacteria | *A. brasiliensis* | 2.5×10⁵ | Full | Full | Full | Full | 3.6×10⁴ |
| | *C. albicans* | 3×10⁵ | Full | Full | Full | 5.7×10² | 0 |
| | *P. aeruginosa* | 9.4×10⁶ | Full | Full | Full | Full | Full |
| | *E. coli* | 4×10⁶ | 4×10³ | 6.5×10² | 2.4×10² | 0 | 0 |
| | *S. aureus* | 9×10⁶ | Full | Full | Full | Full | 4.96×10³ |
| Sharomix | *A. brasiliensis* | 2.5×10⁵ | 0 | 0 | 0 | 0 | 0 |
| | *C. albicans* | 3×10⁵ | 0 | 0 | 0 | 0 | 0 |
| | *P. aeruginosa* | 9.4×10⁶ | 0 | 0 | 0 | 0 | 0 |
| | *E. coli* | 4×10⁶ | 0 | 0 | 0 | 0 | 0 |
| | *S. aureus* | 9×10⁶ | 0 | 0 | 0 | 0 | 0 |

As observed from **Table 10,** addition of *Wasabia* extract improves antimicrobial activity against all of the tested microorganisms.

### Example 11: Composition with benzoin, saponin and glyceryl caprylate

10% Sumatra benzoin (BZ-1) in MCT extract was prepared as in Example 1.

Glyceryl caprylate and benzoin extracts were added to a non-preserved cream formulation in different concentration, with and without *Sapindus mukorossi* extract. The results are provided in **Table 11.**

Tested Microorganisms: *E. coli* (ATCC 8739); S. *aureus* (ATCC 6538); *P. aeruginosa (ATCC 9027); C. albicans (ATCC 10231) and A. Brasiliensis (ATCC 16404).*

**Table 11: Total count for different microorganisms after contact with the examined ingredients**

| **Tested Formula** | **Tested organism** | **Initial inoculums level (cfu/ml)** | **2 days** | **7 days** | **14 days** |
|---|---|---|---|---|---|
| SN 0.2 wt% | *A. brasiliensis* | 4.2×10⁵ | 4.24×10⁴ | 4.80×10⁴ | 2.40×10⁴ |
| | *C. albicans* | 4.1×10⁵ | 0 | 0 | 0 |
| | *P. aeruginosa* | 1.27×10⁷ | 8.90×10² | full | full |
| | *E. coli* | 6.65×10⁶ | full | 1.00×10¹ | 0 |
| | *S. aureus* | 6.4×10⁶ | full | full | full |
| SN 0.5 wt% | *A. brasiliensis* | 4.2×10⁵ | 2.32×10⁴ | 3.00×10⁴ | 3.20×10⁴ |
| | *C. albicans* | 4.1×10⁵ | 0 | 0 | 0 |
| | *P. aeruginosa* | 1.27×10⁷ | 6.80×10² | full | full |
| | *E. coli* | 6.65×10⁶ | 3.42×10⁴ | 1.00×10¹ | 0 |
| | *S. aureus* | 6.4×10⁶ | full | full | full |
| BZ-10.1 wt% | *A. brasiliensis* | 4.2×10⁵ | full | full | full |
| | *C. albicans* | 4.1×10⁵ | full | full | 1.40×10⁴ |
| | *P. aeruginosa* | 1.27×10⁷ | 1.10×10² | 9.10×10² | full |
| | *E. coli* | 6.65×10⁶ | 1.28×10⁴ | 1.00×10¹ | 0 |
| | *S. aureus* | 6.4×10⁶ | 7.20×10³ | 1.00×10¹ | 0 |
| BZ-1 0.2 wt% | *A. brasiliensis* | 4.2×10⁵ | full | full | full |
| | *C. albicans* | 4.1×10⁵ | full | full | 9.60×10³ |
| | *P. aeruginosa* | 1.27×10⁷ | 1.70×10² | 2.00×10¹ | 1.40×10² |
| | *E. coli* | 6.65×10⁶ | 3.76×10³ | 0 | 0 |
| | *S. aureus* | 6.4×10⁶ | 2.50×10² | 0 | 0 |
| Glyceryl caprylate 0.005 wt% | *A. brasiliensis* | 4.2×10⁵ | full | full | full |
| | *C. albicans* | 4.1×10⁵ | full | full | full |
| | *P. aeruginosa* | 1.27×10⁷ | 6.24×10³ | full | full |
| | *E. coli* | 6.65×10⁶ | Full | 1.12×10⁴ | 3.20×10² |
| | *S. aureus* | 6.4×10⁶ | full | full | Full |
| Glyceryl caprylate 0.01 wt% | *A. brasiliensis* | 4.2×10⁵ | full | full | Full |
| | *C. albicans* | 4.1×10⁵ | full | full | Full |
| | *P. aeruginosa* | 1.27×10⁷ | 7.20×10² | full | Full |
| | *E. coli* | 6.65×10⁶ | Full | full | 2.00×10³ |
| | *S. aureus* | 6.4×10⁶ | full | full | Full |
| Glyceryl caprylate 0.02 wt% | *A. brasiliensis* | 4.2×10⁵ | full | full | Full |
| | *C. albicans* | 4.1×10⁵ | full | full | Full |
| | *P. aeruginosa* | 1.27×10⁷ | 1.56×10³ | full | Full |
| | *E. coli* | 6.65×10⁶ | Full | full | 2.10×10³ |
| | *S. aureus* | 6.4×10⁶ | full | full | Full |
| Glyceryl caprylate 0.05 wt% | *A. brasiliensis* | 4.2×10⁵ | full | full | Full |
| | *C. albicans* | 4.1×10⁵ | full | full | Full |
| | *P. aeruginosa* | 1.27×10⁷ | 0 | 0 | 2.00×10⁴ |
| | *E. coli* | 6.65×10⁶ | full | 4.80×10³ | 3.00×10¹ |
| | *S. aureus* | 6.4×10⁶ | full | full | 3.20^{x}10⁴ |
| Glyceryl caprylate 0.1 wt% | *A. brasiliensis* | 4.2×10⁵ | full | full | full |
| | *C. albicans* | 4.1×10⁵ | full | 3.20×10³ | 2.90×10² |
| | *P. aeruginosa* | 1.27×10⁷ | 0 | 0 | 0 |
| | *E. coli* | 6.65×10⁶ | 0 | 0 | 0 |
| | *S. aureus* | 6.4×10⁶ | full | full | 1.11×10³ |
| SN 0.2 wt% | *A. brasiliensis* | 4.2×10⁵ | 1.52×10⁴ | 8.80×10³ | 4.40×10³ |
| +BZ-10.1 wt% | *C. albicans* | 4.1×10⁵ | 0 | 0 | 0 |
| | *P. aeruginosa* | 1.27×10⁷ | 1.70×10² | 4.80×10³ | full |
| | *E. coli* | 6.65×10⁶ | full | 1.30×10² | 0 |
| | *S. aureus* | 6.4×10⁶ | 5.52×10³ | 0 | 0 |
| SN 0.2 wt% +BZ-10.2 wt% | *A. brasiliensis* | 4.2×10⁵ | 3.04×10⁴ | 1.20×10⁴ | 2.00×10⁴ |
| | *C. albicans* | 4.1×10⁵ | 8.70×10² | 7.00×10¹ | 1.00×10¹ |
| | *P. aeruginosa* | 1.27×10⁷ | 0 | 2.00×10¹ | 0 |
| | *E. coli* | 6.65×10⁶ | 1.60×10³ | 0 | 0 |
| | *S. aureus* | 6.4×10⁶ | 3.00×10¹ | 0 | 0 |
| SN 0.5 wt% +BZ-10.1 wt% | *A. brasiliensis* | 4.2×10⁵ | 8.70×10³ | 1.80×10³ | 4.10×10² |
| | *C. albicans* | 4.1×10⁵ | 0 | 0 | 0 |
| | *P. aeruginosa* | 1.27×10⁷ | 8.00×10¹ | 4.20×10² | 5.20×10³ |
| | *E. coli* | 6.65×10⁶ | full | 1.00×10³ | 0 |
| | *S. aureus* | 6.4×10⁶ | full | 0 | 0 |
| SN 0.5 wt% +BZ-10.2 wt% | *A. brasiliensis* | 4.2×10⁵ | 1.88×10⁴ | 2.50×10³ | 1.40×10³ |
| | *C. albicans* | 4.1×10⁵ | 0 | 0 | 0 |
| | *P. aeruginosa* | 1.27×10⁷ | 2.00×10¹ | 2.00×10¹ | 0 |
| | *E. coli* | 6.65×10⁶ | full | 9.00×10² | 0 |
| | *S. aureus* | 6.4×10⁶ | 630 | 0 | 0 |
| SN 0.2 wt% +BZ-10.1 wt% +Gly. cap. 0.005 wt% | *A. brasiliensis* | 4.2×10⁵ | 2.16×10⁴ | 6.80×10³ | 3.40×10³ |
| | *C. albicans* | 4.1×10⁵ | 1.00×10¹ | 1.00×10¹ | 0 |
| | *P. aeruginosa* | 1.27×10⁷ | 5.00×10¹ | 2.80×10² | full |
| | *E. coli* | 6.65×10⁶ | full | 2.00×10¹ | 0 |
| | *S. aureus* | 6.4×10⁶ | 6.08×10³ | 0 | 0 |
| SN 0.2 wt% +BZ-10.1 wt% +Gly. cap. 0.01 wt% | *A. brasiliensis* | 4.2×10⁵ | 2.16×10⁴ | 8.40×10³ | 4.90×10³ |
| | *C. albicans* | 4.1×10⁵ | 1.50×10² | 0 | 0 |
| | *P. aeruginosa* | 1.27×10⁷ | 3.00×10¹ | 1.00×10² | 7.60×10³ |
| | *E. coli* | 6.65×10⁶ | 1.41×10⁴ | 0 | 0 |
| | *S. aureus* | 6.4×10⁶ | 3.76×10³ | 0 | 0 |
| SN 0.2 wt% +BZ-10.1 wt% +Gly. cap. 0.02 wt% | *A. brasiliensis* | 4.2×10⁵ | 1.36×10⁴ | 5.30×10³ | 3.50×10³ |
| | *C. albicans* | 4.1×10⁵ | 2.60×10² | 7.00×10¹ | 1.00×10¹ |
| | *P. aeruginosa* | 1.27×10⁷ | 3.00×10¹ | 4.50×10² | 2.24×10⁴ |
| | *E. coli* | 6.65×10⁶ | 5.92×10³ | 0 | 0 |
| | *S. aureus* | 6.4×10⁶ | 1.14×10⁴ | 0 | 0 |
| SN 0.2 wt% +BZ-10.1 wt% +Gly. cap. 0.05 wt% | *A. brasiliensis* | 4.2×10⁵ | 1.72×10⁴ | 5.60×10³ | 3.60×10³ |
| | *C. albicans* | 4.1×10⁵ | 1.02×10⁴ | 5.10×10² | 4.70×10² |
| | *P. aeruginosa* | 1.27×10⁷ | 0 | 0 | 1.00×10² |
| | *E. coli* | 6.65×10⁶ | 0 | 0 | 0 |
| | *S. aureus* | 6.4×10⁶ | 1.64×10³ | 0 | 0 |
| SN 0.2 wt% +BZ-10.1 wt% +Gly. cap. 0.1 wt% | *A. brasiliensis* | 4.2×10⁵ | full | 4.00×10⁴ | 1.00×10⁴ |
| | *C. albicans* | 4.1×10⁵ | 3.32×10³ | 0 | 0 |
| | *P. aeruginosa* | 1.27×10⁷ | 0 | 0 | 0 |
| | *E. coli* | 6.65×10⁶ | 0 | 0 | 0 |
| | *S. aureus* | 6.4×10⁶ | 1.80×10² | 0 | 0 |
| SN 0.2 wt% +BZ-10.2 wt% +Gly. cap. 0.005 wt% | *A. brasiliensis* | 4.2×10⁵ | 4.00×10⁴ | 3.00×10⁴ | 1.26×10⁴ |
| | *C. albicans* | 4.1×10⁵ | 2.16×10³ | 4.80×10² | 3.90×10² |
| | *P. aeruginosa* | 1.27×10⁷ | 0 | 1.70×10² | 0 |
| | *E. coli* | 6.65×10⁶ | 1.30×10⁴ | 0 | 0 |
| | *S. aureus* | 6.4×10⁶ | 2.80×10² | 0 | 0 |
| SN 0.2 wt% +BZ-10.2 wt% +Gly. cap. 0.01 wt% | *A. brasiliensis* | 4.2×10⁵ | 2.12×10⁴ | 6.50×10³ | 3.00×10³ |
| | *C. albicans* | 4.1×10⁵ | 2.40×10³ | 5.20×10² | 6.20×10² |
| | *P. aeruginosa* | 1.27×10⁷ | 0 | 0 | 0 |
| | *E. coli* | 6.65×10⁶ | 1.80×10³ | 0 | 0 |
| | *S. aureus* | 6.4×10⁶ | 4.00×10² | 0 | 0 |
| SN 0.2 wt% +BZ-10.2 wt% +Gly. cap. 0.02 wt% | *A. brasiliensis* | 4.2×10⁵ | 2.48×10⁴ | 1.20×10⁴ | 2.90×10³ |
| | *C. albicans* | 4.1×10⁵ | 1.34×10⁴ | 7.10×10² | 1.03×10³ |
| | *P. aeruginosa* | 1.27×10⁷ | 0 | 0 | 3.00×10¹ |
| | *E. coli* | 6.65×10⁶ | 2.70×10² | 0 | 0 |
| | *S. aureus* | 6.4×10⁶ | 3.80×10² | 0 | 0 |
| SN 0.2 wt% +BZ-10.2 wt% +Gly. cap. 0.05 wt% | *A. brasiliensis* | 4.2×10⁵ | 2.56×10⁴ | 1.80×10⁴ | 6.40×10³ |
| | *C. albicans* | 4.1×10⁵ | full | full | 1.32×10⁴ |
| | *P. aeruginosa* | 1.27×10⁷ | 0 | 0 | 0 |
| | *E. coli* | 6.65×10⁶ | 0 | 0 | 0 |
| | *S. aureus* | 6.4×10⁶ | 1. 80×10² | 0 | 0 |
| SN 0.2 wt% +BZ-10.2 wt% +Gly. cap. 0.1 wt% | *A. brasiliensis* | 4.2×10⁵ | 5.12×10⁴ | 2.64×10⁴ | 1.28×10⁴ |
| | *C*. *albicans* | 4.1×10⁵ | 5.12×10³ | 3.00×10¹ | 1.00×10¹ |
| | *P. aeruginosa* | 1.27×10⁷ | 0 | 0 | 0 |
| | *E. coli* | 6.65×10⁶ | 0 | 0 | 0 |
| | *S. aureus* | 6.4×10⁶ | 3.00×10¹ | 0 | 0 |
| SN 0.5 wt% +BZ-10.1 wt% +Gly. cap. 0.005 wt% | *A. brasiliensis* | 4.2×10⁵ | 8.30×10³ | 1.70×10² | 8.00×10² |
| | *C*. *albicans* | 4.1×10⁵ | 0 | 0 | 0 |
| | *P. aeruginosa* | 1.27×10⁷ | 1.70×10² | 8.00×10³ | full |
| | *E. coli* | 6.65×10⁶ | full | 8.00×10² | 0 |
| | *S. aureus* | 6.4×10⁶ | full | 0 | 0 |
| SN 0.5 wt% +BZ-10.1 wt% +Gly. cap. 0.01 wt% | *A. brasiliensis* | 4.2×10⁵ | 7.10×10³ | 1.20×10³ | 2.90×10² |
| | *C*. *albicans* | 4.1×10⁵ | 0 | 0 | 0 |
| | *P. aeruginosa* | 1.27×10⁷ | 1.80×10² | 2.40×10³ | 1.07×10³ |
| | *E. coli* | 6.65×10⁶ | full | 4.50×10² | 0 |
| | *S. aureus* | 6.4×10⁶ | full | 0 | 0 |
| SN 0.5 wt% +BZ-10.1 wt% +Gly. cap. 0.02 wt% | *A. brasiliensis* | 4.2×10⁵ | 8.20×10³ | 2.10×10³ | 5.70×10² |
| | *C*. *albicans* | 4.1×10⁵ | 1.00×10¹ | 0 | 0 |
| | *P. aeruginosa* | 1.27×10⁷ | 8.00×10¹ | 1.32×10³ | 2.23×10³ |
| | *E. coli* | 6.65×10⁶ | full | 1.60×10² | |
| | *S. aureus* | 6.4×10⁶ | full | 0 | 0 |
| SN 0.5 wt% +BZ-10.1 wt% +Gly. cap. 0.05 wt% | *A. brasiliensis* | 4.2×10⁵ | 1.84×10⁴ | 4.30×10³ | 1.72×10³ |
| | *C. albicans* | 4.1×10⁵ | 7.00×10¹ | 0 | 0 |
| | *P. aeruginosa* | 1.27×10⁷ | 0 | 1.20×10³ | full |
| | *E. coli* | 6.65×10⁶ | 3.32×10³ | 0 | 0 |
| | *S. aureus* | 6.4×10⁶ | full | 0 | 0 |
| SN 0.5 wt% +BZ-1 0.1 wt% +Gly. cap. 0.1 wt% | *A. brasiliensis* | 4.2×10⁵ | full | 2.00×10⁴ | 8.00×10³ |
| | *C. albicans* | 4.1×10⁵ | 4.16×10³ | 1.00×10¹ | 0 |
| | *P. aeruginosa* | 1.27×10⁷ | 0 | 0 | 0 |
| | *E. coli* | 6.65×10⁶ | 0 | 0 | 0 |
| | *S. aureus* | 6.4×10⁶ | full | 0 | 0 |
| SN 0.5 wt% +BZ-1 0.2 wt% +Gly. cap. 0.005 wt% | *A. brasiliensis* | 4.2×10⁵ | 3.50×10³ | 2.303 | 7.00×10² |
| | *C. albicans* | 4.1×10⁵ | 2.00×10¹ | 0 | 0 |
| | *P. aeruginosa* | 1.27×10⁷ | 1.00×10¹ | 1.00×10¹ | 5.00×10¹ |
| | *E. coli* | 6.65×10⁶ | full | 4.20×10² | 0 |
| | *S. aureus* | 6.4×10⁶ | 2.16×10³ | 0 | 0 |
| SN 0.5 wt% +BZ-1 0.2 wt% +Gly. cap. 0.01 wt% | *A. brasiliensis* | 4.2×10⁵ | 1.16×10⁴ | 3.10×10³ | 7.70×10² |
| | *C. albicans* | 4.1×10⁵ | 1.00×10¹ | 0 | 0 |
| | *P. aeruginosa* | 1.27×10⁷ | 4.00×10¹ | 1.00×10¹ | 0 |
| | *E. coli* | 6.65×10⁶ | full | 7.00×10¹ | 0 |
| | *S. aureus* | 6.4×10⁶ | 2.04×10³ | 0 | 0 |
| SN 0.5 wt% +BZ-1 0.2 wt% +Gly. cap. 0.02 wt% | *A. brasiliensis* | 4.2×10⁵ | 1.64×10⁴ | 3.10×10³ | 8.00×10² |
| | *C. albicans* | 4.1×10⁵ | 2.90×10² | 5.00×10¹ | 0 |
| | *P. aeruginosa* | 1.27×10⁷ | 4.00×10¹ | 0 | 0 |
| | *E. coli* | 6.65×10⁶ | 9.00×10¹ | 0 | 0 |
| | *S. aureus* | 6.4×10⁶ | 1.39×10⁴ | 0 | 0 |
| SN 0.5 wt% +BZ-1 0.2 wt% +Gly. cap. 0.05 wt% | *A. brasiliensis* | 4.2×10⁵ | 1.32×10⁴ | 3.70×10³ | 1.32×10³ |
| | *C. albicans* | 4.1×10⁵ | 2.60×10² | 6.00×10¹ | 1.00×10¹ |
| | *P. aeruginosa* | 1.27×10⁷ | 1.00×10² | 2.00×10¹ | 0 |
| | *E. coli* | 6.65×10⁶ | 9.00×10¹ | 0 | 0 |
| | *S. aureus* | 6.4×10⁶ | 1.12×10⁴ | 0 | 0 |
| SN 0.5 wt% +BZ-1 0.2 wt% +Gly. cap. 0.1 wt% | *A. brasiliensis* | 4.2×10⁵ | full | 1.21×10⁴ | 3.80×10³ |
| | *C. albicans* | 4.1×10⁵ | 7.36×10³ | 1.50×10² | 2.00×10¹ |
| | *P. aeruginosa* | 1.27×10⁷ | 0 | 0 | 0 |
| | *E. coli* | 6.65×10⁶ | 0 | 0 | 0 |
| | *S. aureus* | 6.4×10⁶ | 8.32×10³ | 0 | 0 |
| Cream + bacteria | *A. brasiliensis* | 4.2×10⁵ | full | 9.60×10⁴ | full |
| | *C. albicans* | 4.1×10⁵ | full | full | full |
| | *P. aeruginosa* | 1.27×10⁷ | 1.87×10⁴ | full | full |
| | *E. coli* | 6.65×10⁶ | Full | 1.36×10³ | 6.00×10¹ |
| | *S. aureus* | 6.4×10⁶ | full | full | full |
| Sharomix 1% | *A. brasiliensis* | 4.2×10⁵ | 0 | 0 | 0 |
| | *C. albicans* | 4.1×10⁵ | 0 | 0 | 0 |
| | *P. aeruginosa* | 1.27×10⁷ | 0 | 0 | 0 |
| | *E. coli* | 6.65×10⁶ | 0 | 0 | 0 |
| | *S. aureus* | 6.4×10⁶ | 0 | 0 | 0 |

As observed from **Table 11,** the addition of glyceryl caprylate improved the antimicrobial activity of the tested formulations.

### Example 12: Reference example

It is noted that not every plant-base extract, which is known to have antimicrobial activity *per-se,* may result in an increased antimicrobial activity when combined with a saponin, as compared to compositions of the present invention.

For example, *thyme* and *phellodendron* extracts are known in the field of the invention to have antimicrobial activity and are often used in various cosmetic products as preservatives.

In order to show the uniqueness of formulations of the present invention, *thyme* and/or *phellodendron* extracts were combined with saponin (as prepared in Example 1), and their antimicrobial activity were tested over time, according to ISO 11920 Pharmacopoeia (with one exception: the examined bacteria are P. aeruginosa and A. niger).

The test was conducted on 4 g of commercial samples of a non-preserved lotion or liquid soap. The saponin used was derived from *Camellia Oleifera.* The other preservatives were: (a) *Thyme* leaves extract (not essential oil), and/or (b) *Phellodendron* leaves extract. Each sample was separately inoculated by one of the two microorganisms. The number of surviving microorganisms was monitored periodically during an incubation of 4 weeks and the colony forming units (CFU) were counted. The results together with the preservatives' concentrations [wt%] are shown in **Tables 12-1** and **12-2.**

Tested Microorganisms: *E. coli* (ATCC 8739); S. *aureus* (ATCC 6538); *P. aeruginosa (ATCC 9027); C. albicans (ATCC 10231) and A. Brasiliensis (ATCC 16404).*

**Table 12-1: Total count for different microorganisms after contact with the examined ingredients; lotion base**

| **Tested Formula** | **Test organism** | **Day 0** | **7 days** | **14 days** | **28 days** |
|---|---|---|---|---|---|
| Phellodendron 1%, C. oleifera SAP 1% | *P. aeruginosa* | 1 × 10⁵ | Full | 0 | 0 |
| | *A.niger* | 1 × 10⁵ | 6.7×10⁴ | 7.4×10⁴ | Full |
| Phellodendron 0.5%, C. oleifera SAP 0.5% | *P. aeruginosa* | 1 × 10⁵ | 0 | 0 | 0 |
| | *A.niger* | 1 × 10⁵ | 5.7×10⁴ | 4.4×10⁴ | 2.8×10⁴ |
| Phellodendron 0.2%, C. oleifera SAP 0.2% | *P. aeruginosa* | 1 × 10⁵ | Full | Full | Full |
| | *A.niger* | 1 × 10⁵ | 1.2×10⁵ | 4×10⁴ | 4.3×10⁴ |
| Phellodendron 0.1%, C. oleifera SAP 0.1% | *P. aeruginosa* | 1×10⁵ | Full | 8×10⁴ | Full |
| | *A.niger* | 1×10⁵ | 1×10⁵ | Full | 4×10⁴ |
| Thyme 1%, Phellodendron 1%, C. oleifera SAP 1% | *P. aeruginosa* | 1×10⁵ | 0 | 0 | 1.2×10³ |
| | *A.niger* | 1×10⁵ | Full | Full | full |
| Thyme 0.5%, Phellodendron 0.5%, C. oleifera SAP 0.5% | *P. aeruginosa* | 1×10⁵ | 0 | 0 | 0 |
| | *A.niger* | 1×10⁵ | full | full | full |
| Thyme 0.2%, Phellodendron 0.2%, C. oleifera SAP 0.2% | *P. aeruginosa* | 1×10⁵ | Full | Full | Full |
| | *A.niger* | 1×10⁵ | 1.2×10⁵ | 3.2×10⁴ | 7×10⁴ |
| Thyme 0.1%, Phellodendron 0.1%, C. oleifera SAP 0.1% | *P. aeruginosa* | 1×10⁵ | Full | Full | Full |
| | *A.niger* | 1×10⁵ | 5×10⁴ | 4×10⁴ | 4.4×10⁴ |
| Positive cont - (synthetic preservatives) | *P. aeruginosa* | 1×10⁵ | 0 | 0 | 0 |
| | *A.niger* | 1×10⁵ | 0 | 0 | 0 |
| Bacteria +cream (no additives) | *P. aeruginosa* | 1×10⁵ | Full | Full | Full |
| | *A.niger* | 1×10⁵ | 6.7×10⁴ | 3.4×10⁴ | 4×10⁴ |
| Cream | *P. aeruginosa* | 0 | 0 | 0 | 0 |
| | *A.niger* | 0 | 0 | 0 | 0 |

**Table 12-2: Total count for different microorganisms after contact with the examined ingredients; liquid soap base**

| **Tested Formula** | **Test organism** | **Day 0** | **7 days** | **14 days** | **28 days** |
|---|---|---|---|---|---|
| Phellodendron 1%, C. oleifera SAP 1% | *P. aeruginosa* | 1×10⁵ | Full | Full | Full |
| | *A.niger* | 1×10⁵ | 1.2×10⁴ | 1.2×10⁴ | 8×10³ |
| Phellodendron 0.5%, C. oleifera SAP 0.5% | *P. aeruginosa* | 1×10⁵ | Full | Full | Full |
| | *A.niger* | 1×10⁵ | 1.7×10⁴ | 7×10⁴ | 9.5×10³ |
| Phellodendron 0.2%, C. oleifera SAP 0.2% | *P. aeruginosa* | 1×10⁵ | Full | Full | Full |
| | *A.niger* | 1×10⁵ | 2×10⁴ | 1.4×10⁴ | 2.4×10³ |
| Phellodendron 0.1%, C. oleifera SAP 0.1% | *P. aeruginosa* | 1×10⁵ | Full | Full | Full |
| | *A.niger* | 1×10⁵ | 4×10⁴ | 1.8×10⁴ | 2×10³ |
| Thyme 1%, Phellodendron 1%, C. oleifera SAP 1% | *P. aeruginosa* | 1×10⁵ | Full | Full | Full |
| | *A.niger* | 1×10⁵ | 8×10³ | 8×10³ | 3.6×10³ |
| Thyme 0.5%, Phellodendron 0.5%, C. oleifera SAP 0.5% | *P. aeruginosa* | 1×10⁵ | Full | Full | Full |
| | *A.niger* | 1×10⁵ | 1×10⁴ | 3.1×10³ | 1.2×10³ |
| Thyme 0.2%, Phellodendron 0.2%, C. oleifera SAP 0.2% | *P. aeruginosa* | 1×10⁵ | Full | Full | Full |
| | *A.niger* | 1×10⁵ | 2.4×10⁴ | 7.4×10³ | 1.4×10³ |
| Thyme 0.1%, Phellodendron 0.1%, C. oleifera SAP 0.1% | *P. aeruginosa* | 1×10⁵ | Full | Full | Full |
| | *A.niger* | 1×10⁵ | 3.1×10⁴ | 9×10³ | 1.4×10³ |
| Positive cont - (synthetic preservatives) | *P. aeruginosa* | 1×10⁵ | 0 | 0 | 0 |
| | *A.niger* | 1×10⁵ | 0 | 0 | 0 |
| Bacteria +cream (no additives) | *P. aeruginosa* | 1×10⁵ | Full | Full | Full |
| | *A.niger* | 1×10⁵ | 4.8×10⁴ | 6.4×10⁴ | 2.2×10³ |
| Cream | *P. aeruginosa* | 0 | 0 | 0 | 0 |
| | *A.niger* | 0 | 0 | 0 | 0 |

As can be seen from **Tables 12-1 and 12-2,** none of the combinations demonstrated effective annihilation of the inoculation microorganisms in the different tested mixtures. It is also of note that the only combinations that demonstrated effectiveness were Phellodendron 0.5% + C. oleifera SAP 0.5%; or Thyme 0.5% + Phellodendron 0.5% + C. oleifera SAP 0.5%, however these combinations only demonstrated effective activity against P. aeruginosa, while no significant activity was observed for A. Niger.

From these results it is clearly evident that not every extract, which is known to have antimicrobial activity on its own, may be combined with a saponin material for obtaining a desired antimicrobial activity or, in fact, any antimicrobial activity.

## Claims

1. A composition having an antimicrobial activity, comprising
saponin extract obtained from a saponin-containing plant source extracted by water, alcohol or a water/alcohol solution; and
a *Styrax* extract from the resin and/or bark of at least one plant species of the genus *Styrax* selected from *Styrax paralleloneurus* (Sumatra benzoin), *Styrax tonkinensis* (Siam Benzoin) and mixtures thereof, the Styrax extract being obtained by mixing the resin and/or bark with a hydrophobic solvent;
the saponin extract being from a saponin source different from said plant species of the genus *Styrax,* the *Styrax* extract containing at least one of cinnamic acid; cinnamic acid derivatives selected from P-coumaryl cinnamate, coniferyl cinnamate, cinnamyl cinnamate, benzyl cinnamate, and cinnamic acid esters, and/or benzoic acid derivatives selected from coniferyl benzoate, cinnamyl benzoate, P-coumaryl benzoate, and benzoic acid esters.

2. The composition of claim 1, wherein the total amount of cinnamic acid, cinnamic acid derivatives and benzoic acid derivatives in the *Styrax* extract is between about 0.1 and 10 wt%.

3. The composition of claim 1 or 2, wherein said Styrax extract further comprises terpenes and terpenoids.

4. The composition of any one of claims 1 to 3, wherein the Styrax extract further comprises pinoresinol.

5. The composition of any one of claims 1 to 4, wherein the weight ratio between the saponin extract and the extract from at least one plant species of the genus *Styrax* is between about 1:50 to about 50:1.

6. The composition of any one of claims 1 to 5, further comprising at least one additional plant extract, optionally wherein the additional plant extract is selected from extracts of the genera *Wasabia, Acacia, Terminalia, Olea,* and mixtures thereof, optionally wherein the additional plant extract is selected from *Wasabia japonica* extract, *Acacia arabica* (gum arabic tree, Babul) extract, *Terminalia bellerica* extract, *Olea europaea* extract and mixtures thereof.

7. The composition of claim 6, wherein the weight ratio between the additional plant extract and the *Styrax* extract is between about 1:400 and 400:1, between about 1:200 and 200:1, between about 1:100 and 100:1 or between about 1:10 and 10:1.

8. The composition of any one of claims 1 to 7, wherein the saponin extract is obtained from a plant source selected from shikakai, soyabeans, beans, peas (*Pisum sativum*), lucerne, tea, spinach, sugar beet, quinoa, liquorice, sunflower, horse chestnut, ginseng, oats, capsicum peppers, aubergine, tomato seed, alliums, asparagus, yam, fenugreek, yucca and ginseng, lucerne, mung beans, *Bupleurum falcatum, Camellia oleifera, Camellia sinensis, Desmodium adscendens, Gypsophila, Panax quinqufolius, Panax japonicas, Quillaja saponaria, Sapindus delavayi, Sapindus mukorossi, Sapindus marginatus, Sapindus saponaria, Sapindus trifoliatus, Saponaria officinalis,* and *Yucca schidigera* or any mixture thereof.

9. The composition of any one of claims 1 to 8, wherein the saponin extract is in a content of at least 0.001 wt% of the composition, optionally wherein the composition comprises saponin extract in a content of between about 0.01 and 2 wt%.

10. The composition of any one of claims 1 to 9, wherein the composition is formulated as a preservative formulation, an antimicrobial formulation, a pharmaceutical composition, a disinfectant formulation and a cosmetic formulation.

11. A pharmaceutical composition comprising the composition as defined in any one of claims 1 to 10 and a pharmaceutically acceptable carrier or diluent.

12. A cosmetic product comprising the composition as defined in any one of claims 1 to 10.

## Patentansprüche

1. Zusammensetzung mit antimikrobieller Aktivität, umfassend
Saponinextrakt, der aus einer Saponin-enthaltenden Pflanzenquelle gewonnen wurde, extrahiert durch Wasser, Alkohol oder eine Wasser/Alkohol-Lösung; und
einem Styrax-Extrakt aus dem Harz und/oder der Rinde mindestens einer Pflanzenart der Gattung *Styrax,* ausgewählt aus *Styrax paralleloneurus* (Sumatra-Benzoin), *Styrax tonkinensis* (Siam Benzoin) und Mischungen davon, wobei der Styrax-Extrakt durch Mischen des Harzes und/oder der Rinde mit einem hydrophoben Lösungsmittel gewonnen wird;
wobei der Saponin-Extrakt aus einer Saponin-Quelle stammt, die sich von dieser Pflanzenart der Gattung *Styrax* unterscheidet, wobei der Styrax-Extrakt mindestens eines von Zimtsäure; Zimtsäurederivaten, ausgewählt aus P-Cumarylcinnamat, Coniferylcinnamat, Cinnamylcinnamat, Benzylcinnamat und Zimtsäureestern, und/oder Benzoesäurederivaten, ausgewählt aus Coniferylbenzoat, Cinnamylbenzoat, P-Cumarylbenzoat und Benzoesäureestern, enthält.

2. Zusammensetzung nach Anspruch 1, wobei die Gesamtmenge an Zimtsäure, Zimtsäurederivaten und Benzoesäurederivaten im Styrax-Extrakt zwischen etwa 0,1 und 10 Gew.-% beträgt.

3. Zusammensetzung nach Anspruch 1 oder 2, wobei der Styrax-Extrakt ferner Terpene und Terpenoide umfasst.

4. Zusammensetzung nach einem der Ansprüche 1 bis 3, wobei der Styrax-Extrakt ferner Pinoresinol umfasst.

5. Zusammensetzung nach einem der Ansprüche 1 bis 4, wobei das Gewichtsverhältnis zwischen dem Saponinextrakt und dem Extrakt aus mindestens einer Pflanzenart der Gattung Styrax zwischen etwa 1:50 bis etwa 50:1 beträgt.

6. Zusammensetzung nach einem der Ansprüche 1 bis 5, ferner umfassend mindestens einen zusätzlichen Pflanzenextrakt, wobei der zusätzliche Pflanzenextrakt gegebenenfalls ausgewählt ist aus Extrakten der Gattungen *Wasabia, Acacia, Terminalia, Olea,* und Mischungen davon, gegebenenfalls wobei der zusätzliche Pflanzenextrakt ausgewählt ist aus *Wasabia japonica-*Extrakt, *Acacia arabica* (Gummi arabicum Baum, Babul)-Extrakt, *Terminalia bellerica*-Extrakt, *Olea europaea-Extrakt* und Mischungen davon.

7. Zusammensetzung nach Anspruch 6, wobei das Gewichtsverhältnis zwischen dem zusätzlichen Pflanzenextrakt und dem Styrax-Extrakt zwischen etwa 1:400 und 400:1, zwischen etwa 1:200 und 200:1, zwischen etwa 1:100 und 100:1 oder zwischen etwa 1:10 und 10:1 liegt.

8. Zusammensetzung nach einem der Ansprüche 1 bis 7, wobei der Saponinextrakt aus einer Pflanzenquelle, ausgewählt aus Shikakai, Sojabohnen, Bohnen, Erbsen *(Pisum sativum),* Luzerne, Tee, Spinat, Zuckerrübe, Quinoa, Süßholz, Sonnenblume, Rosskastanie, Ginseng, Hafern, Paprika, Aubergine, Tomatensamen, Allium, Spargel, Yamswurzel, Bockshornklee, Yucca und Ginseng, Luzerne, Mungobohnen, *Bupleurum falcatum, Camellia oleifera, Camellia sinensens, Desmodium adscendens Gypsophila, Panax quinqufolius, Panax japonicas, Quillaja saponaria, Sapindus delavayi, Sapindus mukorossi, Sapindus marginatus, Sapindus saponaria, Sapindus trifoliatus, Saponaria officinalis* und *Yucca schidigera* oder irgendeiner Mischung davon, gewonnen wird.

9. Zusammensetzung nach einem der Ansprüche 1 bis 8, wobei der Saponinextrakt in einem Gehalt von mindestens 0,001 Gew.-% der Zusammensetzung vorliegt, wobei gegebenenfalls die Zusammensetzung Saponinextrakt in einem Gehalt zwischen etwa 0,01 und 2 Gew.-% umfasst.

10. Zusammensetzung nach einem der Ansprüche 1 bis 9, wobei die Zusammensetzung als Konservierungsformulierung, antimikrobielle Formulierung, pharmazeutische Zusammensetzung, desinfizierende Formulierung und kosmetische Formulierung formuliert ist.

11. Pharmazeutische Zusammensetzung, umfassend die Zusammensetzung nach einem der Ansprüche 1 bis 10 und einen pharmazeutisch verträglichen Träger oder ein pharmazeutisch verträgliches Verdünnungsmittel.

12. Kosmetisches Produkt, umfassend die Zusammensetzung nach einem der Ansprüche 1 bis 10.

## Revendications

1. Composition ayant une activité antimicrobienne, comprenant
un extrait de saponine obtenu à partir d'une source végétale contenant de la saponine extraite avec de l'eau, un alcool, ou une solution hydro-alcoolique ; et
un extrait de *Styrax* provenant de la résine et/ou de l'écorce d'au moins une espèce végétale du genre *Styrax* choisie parmi *Styrax paralleloneurus* (benjoin de Sumatra), *Styrax tonkinensis* (benjoin de Siam), et leurs mélanges, l'extrait de Styrax étant obtenu par mélange de la résine et/ou de l'écorce avec un solvant hydrophobe ;
l'extrait de saponine provenant d'une source de saponine différente de ladite espèce végétale du genre *Styrax,* l'extrait de *Styrax* contenant au moins l'un parmi l'acide cinnamique, les dérivés d'acide cinnamique choisis parmi le cinnamate de P-coumaryle, le cinnamate de coniféryle, le cinnamate de cinnamyle, le cinnamate de benzyle, et les esters d'acide cinnamique, et/ou les dérivés d'acide benzoïque choisis parmi le benzoate de coniféryle, le benzoate de cinnamyle, le benzoate de P-coumaryle, et les esters d'acide benzoïque.

2. Composition selon la revendication 1, dans laquelle la quantité totale d'acide cinnamique, de dérivés d'acide cinnamique et de dérivés d'acide benzoïque dans l'extrait de *Styrax* est comprise entre environ 0,1 et 10 % en poids.

3. Composition selon la revendication 1 ou 2, dans laquelle ledit extrait de Styrax comprend en outre des terpènes et des terpénoïdes.

4. Composition selon l'une quelconque des revendications 1 à 3, dans laquelle l'extrait de Styrax comprend en outre du pinorésinol.

5. Composition selon l'une quelconque des revendications 1 à 4, dans laquelle le rapport en poids entre l'extrait de saponine et l'extrait provenant d'au moins une espèce végétale du genre *Styrax* est compris entre environ 1/50 et environ 50/1.

6. Composition selon l'une quelconque des revendications 1 à 5, comprenant en outre au moins un extrait végétal additionnel, éventuellement dans laquelle l'extrait végétal additionnel est choisi parmi les extraits des genres *Wasabia, Acacia, Terminalia, Olea,* et leurs mélanges, éventuellement dans laquelle l'extrait végétal additionnel est choisi parmi l'extrait de *Wasabia japonica,* l'extrait *d'Acacia arabica* (acacia, babul), l'extrait de *Terminalia bellerica,* l'extrait d'*Olea europaea* et leurs mélanges.

7. Composition selon la revendication 6, dans laquelle le rapport en poids entre l'extrait végétal additionnel et l'extrait de *Styrax* est compris entre environ 1/400 et 400/1, entre environ 1/200 et 200/1, entre environ 1/100 et 100/1, ou entre environ 1/10 et 10/1.

8. Composition selon l'une quelconque des revendications 1 à 7, dans laquelle l'extrait de saponine est obtenu à partir d'une source végétale choisie parmi le shikakai, le soja, le haricot, le pois (*Pisum sativum*), la luzerne, le thé, l'épinard, la betterave à sucre, le quinoa, la réglisse, le tournesol, le marron d'Inde, le ginseng, l'avoine, le piment, l'aubergine, les pépins de tomate, les plantes du genre Allium, l'asperge, l'igname, le fenugrec, le yucca et le ginseng, la luzerne, le haricot mungo, *Bupleurum falcatum, Camellia oleifera, Camellia sinensis, Desmodium adscendens, Gypsophila, Panax quinqufolius, Panax japonicas, Quillaja saponiaria, Sapindus delavayi, Sapindus mukorossi, Sapindus marginatus, Sapindus saponaria, Sapindus trifoliatus, Saponiaria officinalis,* et *Yucca shidigera,* ou l'un quelconque de leurs mélanges.

9. Composition selon l'une quelconque des revendications 1 à 8, dans laquelle l'extrait de saponine est présent en une teneur d'au moins 0,001 % en poids de la composition, éventuellement laquelle composition comprend l'extrait de saponine en une teneur comprise entre environ 0,01 et 2 % en poids.

10. Composition selon l'une quelconque des revendications 1 à 9, laquelle composition est formulée comme une formulation de conservateur, une formulation antimicrobienne, une composition pharmaceutique, une formulation désinfectante et une formulation cosmétique.

11. Composition pharmaceutique comprenant la composition telle que définie dans l'une quelconque des revendications 1 à 10 et un véhicule ou diluant pharmaceutiquement acceptable.

12. Produit cosmétique comprenant la composition telle que définie dans l'une quelconque des revendications 1 à 10.
